(19) **Europäisches Patentamt / European Patent Office / Office européen des brevets**

(11) **EP 2 348 119 B1**

(12) **EUROPEAN PATENT SPECIFICATION**

(45) Date of publication and mention of the grant of the patent:
**26.04.2017 Bulletin 2017/17**

(51) Int Cl.:
**C12N 15/867** (2006.01)   **C12N 7/02** (2006.01)
**C12N 5/10** (2006.01)

(21) Application number: **10012190.4**

(22) Date of filing: **03.02.2003**

(54) **Multicistronic lentiviral vector**

Multicistronische lentiviraler Vektor

Vecteur de lentivirus multicistronique

(84) Designated Contracting States:
**AT BE BG CH CY CZ DE DK EE ES FI FR GB GR HU IE IT LI LU MC NL PT SE SI SK TR**

(30) Priority: **01.02.2002 GB 0202403**
**31.05.2002 GB 0212768**

(43) Date of publication of application:
**27.07.2011 Bulletin 2011/30**

(62) Document number(s) of the earlier application(s) in accordance with Art. 76 EPC:
**03734767.1 / 1 504 108**

(73) Proprietor: **Oxford BioMedica (UK) Limited**
**Oxford Science Park**
**Oxford, OX4 4GA (GB)**

(72) Inventors:
• **Radcliffe, Philippa**
  **The OxfordScience Park,**
  **Oxford OX4 4GA (GB)**
• **Miskin, James E.**
  **The OxfordScience Park,**
  **Oxford OX4 4GA (GB)**
• **Wilkes, Fraser J.**
  **The OxfordScience Park,**
  **Oxford OX4 4GA (GB)**
• **Mitrophanous, Kyriacos A.**
  **The OxfordScience Park,**
  **Oxford OX4 4GA (GB)**

(74) Representative: **D Young & Co LLP**
**120 Holborn**
**London EC1N 2DY (GB)**

(56) References cited:
WO-A-94/05785    WO-A-99/32646
WO-A1-96/37623    WO-A2-01/79518
WO-A2-97/14809

• ROHLL J B ET AL: "DESIGN, PRODUCTION, SAFETY, EVALUATION, AND CLINICAL APPLICATIONS OF NONPRIMATE LENTIVIRAL VECTORS", METHODS IN ENZYMOLOGY; [METHODS IN ENZYMOLOGY], ACADEMIC PRESS INC, SAN DIEGO, CA, US, vol. 346, 1 January 2002 (2002-01-01), pages 466-500, XP009053525, ISSN: 0076-6879, DOI: 10.1016/S0076-6879(02)46072-7
• GUAN Y ET AL: "Construction and in vitro properties of a series of attenuated simian immunodeficiency viruses with all accessory genes deleted.", May 2001 (2001-05), JOURNAL OF VIROLOGY. MAY 2001, VOL. 75, NR. 9, PAGE(S) 4056 - 4067, XP002287264, ISSN: 0022-538X * the whole document *
• SCHNEIDER R ET AL: "Inactivation of the human immunodeficiency virus type 1 inhibitory elements allows rev-independent expression of gag and gag/protease and particle formation", JOURNAL OF VIROLOGY, THE AMERICAN SOCIETY FOR MICROBIOLOGY, US, vol. 71, no. 7, July 1997 (1997-07), pages 4892-4903, XP002137891, ISSN: 0022-538X
• INDRACCOLO S ET AL: "DNA immunization of mice aginst SIVmac293 gag and env using rev-independent expression plasmids", AIDS RESEARCH AND HUMAN RETROVIRUSES, MARY ANN LIEBERT, US, vol. 14, no. 1, January 1998 (1998-01), pages 83-90, XP002101009, ISSN: 0889-2229

**(Cont. next page)**

- KOTSOPOULOU E ET AL: "A Rev-independent human immunodeficiency virus type 1 (HIV-1)-based vector that exploits a codon-optimized HIV-1 gag-pol gene", JOURNAL OF VIROLOGY, THE AMERICAN SOCIETY FOR MICROBIOLOGY, US, vol. 74, no. 10, May 2000 (2000-05), pages 4839-4852, XP002140792, ISSN: 0022-538X
- BRAY M ET AL: "A SMALL ELEMENT FROM THE MASON-PFIZER MONKEY VIRUS GENOME MAKES HUMAN IMMUNODEFICIENCY VIRUS TYPE 1 EXPRESSION AND REPLICATION REV-INDEPENDENT", PROCEEDINGS OF THE NATIONAL ACADEMY OF SCIENCES OF USA, NATIONAL ACADEMY OF SCIENCE. WASHINGTON, US, vol. 91, February 1994 (1994-02), pages 1256-1260, XP000857729, ISSN: 0027-8424
- SRINIVASAKUMAR N ET AL: "Novel Tat-encoding bicistronic human immunodeficiency virus type 1-based gene transfer vectors for high-level transgene expression.", JOURNAL OF VIROLOGY. JUL 2000, vol. 74, no. 14, July 2000 (2000-07), pages 6659-6668, XP002287260, ISSN: 0022-538X
- FULLER M ET AL: "Helper plasmids for production of HIV-1-derived vectors.", HUMAN GENE THERAPY. 20 NOV 2001, vol. 12, no. 17, 20 November 2001 (2001-11-20), pages 2081-2093, XP002287261, ISSN: 1043-0342
- VON GEGERFELT A ET AL: "Replacement of posttranscriptional regulation in SIVmac239 generated a Rev-independent infectious virus able to propagate in rhesus peripheral blood mononuclear cells.", VIROLOGY. 9 JUN 1997, vol. 232, no. 2, 9 June 1997 (1997-06-09), pages 291-299, XP002287262, ISSN: 0042-6822
- OHLMANN T ET AL: "AN INTERNAL RIBOSOME ENTRY SEGMENT PROMOTES TRANSLATION OF THE SIMIAN IMMUNODEFICIENCY VIRUS GENOMIC RNA", JOURNAL OF BIOLOGICAL CHEMISTRY, AMERICAN SOCIETY OF BIOLOGICAL CHEMISTS, BALTIMORE, MD, US, vol. 275, no. 16, 21 April 2000 (2000-04-21), pages 11899-11906, XP002950355, ISSN: 0021-9258
- AZZOUZ MIMOUN ET AL: "Multicistronic lentiviral vector-mediated striatal gene transfer of aromatic L-amino acid decarboxylase, tyrosine hydroxylase, and GTP cyclohydrolase I induces sustained transgene expression, dopamine production, and functional improvement in a rat model of Parkinson's disease.", THE JOURNAL OF NEUROSCIENCE : THE OFFICIAL JOURNAL OF THE SOCIETY FOR NEUROSCIENCE. 1 DEC 2002, vol. 22, no. 23, 1 December 2002 (2002-12-01), pages 10302-10312, XP002287263, ISSN: 1529-2401
- BALAGGAN K S ET AL: "Stable and efficient intraocular gene transfer using pseudotyped EIAV lentiviral vectors", JOURNAL OF GENE MEDICINE, JOHN WILEY & SONS, INC, US, vol. 8, no. 3, 1 March 2006 (2006-03-01), pages 275-285, XP002442462, ISSN: 1099-498X, DOI: 10.1002/JGM.845
- K S BALAGGAN ET AL: "EIAV vector-mediated delivery of endostatin or angiostatin inhibits angiogenesis and vascular hyperpermeability in experimental CNV", GENE THERAPY, vol. 13, no. 15, 1 August 2006 (2006-08-01), pages 1153-1165, XP55007965, ISSN: 0969-7128, DOI: 10.1038/sj.gt.3302769
- NGUYEN K-L ET AL: "Codon optimization of the HIV-1 vpu and vif genes stabilizes their mRNA and allows for highly efficient Rev-independent expression", VIROLOGY, ACADEMIC PRESS,ORLANDO, US, vol. 319, no. 2, 20 February 2004 (2004-02-20), pages 163-175, XP004491664, ISSN: 0042-6822

**Description**

The present invention relates to a lentiviral vector genome, a lentiviral vector production system and a lentiviral vector particle comprising the genome, and particularly but not exclusively to its use in therapy.

Retroviral vector systems, such as lentiviral vector systems, have been proposed as a delivery system for *inter alia* the transfer of a nucleotide of interest to one or more sites of interest. Indeed, the concept of using viral vectors for gene therapy is well known (Verma and Somia (1997) Nature 389:239-242). Retrovirus genomes contain accessory genes, such as a rev gene, a *tat* gene, a *vif* gene, a *nef* gene, a *vpr* gene or an S2 gene. The deletion of such accessory genes, particularly when using retroviral vector systems in gene therapy is highly advantageous. Firstly it permits vectors to be produced without genes normally associated with disease in retroviral (e.g. HIV) infections. Secondly, the deletion of accessory genes permits the vector to package more heterologous DNA. Thirdly, genes whose function is unknown such as *dUTPase* and *S2,* may be omitted, thus reducing the risk of causing undesirable effects. We have previously taught, e.g. in our WO98/17815, how to remove many of the accessory genes. Further in our WO99/45126 we describe codon optimisation of the gag-pol sequence as a means of seeking to overcome the Rev/RRE requirement for export and to enhance RNA stability. However, the need remains to provide strategies for the provision of useful and safe viral vectors, and efficient means for their production. The present invention addresses these problems and particularly advantageously aims to provide a safer system in which viral accessory genes, such as *rev,* are not required in either the viral vector particle which is used in treatment, or in its production.

Kingsman SM, FDA/BRMA 25-26 Oct 2001 is a set of slides entitled "Safety Features In The Design, Manufacture and Clinical Monitoring of Lentivectors For The Treatment Of Parkinson's Disease, Prostate Cancer and AIDS.

Fuller M and Anson DS, Human Gene Therapy, 2001, 12:2081-2093 relates to helper plasmids for production of HIV-1 derived vectors.

In one aspect of the present invention there is provided a multicistronic vector genome derivable from a lentivirus, for use in a *rev-* independent lentiviral production system for producing a lentivirus-derived vector particle, said genome comprising a packaging signal, a heterologous open reading frame (ORF) downstream of a viral LTR and upstream of a promoter, wherein the promoter controls the expression of at least one downstream nucleotide of interest (NOI) useful in the treatment of a neurodegenerative disorder, and wherein the heterologous ORF is operably linked to the LTR; wherein the nucleic acid sequence encoding the auxiliary gene *rev* is disrupted such that said auxiliary gene is incapable of encoding the functional auxiliary protein, or is removed from the lentiviral vector genome, and wherein the Rev Response element (RRE) is disrupted such that said RRE is non-functional, or is removed from the lentiviral vector genome, and wherein the lentiviral vector is a self-inactivating lentiviral vector.

Thus, we have now found it possible to provide a viral vector which is independent of *rev* without detrimental effect on viral titre.

Preferably the vector genome is bi-, tricistronic or quadcistronic.

In one embodiment the first nucleic acid sequence is a reporter moiety or selectable marker.

In another embodiment the first nucleic acid sequence encodes a modulator gene.

Preferably the modulator gene is selected from tetracycline repressors, such as TetR, and tetracycline-controlled transactivators, such as tTA and rtTA.

Preferably the tetracycline repressor is codon optimised for expression in a mammalian cell.

Preferably the tetracycline repressor is linked to a nuclear localisation signal.

In a preferred embodiment the vector comprises more than one NOIs downstream of the internal promoter or IRES.

Preferably the NOI gives rise to a therapeutic effect.

In one embodiment one or more of the NOIs are operably linked to a tetracycline operator.

In another embodiment the vector comprises a further tetracycline repressor downstream of the internal promoter.

Preferably the vector is derived from HIV-1, HIV-2, SIV, FIV, BLV, EIAV, or visna lentivirus.

Preferably the vector is derived from a non-primate lentivirus.

Preferably the vector genome comprises a cPPT sequence.

Preferably the vector genome comprises a post-transcriptional regulatory element or a translational element.

Preferably the ATG motifs of the gag packaging signal of the wild type lentiviral vector genome are ATTG motifs.

Preferably the distance between the R regions of the vector genome is substantially the same as that in the wild type lentiviral vector.

Preferably the 3' U3 region of the vector genome includes sequence from a viral and/or a eukaryotic promoter.

Preferably the 3' U3 region of the vector genome includes a wild type viral and/or a eukaryotic promoter.

Preferably the viral promoter is an EIAV or MLV U3 region.

Thus according to the present disclosure there is provided a lentiviral vector wherein the vector has the following: the ATG motifs of the gag packaging signal of the wild type lentiviral vector are ATTG motifs; the distance between the R regions of the lentiviral vector is substantially the same as that in the wild type lentiviral vector; and the 3' U3 region of the lentiviral vector includes sequence from a viral and/or eukaryotic promoter. Preferably, the 3' U3 region may include sequence from either EIAV or MLV U3 region, or other viral or eukaryotic promoters. In one embodiment, the 3' U3 region may include wild type viral or eukaryotic promoters.

According to another aspect of the present disclosure there is provided a lentiviral vector production system for producing a lentivirus-derived vector particle, which system comprises a set of nucleic acid sequences encoding the components of the vector genome including the vector genome of the invention, Gag and Pol proteins, and Env protein or a functional substitute therefor and wherein a nucleic acid sequence encoding the auxiliary gene *rev,* is disrupted such that said auxiliary gene is incapable of encoding the functional auxiliary protein, or is not present in the lentiviral vector production system, and is not supplied *in trans,* and RRE is disrupted such that RRE is non-functional, or is not present in the lentiviral vector production system, and is not supplied *in trans.*

Preferably in the system the nucleic acid sequences encoding at least one of the auxiliary genes *vpr, vif, tat* and *nef,* or analogous auxiliary genes, from the lentivirus from which said particles are derived, are also disrupted such that said auxiliary genes are incapable of encoding the functional auxiliary proteins, or removed from the system.

Preferably the vector is derived from HIV-1, HIV-2, SIV, FIV, BLV, EIAV, or visna lentivirus.

Preferably the vector is derived from a non-primate lentivirus.

Preferably the set of nucleic acid sequences encoding the components of the vector includes three DNA constructs which encode the RNA genome of the vector, Gag and Pol proteins, and Env protein, or functional substitutes therefor.

According to another aspect of the present invention there is provided a DNA construct for use in the system of the present invention, said DNA construct encoding a packagable RNA vector genome according to the invention.

According to another aspect of the present invention there is provided a set of DNA constructs for use in the system of the invention comprising the DNA construct according to the invention, and a DNA construct encoding Gag and Pol proteins or functional substitute thereof.

Preferably the set further comprises a DNA construct encoding Env protein or a functional substitute thereof.

According to another aspect of the present invention there is provided a process for preparing a lentiviral vector particle comprising introducing a set of nucleic acid sequences or DNA constructs of the invention into a host cell, obtaining the lentiviral vector particle, and a lentiviral vector particle produced by the system or process according to the invention.

According to another aspect of the present invention there is provided a lentiviral -derived vector particle comprising an RNA genome of the vector, Gag and Pol proteins, and Env protein, or functional substitutes therefor, wherein the genome of the vector is according to the invention.

In another aspect there is provided a cell transduced with the lentiviral vector particle or DNA construct of the invention.

In another aspect of the invention there is provided a pharmaceutical composition comprising the vector, the system, a particle or a cell in accordance with the invention, together with a pharmaceutically acceptable carrier or diluent.

In another aspect of the invention there is provided use of a lentiviral vector particle or DNA construct or cell of the invention for the preparation of a medicament to deliver an NOI to a target site in need of same.

In another aspect of the invention there is provided a delivery system in the form of a lentiviral vector particle or DNA construct or cell of the invention for use in medicine.

Preferably the lentiviral vector of the present invention has a minimal viral genome.

As used herein, the term "minimal viral genome" means that the lentiviral vector has been manipulated so as to remove the non-essential elements and to retain the essential elements in order to provide the required functionality to infect, transduce and deliver a nucleotide sequence of interest to a target host cell.

Lentiviral vectors of the invention will include primate lentiviral vectors such as HIV vectors (for example, HIV-1 and HIV-2 vectors) and SIV vectors, and non-primate lentiviral vectors. Primate lentiviral vectors have a number of disadvantages which may limit their therapeutic application to certain diseases. For example, HIV-1 has the disadvantage of being a human pathogen carrying potentially oncogenic proteins and sequences. There is the risk that introduction of vector particles produced in packaging cells which express HIV gag-pol will introduce these proteins into an individual leading to seroconversion. Therefore, in a particularly preferred embodiment, the lentiviral vector will be a non-primate lentiviral vector, such as EIAV, FIV, BIV, CAEV or MVV, with EIAV being especially preferred. Non-primate lentiviral-based vectors do not introduce HIV proteins into individuals.

Preferably the lentivirus vector is an EIAV vector.

DETAILED ASPECTS OF THE INVENTION

Various preferred features and embodiments of the present invention will now be described by way of non-limiting example. Although in general the techniques mentioned herein are well known in the art, reference may be made in particular to Sambrook et al., Molecular Cloning, A Laboratory Manual (1989) and Ausubel et al., Short Protocols in Molecular Biology (1999) 4th Ed, John Wiley & Sons, Inc (as well as the complete version Current Protocols in Molecular Biology).

RETROVIRUSES AND LENTIVIRUSES

As previously mentioned, the concept of using viral vectors for gene therapy is well known (Verma and Somia (1997) Nature 389:239-242).

There are many retroviruses. For the present application, the term "retrovirus" includes: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other retroviridiae including lentiviruses.

A detailed list of retroviruses may be found in Coffin et al ("Retroviruses" 1997 Cold Spring Harbour Laboratory Press Eds: JM Coffin, SM Hughes, HE Varmus pp 758-763).

Lentiviruses also belong to the retrovirus family, but they can infect both dividing and non-dividing cells (Lewis et al (1992) EMBO J. 3053-3058).

The lentivirus group can be split into "primate" and "non-primate". Examples of primate lentiviruses include the human immunodeficiency virus (HIV), the causative agent of human acquired immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

Details on the genomic structure of some lentiviruses may be found in the art. By way of example, details on HIV and EIAV may be found from the NCBI Genbank database (i.e. Genome Accession Nos. AF033819 and AF033820 respectively). Details of HIV variants may also be found at http://hiv-web.lanl.gov. Details of EIAV variants may be found through http://www.ncbi.nlm.nih.gov.

During the process of infection, a retrovirus initially attaches to a specific cell surface receptor. On entry into the susceptible host cell, the retroviral RNA genome is then copied to DNA by the virally encoded reverse transcriptase which is carried inside the parent virus. This DNA is transported to the host cell nucleus where it subsequently integrates into the host genome. At this stage, it is typically referred to as the provirus. The provirus is stable in the host chromosome during cell division and is transcribed like other cellular genes. The provirus encodes the proteins and other factors required to make more virus, which can leave the cell by a process sometimes called "budding".

Each retroviral genome comprises genes called *gag, pol* and *env* which code for virion proteins and enzymes. These genes are flanked at both ends by regions called long terminal repeats (LTRs). The LTRs are responsible for proviral integration, and transcription. They also serve as enhancer-promoter sequences. In other words, the LTRs can control the expression of the viral genes. Encapsidation of the retroviral RNAs occurs by virtue of a *psi* sequence located at the 5' end of the viral genome.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5'end of the RNA. The sizes of the three elements can vary considerably among different retroviruses.

For the viral genome, the site of transcription initiation is at the boundary between U3 and R in the left hand side LTR and the site of poly (A) addition (termination) is at the boundary between R and U5 in the right hand side LTR. U3 contains most of the transcriptional control elements of the provirus, which include the promoter and multiple enhancer sequences responsive to cellular and in some cases, viral transcriptional activator proteins. Some retroviruses have any one or more of the following genes that code for proteins that are involved in the regulation of gene expression: *tat, rev, tax* and *rex.*

With regard to the structural genes *gag, pol* and *env* themselves, *gag* encodes the internal structural protein of the virus. Gag protein is proteolytically processed into the mature proteins MA (matrix), CA (capsid) and NC (nucleocapsid). The *pol* gene encodes the reverse transcriptase (RT), which contains DNA polymerase, associated RNase H and integrase (IN), which mediate replication of the genome. The *env* gene encodes the surface (SU) glycoprotein and the transmembrane (TM) protein of the virion, which form a complex that interacts specifically with cellular receptor proteins. This interaction leads ultimately to infection by fusion of the viral membrane with the cell membrane.

Retroviruses may also contain "additional" genes which code for proteins other than gag, pol and env. Examples of additional genes include in HIV, one or more of *vif, vpr, vpx, vpu, tat, rev* and *nef*. EIAV has (amongst others) the additional gene *S2.*

Proteins encoded by additional genes serve various functions, some of which may be duplicative of a function provided by a cellular protein. In EIAV, for example, *tat* acts as a transcriptional activator of the viral LTR. It binds to a stable, stem-loop RNA secondary structure referred to as TAR. Rev regulates and co-ordinates the expression of viral genes through rev-response elements (RRE). The mechanisms of action of these two proteins are thought to be broadly similar to the analogous mechanisms in the primate viruses. The function of S2 is unknown. In addition, an EIAV protein, Ttm, has been identified that is encoded by the first exon of *tat* spliced to the *env* coding sequence at the start of the transmembrane protein.

The lentiviral vector of the present invention is a recombinant lentiviral vector.

As used herein, the term "recombinant lentiviral vector" (RLV) refers to a vector with sufficient genetic information to allow packaging of an RNA genome, in the presence of packaging components, into a viral particle capable of infecting and transducing a target cell. Infection and transduction of a target cell includes reverse transcription and integration into the target cell genome. The RLV carries non-viral coding sequences which are to be delivered by the vector to the target cell. An RLV is incapable of independent replication to produce infectious retroviral particles within the final target cell. Usually the RLV lacks a functional *gag-pol* and/or *env* gene and/or other genes essential for replication. The vector of the present invention may be configured as a split-intron vector. An example of a split intron vector is described WO 99/15683.

Preferably the recombinant lentiviral vector (RLV) of the present invention has a minimal viral genome.

As used herein, the term "minimal viral genome" means that the viral vector has been manipulated so as to remove the non-essential elements and to retain the essential elements in order to provide the required functionality to infect, transduce and deliver a nucleotide sequence of interest to a target host cell. Further details on this strategy can be found in our WO98/17815.

A minimal lentiviral genome for use in the present invention will therefore comprise (5') R - U5 - one or more first nucleotide sequences - (regulatory element - NOI)$_n$ - U3-R (3'). However, the plasmid vector used to produce the lentiviral genome within a host cell/packaging cell will also include transcriptional regulatory control sequences operably linked to the lentiviral genome to direct transcription of the genome in a host cell/packaging cell. These regulatory sequences may be the natural sequences associated with the transcribed retroviral sequence, i.e. the 5' U3 region, or they may be a heterologous promoter such as another viral promoter, for example the CMV promoter. Some lentiviral genomes require additional sequences for efficient virus production. For example, in the case of HIV, *rev* and RRE sequence are preferably included. However the requirement for *rev* and RRE is eliminated in the present invention. Requirement for *rev* and RRE is further reduced or eliminated by codon optimisation. Further details of this strategy can be found in our WO01/79518.

The vector may have at least one of the following: the ATG motifs of the gag packaging signal of the wild type viral vector are ATTG motifs; the distance between the R regions of the viral vector is substantially the same as that in the wild type viral vector; the 3' U3 region of the viral vector includes sequence from an MLV U3 region; and a nucleotide sequence operably linked to the viral LTR and wherein said nucleotide sequence is upstream of an internal promoter and wherein said nucleotide sequence preferably encodes a polypeptide or fragment thereof.

In a preferred embodiment, the system used in the present invention is based on a so-called "minimal" system in which some or all of the additional genes have be removed.

In the present invention, the lentiviral vector is a self-inactivating vector. In other words the viral promoter is a self-inactivating LTR.

By way of example, self-inactivating retroviral vectors have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive provirus (Yu et al 1986 Proc Natl Acad Sci 83: 3194-3198; Dougherty and Temin 1987 Proc Natl Acad Sci 84: 1197-1201; Hawley et al 1987 Proc Natl Acad Sci 84: 2406-2410; Yee et al 1987 Proc Natl Acad Sci 91: 9564-9568). However, any promoter(s) internal to the LTRs in such vectors will still be transcriptionally active. This strategy has been employed to eliminate effects of the enhancers and promoters in the viral LTRs on transcription from internally placed genes. Such effects include increased transcription (Jolly et al 1983 Nucleic Acids Res 11: 1855-1872) or suppression of transcription (Emerman and Temin 1984 Cell 39: 449-467). This strategy can also be used to eliminate downstream transcription from the 3' LTR into genomic DNA (Herman and Coffin 1987 Science 236: 845-848). This is of particular concern in human gene therapy where it is of critical importance to prevent the adventitious activation of an endogenous oncogene.

In one embodiment of the present invention the lentiviral vector is derived from a non-primate lentivirus. The non-primate lentivirus may be any member of the family of lentiviridae which does not naturally infect a primate and may include a feline immunodeficiency virus (FIV), a bovine immunodeficiency virus (BIV), a caprine arthritis encephalitis virus (CAEV), a Maedi visna virus (MVV) or an equine infectious anaemia virus (EIAV). Preferably the lentivirus is an EIAV. Equine infectious anaemia virus infects all equidae resulting in plasma viremia and thrombocytopenia (Clabough, et al. 1991. J Virol. 65:6242-51). Virus replication is thought to be controlled by the process of maturation of monocytes into macrophages.

EIAV has the simplest genomic structure of the lentiviruses and is particularly preferred for use in the present invention. In addition to the *gag, pol* and *env* genes EIAV encodes three other genes: *tat, rev,* and *S2. Tat* acts as a transcriptional activator of the viral LTR (Derse and Newbold1993 Virology. 194:530-6; Maury, et al 1994 Virology. 200:632-42) and Rev regulates and coordinates the expression of viral genes through rev-response elements (RRE) (Martarano et al 1994 J Virol. 68:3102-11). The mechanisms of action of these two proteins are thought to be broadly similar to the analogous mechanisms in the primate viruses (Martano et al ibid). The function of S2 is unknown. In addition, an EIAV protein, Ttm, has been identified that is encoded by the first exon of *tat* spliced to the *env* coding sequence at the start of the transmembrane protein.

In our WO99/32646 we give details of features which may advantageously be applied to the present invention. In particular, it will be appreciated that the non-primate lentivirus genome (1) preferably comprises a deleted *gag* gene wherein the deletion in gag removes one or more nucleotides downstream of about nucleotide 350 or 354 of the *gag* coding sequence; (2) preferably has one or more accessory genes absent from the non-primate lentivirus genome; (3) preferably lacks the *tat* gene but includes the leader sequence between the end of the 5' LTR and the ATG of *gag*; and (4) combinations of (1), (2) and (3). In a particularly preferred embodiment the lentiviral vector comprises all of features (1) and (2) and (3).

Use may also be made of a lentiviral, e.g. non-primate, vector wherein the vector has at least one of the following: the ATG motifs of the gag packaging signal of the wild type lentiviral vector are ATTG motifs; the distance between the R regions of the lentiviral vector is substantially the same as that in the wild type lentiviral vector; the 3' U3 region of the lentiviral vector includes sequence from an MLV U3 region; and a nucleotide sequence operably linked to the viral LTR and wherein said nucleotide sequence is upstream of an internal promoter and wherein said nucleotide sequence preferably encodes a polypeptide or fragment thereof.

It will be appreciated that the present invention may be used to deliver a nucleotide of interest (NOI) to a target cell. In a further preferred embodiment of the first aspect of the invention more than one nucleotides of interest (NOI) is introduced into the vector at the cloning site. In the present invention, the NOI is useful in treatment of a neurodegenerative disease.

DELIVERY SYSTEMS

Lentiviral vector systems have been proposed as a delivery system for *inter alia* the transfer of a NOI to one or more sites of interest. The transfer can occur *in vitro, ex vivo, in vivo,* or combinations thereof. Lentiviral vector systems have even been exploited to study various aspects of the retrovirus life cycle, including receptor usage, reverse transcription and RNA packaging (reviewed by Miller, 1992 Curr Top Microbiol Immunol 158:1-24).

A recombinant lentiviral vector particle is capable of transducing a recipient cell with an NOI. Once within the cell the RNA genome from the vector particle is reverse transcribed into DNA and integrated into the DNA of the recipient cell.

As used herein, the term "vector genome" refers to both to the RNA construct present in the retroviral vector particle and the integrated DNA construct. The term also embraces a separate or isolated DNA construct capable of encoding such an RNA genome. A lentiviral genome should comprise at least one component part derivable from a lentivirus. The term "derivable" is used in its normal sense as meaning a nucleotide sequence or a part thereof which need not necessarily be obtained from a lentivirus but instead could be derived therefrom. By way of example, the sequence may be prepared synthetically or by use of recombinant DNA techniques.

The viral vector genome is preferably "replication defective" by which we mean that the genome does not comprise sufficient genetic information alone to enable independent replication to produce infectious viral particles within the recipient cell. In a preferred embodiment, the genome lacks a functional *env, gag* or *pol* gene. In a highly preferred embodiment the genome lacks *env, gag* and *pol* genes.

The viral vector genome may comprise some of the long terminal repeats (LTRs).

The sequence may also comprise or act as an enhancer-promoter sequence.

The viral vector genome of the first aspect of the disclosure may be provided as a kit of parts. For example, the kit may comprise (i) a plasmid or plasmids containing the NOIs and internal regulatory element sequence(s); and (ii) a retroviral genome construct with suitable restriction enzyme recognition sites for cloning the NOIs and regulatory element(s) into the viral genome.

It is known that the separate expression of the components required to produce a lentiviral vector particle on separate DNA sequences cointroduced into the same cell will yield lentiviral particles carrying defective lentiviral genomes that carry therapeutic genes. This cell is referred to as the producer cell (see below).

There are two common procedures for generating producer cells. In one, the sequences encoding retroviral Gag, Pol and Env proteins are introduced into the cell and stably integrated into the cell genome; a stable cell line is produced which is referred to as the packaging cell line. The packaging cell line produces the proteins required for packaging retroviral RNA but it cannot bring about encapsidation due to the lack of a *psi* region. However, when a vector genome according to the first aspect of the invention (having a *psi* region) is introduced into the packaging cell line, the helper proteins can package the *psi*-positive recombinant vector RNA to produce the recombinant virus stock. This can be used to transduce the NOI into recipient cells. The recombinant virus whose genome lacks all genes required to make viral proteins can infect only once and cannot propagate. Hence, the NOI is introduced into the host cell genome without the generation of potentially harmful retrovirus. A summary of the available packaging lines is presented in "Retroviruses" (1997 Cold Spring Harbour Laboratory Press Eds: JM Coffin, SM Hughes, HE Varmus pp 449).

The present invention also provides a packaging cell line comprising a viral vector genome of the first aspect of the invention. For example, the packaging cell line may be transduced with a viral vector system comprising the genome or transfected with a plasmid carrying a DNA construct capable of encoding the RNA genome. The present invention also provides a lentiviral vector particle produced by such a cell.

The second approach is to introduce the three different DNA sequences that are required to produce a retroviral vector particle i.e. the *env* coding sequences, the *gag-pol* coding sequence and the defective retroviral genome containing one or more NOIs into the cell at the same time by transient transfection and the procedure is referred to as transient triple transfection (Landau & Littman 1992; Pear et al 1993). The triple transfection procedure has been optimised (Soneoka et al 1995; Finer et al 1994). WO 94/29438 describes the production of producer cells *in vitro* using this multiple DNA transient transfection method.

The components of the viral system which are required to complement the vector genome may be present on one or more "producer plasmids" for transfecting into cells.

The present invention also provides a vector system, comprising

(i) a viral genome according to the first aspect of the invention;
(ii) a nucleotide sequence coding for lentiviral gag and pol proteins;
(iii) nucleotide sequences encoding other essential viral packaging components not encoded by the nucleotide sequence of ii). In a preferred embodiment, the nucleotide sequence of (iii) is capable of encoding an env protein. The present invention also provides a cell transfected with such a vector system and a lentiviral vector particle produced by such a cell. Preferably the gag-pol sequence is codon optimised for use in the particular producer cell (see below).

The env protein encoded by the nucleotide sequence of iii) may be a homologous retroviral or lentiviral env protein. Alternatively, it may be a heterologous env, or an env from a non-retro or lentivirus (see below under "pseudotyping").

The term "viral vector system" is used generally to mean a kit of parts which can be used when combined with other necessary components for viral particle production to produce viral particles in host cells. For example, the retroviral vector genome may lack one or more of the genes needed for viral replication. This may be combined in a kit with a further complementary nucleotide sequence or sequences, for example on one or more producer plasmids. By cotransfection of the genome together with the producer plasmid(s), the necessary components should be provided for the production of infectious viral particles.

Alternatively, the complementary nucleotide sequence(s) may be stably present within a packaging cell line that is included in the kit.

The present invention also relates to a lentiviral vector system which is capable of delivering an RNA genome to a recipient cell as defined in the claims, wherein the genome is longer than the wild type genome of the retrovirus. The vector system may, for example, be an ELAV vector system.

Preferably the RNA genome of the vector system has up to 5%, more preferably up to 10% more bases than the wild-type genome. Preferably the RNA genome is about 10% longer than the wild-type genome. For example, wild type EIAV comprises an RNA genome of approximately 8 kb. An EIAV vector system of the present invention may have an RNA genome of up to (preferably about) 8.8 kb.

The lentiviral vector system of the present invention is a self-inactivating (SIN) vector system.

By way of example, self-inactivating retroviral vector systems have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive provirus. However, any promoter(s) internal to the LTRs in such vectors will still be transcriptionally active. This strategy has been employed to eliminate effects of the enhancers and promoters in the viral LTRs on transcription from internally placed genes. Such effects include increased transcription or suppression of transcription. This strategy can also be used to eliminate downstream transcription from the 3' LTR into genomic DNA. This is of particular concern in human gene therapy where it may be important to prevent the adventitious activation of an endogenous oncogene.

Preferably a recombinase assisted mechanism is used which facilitates the production of high titre regulated lentiviral vectors from the producer cells of the present invention.

As used herein, the term "recombinase assisted system" includes but is not limited to a system using the Cre recombinase / loxP recognition sites of bacteriophage P1 or the site-specific FLP recombinase of *S. cerevisiae* which catalyses recombination events between 34 bp FLP recognition targets (FRTs).

The site-specific FLP recombinase of *S. cerevisiae* which catalyses recombination events between 34 bp FLP recognition targets (FRTs) has been configured into DNA constructs in order to generate high level producer cell lines using recombinase-assisted recombination events (Karreman et al (1996) NAR 24:1616-1624). A similar system has been developed using the Cre recombinase / loxP recognition sites of bacteriophage P1 (Vanin et al (1997) J. Virol 71:7820-7826). This was configured into a lentiviral genome such that high titre lentiviral producer cell lines were generated.

By using producer/packaging cell lines, it is possible to propagate and isolate quantities of retroviral vector particles (e.g. to prepare suitable titres of the retroviral vector particles) for subsequent transduction of, for example, a site of interest (such as adult brain tissue). Producer cell lines are usually better for large scale production or vector particles.

Transient transfection has numerous advantages over the packaging cell method. In this regard, transient transfection avoids the longer time required to generate stable vector-producing cell lines and is used if the vector genome or retroviral packaging components are toxic to cells. If the vector genome encodes toxic genes or genes that interfere with the replication of the host cell, such as inhibitors of the cell cycle or genes that induce apoptosis, it may be difficult to generate stable vector-producing cell lines, but transient transfection can be used to produce the vector before the cells die. Also, cell lines have been developed using transient infection that produce vector titre levels that are comparable to the levels obtained from stable vector-producing cell lines (Pear et al 1993, PNAS 90:8392-8396).

Producer cells/packaging cells can be of any suitable cell type. Producer cells are generally mammalian cells but can be, for example, insect cells.

As used herein, the term "producer cell" or "vector producing cell" refers to a cell which contains all the elements necessary for production of lentiviral vector particles.

Preferably, the producer cell is obtainable from a stable producer cell line.

Preferably, the producer cell is obtainable from a derived stable producer cell line.

Preferably, the producer cell is obtainable from a derived producer cell line.

As used herein, the term "derived producer cell line" is a transduced producer cell line which has been screened and selected for high expression of a marker gene. Such cell lines support high level expression from the retroviral genome. The term "derived producer cell line" is used interchangeably with the term "derived stable producer cell line" and the term "stable producer cell line.

Preferably the derived producer cell line includes but is not limited to a retroviral and/or a lentiviral producer cell.

Preferably the derived producer cell line is an HIV or EIAV producer cell line, more preferably an EIAV producer cell line.

Preferably the envelope protein sequences, and nucleocapsid sequences are all stably integrated in the producer and/or packaging cell. However, one or more of these sequences could also exist in episomal form and gene expression could occur from the episome.

As used herein, the term "packaging cell" refers to a cell which contains those elements necessary for production of infectious recombinant virus which are lacking in the RNA genome. Typically, such packaging cells contain one or more producer plasmids which are capable of expressing viral structural proteins (such as codon optimised *gag-pol* and *env*) but they do not contain a packaging signal.

The term "packaging signal" which is referred to interchangeably as "packaging sequence" or *"psi"* is used in reference to the non-coding, *cis*-acting sequence required for encapsidation of retroviral RNA strands during viral particle formation. In HIV-1, this sequence has been mapped to loci extending from upstream of the major splice donor site (SD) to at least the *gag* start codon.

Packaging cell lines suitable for use with the above-described vector constructs may be readily prepared (see also WO 92/05266), and utilised to create producer cell lines for the production of retroviral vector particles. As already mentioned, a summary of the available packaging lines is presented in "Retroviruses" (as above).

Also as discussed above, simple packaging cell lines, comprising a provirus in which the packaging signal has been deleted, have been found to lead to the rapid production of undesirable replication competent viruses through recombination. In order to improve safety, second generation cell lines have been produced wherein the 3'LTR of the provirus is deleted. In such cells, two recombinations would be necessary to produce a wild type virus. A further improvement involves the introduction of the *gag-pol* genes and the *env* gene on separate constructs so-called third generation packaging cell lines. These constructs are introduced sequentially to prevent recombination during transfection.

Preferably, the packaging cell lines are second generation packaging cell lines.

Preferably, the packaging cell lines are third generation packaging cell lines.

In these split-construct, third generation cell lines, a further reduction in recombination may be achieved by changing the codons. This technique, based on the redundancy of the genetic code, aims to reduce homology between the separate constructs, for example between the regions of overlap in the *gag-pol* and *env* open reading frames.

The packaging cell lines are useful for providing the gene products necessary to encapsidate and provide a membrane protein for a high titre vector particle production. The packaging cell may be a cell cultured *in vitro* such as a tissue culture cell line. Suitable cell lines include but are not limited to mammalian cells such as murine fibroblast derived cell lines or human cell lines. Preferably the packaging cell line is a human cell line, such as for example: HEK293, 293-T, TE671, HT1080.

Alternatively, the packaging cell may be a cell derived from the individual to be treated such as a monocyte, macrophage, blood cell or fibroblast. The cell may be isolated from an individual and the packaging and vector components administered *ex vivo* followed by re-administration of the autologous packaging cells.

In more detail, the packaging cell may be an *in vivo* packaging cell in the body of an individual to be treated or it may be a cell cultured *in vitro* such as a tissue culture cell line. Suitable cell lines include mammalian cells such as murine fibroblast derived cell lines or human cell lines. Preferably the packaging cell line is a human cell line, such as for example: 293 cell line, HEK293, 293-T, TE671, HT1080.

Alternatively, the packaging cell may be a cell derived from the individual to be treated such as a monocyte, macrophage, stem cells, blood cell or fibroblast. The cell may be isolated from an individual and the packaging and vector components administered *ex vivo* followed by re-administration of the autologous packaging cells. Alternatively the packaging and vector components may be administered to the packaging cell *in vivo*. Methods for introducing lentiviral packaging and vector components into cells of an individual are known in the art. For example, one approach is to introduce the different DNA sequences that are required to produce a lentiviral vector particle e.g. the *env* coding sequence, the *gag-pol* coding sequence and the defective lentiviral genome into the cell simultaneously by transient triple transfection (Landau & Littman 1992 J. Virol. 66, 5110; Soneoka et al 1995 Nucleic Acids Res 23:628-633).

In one embodiment the vector configurations of the present invention use as their production system, three transcription units expressing a genome, the *gag-pol* components and an envelope. The envelope expression cassette may include one of a number of envelopes such as VSV-G or various murine retrovirus envelopes such as 4070A.

Conventionally these three cassettes would be expressed from three plasmids transiently transfected into an appropriate cell line such as 293T or from integrated copies in a stable producer cell line. An alternative approach is to use another virus as an expression system for the three cassettes, for example baculovirus or adenovirus. These are both nuclear expression systems. To date the use of a poxvirus to express all of the components of a lentiviral vector system has not been described. In particular, given the unusual codon usage of lentiviruses and their requirement for RNA handling systems such as the rev/RRE system it has not been clear whether incorporation of all three cassettes and their subsequent expression in a vector that expresses in the cytoplasm rather than the nucleus is feasible. Until now the possibility remained that key nuclear factors and nuclear RNA handling pathways would be required for expression of the vector components and their function in the gene delivery vehicle. Here we describe such a system and show that lentiviral components can be made in the cytoplasm and that they assemble into functional gene delivery systems. The advantage of this system is the ease with which poxviruses can be handled, the high expression levels and the ability to retain introns in the vector genomes.

The lentiviral vector particle according to the invention will also be capable of transducing cells which are slowly-dividing, and which non-lentiviruses such as MLV would not be able to efficiently transduce. Slowly-dividing cells divide once in about every three to four days including certain tumour cells. Although tumours contain rapidly dividing cells, some tumour cells especially those in the centre of the tumour, divide infrequently. Alternatively the target cell may be a growth-arrested cell capable of undergoing cell division such as a cell in a central portion of a tumour mass or a stem cell such as a haematopoietic stem cell or a CD34-positive cell. As a further alternative, the target cell may be a precursor of a differentiated cell such as a monocyte precursor, a CD33-positive cell, or a myeloid precursor. As a further alternative, the target cell may be a differentiated cell such as a neuron, astrocyte, glial cell, microglial cell, macrophage, monocyte, epithelial cell, endothelial cell or hepatocyte. Target cells may be transduced either *in vitro* after isolation from a human individual or may be transduced directly *in vivo.*

It is highly desirable to use high-titre virus preparations in both experimental and practical applications. Techniques for increasing viral titre include using a *psi* plus packaging signal as discussed above and concentration of viral stocks.

As used herein, the term "high titre" means an effective amount of a lentiviral vector or particle which is capable of transducing a target site such as a cell.

As used herein, the term "effective amount" means an amount of a regulated lentiviral vector or vector particle which is sufficient to induce expression of the NOIs at a target site.

A high-titre viral preparation for a producer/packaging cell is usually of the order of $10^5$ to $10^7$ retrovirus particles per ml. For transduction in tissues such as the brain, it is necessary to use very small volumes, so the viral preparation is concentrated by ultracentrifugation. The resulting preparation should have at least $10^8$ t.u./ml, preferably from $10^8$ to $10^9$ t.u./ml, more preferably at least $10^9$ t.u./ml. (The titer is expressed in transducing units per ml (t.u./ml) as titred on a standard D17 cell line).

The NOIs are operatively linked to one or more promoter/enhancer elements.

Preferably the promoter is a strong promoter such as CMV. The promoter may be a regulated promoter. The promoter may be tissue-specific.

The presence of a sequence termed the central polypurine tract (cPPT) may improve the efficiency of gene delivery to non-dividing cells. This *cis*-acting element is located, for example, in the EIAV polymerase coding region element. Preferably the genome of the present invention comprises a cPPT sequence.

Preferably the viral genome comprises a post-translational regulatory element. For example, the genome may comprise an element such as the woodchuck hepatitis virus posttranscriptional regulatory element (WPRE).

In addition, or in the alternative, the viral genome may comprise a translational enhancer.

PSEUDOTYPING

In the design of retroviral vector systems it is desirable to engineer particles with different target cell specificities to the native virus, to enable the delivery of genetic material to an expanded or altered range of cell types. One manner in which to achieve this is by engineering the virus envelope protein to alter its specificity. Another approach is to introduce a heterologous envelope protein into the vector particle to replace or add to the native envelope protein of the virus.

The term pseudotyping means incorporating in at least a part of, or substituting a part of, or replacing all of, an *env* gene of a viral genome with a heterologous *env* gene, for example an *env* gene from another virus. Pseudotyping is not a new phenomenon and examples may be found in WO 99/61639, WO-A-98/05759, WO-A-98/05754, WO-A-97/17457, WO-A-96/09400, WO-A-91/00047 and Mebatsion et al 1997 Cell 90, 841-847.

In a preferred embodiment of the present invention the vector system is pseudotyped with a gene encoding at least part of the rabies G protein. In a further preferred embodiment of the present invention the vector system is pseudotyped with a gene encoding at least part of the VSV-G protein.

In more detail, the term "lentiviral particle" refers to the packaged lentiviral vector, that is preferably capable of binding to and entering target cells. The components of the particle, as already discussed for the vector, may be modified with respect to the wild type lentivirus. For example, the Env proteins in the proteinaceous coat of the particle may be genetically modified in order to alter their targeting specificity or achieve some other desired function.

Preferably, the viral vector preferentially transduces a certain cell type or cell types.

More preferably, the viral vector is a targeted vector, that is it has a tissue tropism which is altered compared to the native virus, so that the vector is targeted to particular cells.

For lentiviral vectors, this may be achieved by modifying the Env protein. The Env protein of the retroviral secondary vector needs to be a non-toxic envelope or an envelope which may be produced in non-toxic amounts within the primary target cell, such as for example a MMLV amphotropic envelope or a modified amphotropic envelope. The safety feature in such a case is preferably the deletion of regions or sequence homology between lentiviral components.

Preferably the envelope is one which allows transduction of human cells. Examples of suitable *env* genes include, but are not limited to, VSV-G, a MLV amphotropic *env* such as the 4070A env, the RD114 feline leukaemia virus *env* or haemagglutinin (HA) from an influenza virus. The Env protein may be one which is capable of binding to a receptor on a limited number of human cell types and may be an engineered envelope containing targeting moieties. The *env* and *gag-pol* coding sequences are transcribed from a promoter and optionally an enhancer active in the chosen packaging cell line and the transcription unit is terminated by a polyadenylation signal. For example, if the packaging cell is a human cell, a suitable promoter-enhancer combination is that from the human cytomegalovirus major immediate early (hCMV-MIE) gene and a polyadenylation signal from SV40 virus may be used. Other suitable promoters and polyadenylation signals are known in the art.

OPEN READING FRAME (ORF)

The first nucleic acid sequence is a sequence which is capable of increasing the levels of genomic RNA which is available for packaging in the absence of Rev for example compared with the situation in which the first nucleic acid sequence is not present.

The first nucleic acid sequence is an open reading frame (ORF) which increases the level of genomic RNA available for packaging in the absence of Rev. By ORF we include a series of codon triplets that include a 5' initiation codon (which may be a Kozac sequence) running through to a termination codon, and representing a putative or known gene. However any nucleic acid sequence which increases the level of genomic RNA for packaging may be used.

Thus the present invention relates to a multi-cistronic vector genome which has an ORF operably linked to the viral LTR, and upstream of an internal promoter.

When the vector genome is used with packaging components in which Rev is absent, such as through use of a codon optimised *gag-pol* gene, it is possible to produce a totally minimal vector without any accessory viral genes, either in the producer or target cell.

Whilst not wishing to be bound by any theory we believe that the in the system of the invention the transcript of the first nucleic acid sequence is recognised by a cell as being a "true" mRNA and therefore worthy of export from the nucleus. In the absence of such a sequence the mRNA appears to be degraded in the nucleus, through for example "nonsense mediated decay", before it is transported to the cytoplasm and subsequently packaged. The accessory protein Rev appears to allow bypass of this surveillance system and thus maintains viral titres where the vector genome would otherwise be degraded. The present invention provides an alternative and safer way of maintaining viral titres.

It should be noted that a genome in which a portion of the ORF was reversed resulted in differing Rev-dependencies of the parental and derivative genomes. Whilst not wishing to be bound by any theory we believe this is due to the presence of an ORF in the parental (Rev independent) genome which was destroyed in the derivative (Rev-dependent) genome. Thus, the ORF should be in the correct, and not reverse, orientation.

Usefully the ORF may be between about 0.6 to 4 kb, for example about 0.65 or 0.7 to 3.1 or 3.0 kb.

Preferably the first nucleic acid sequence may be a selection gene also referred to as selectable marker. Typical selection genes encode proteins that confer resistance to antibiotics and other toxins, e.g. ampicillin, neomycin, methotrexate or tetracycline, complement auxotrophic deficiencies, or supply critical nutrients not available from complex media. Further details on suitable first nucleic acid sequences are given below in the sections on reporter genes.

It is also possible for the first nucleic acid sequence to be the nucleotide of interest (NOI) and further details on NOIs are given below.

## TETRACYCLINE-REGULATED EXPRESSION SYSTEMS

In another preferred embodiment the first nucleic acid sequence is a Tet repressor gene, optionally linked to a nuclear localisation signal (NLS). Whilst not wishing to be bound by any theory, we believe that the addition of an NLS to the carboxy terminus increases the concentration of TetR in the nucleus since the amino terminus of TetR is important for binding of the tetracycline operator (tetO). Thus, in this embodiment at least one of the downstream NOIs will be the tetO. In one preferred embodiment, at least a second copy of the Tet repressor gene is present downstream of an IRES or internal promoter.

In a further preferred embodiment, the Tet repressor gene is codon optimised for expressed in a mammalian system. Further details on codon optimisation are given below.

Thus, we describe an improved TetR gene sequence for tetracycline-regulated expression in mammalian cells obtained by codon optimisation. Tetracycline-regulated expression systems have wide-ranging utility in many applications where inducible gene expression is advantageous or essential. The improvement in gene expression which may arise from codon optimising the effector protein may significantly enhance the efficiency of the system by facilitating more rapid TetR-mediated expression.

We also describe altering the 5' sequence of the TetR gene such that the first AUG is in a more favourable context for expression, thereby improving the fidelity of initiation by eukaryotic ribosomes. In one embodiment the 5' sequence is 5' gccGCCACCAUGG 3'. (The G is position +4 would change the codon from serine or require insertion of an additional amino acid residue.)

We further describe incorporation of an NLS increasing local concentration of TetR in the nucleus.

## CODON OPTIMISATION

Codon optimisation has previously been described in WO99/41397. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

Many viruses, including HIV and other lentiviruses, use a large number of rare codons and by changing these to correspond to commonly used mammalian codons, increased expression of the packaging components in mammalian producer cells can be achieved. Codon usage tables are known in the art for mammalian cells, as well as for a variety of other organisms.

Codon optimisation has a number of other advantages. By virtue of alterations in their sequences, the nucleotide sequences encoding the packaging components of the viral particles required for assembly of viral particles in the producer cells/packaging cells have RNA instability sequences (INS) eliminated from them. At the same time, the amino acid sequence coding sequence for the packaging components is retained so that the viral components encoded by the sequences remain the same, or at least sufficiently similar that the function of the packaging components is not compromised. Codon optimisation also overcomes the Rev/RRE requirement for export, rendering optimised sequences Rev independent. Codon optimisation also reduces homologous recombination between different constructs within the vector system (for example between the regions of overlap in the gag-pol and env open reading frames). The overall effect of codon optimisation is therefore a notable increase in viral titre and improved safety.

In one embodiment only codons relating to INS are codon optimised. However, in a much more preferred and practical embodiment, the sequences are codon optimised in their entirety, with the exception of the sequence encompassing the frameshift site.

The *gag-pol* gene comprises two overlapping reading frames encoding gag and pol proteins respectively. The expression of both proteins depends on a frameshift during translation. This frameshift occurs as a result of ribosome "slippage" during translation. This slippage is thought to be caused at least in part by ribosome-stalling RNA secondary structures. Such secondary structures exist downstream of the frameshift site in the *gag-pol* gene. For HIV, the region of overlap extends from nucleotide 1222 downstream of the beginning of *gag* (wherein nucleotide 1 is the A of the *gag* ATG) to the end of *gag* (nt 1503). Consequently, a 281 bp fragment spanning the frameshift site and the overlapping region of the two reading frames is preferably not codon optimised. Retaining this fragment will enable more efficient expression of the gag-pol proteins.

For EIAV the beginning of the overlap has been taken to be nt 1262 (where nucleotide 1 is the A of the *gag* ATG). The end of the overlap is at 1461 bp. In order to ensure that the frameshift site and the *gag-pol* overlap are preserved, the wild type sequence has been retained from nt 1156 to 1465.

Derivations from optimal codon usage may be made, for example, in order to accommodate convenient restriction sites, and conservative amino acid changes may be introduced into the gag-pol proteins.

In a highly preferred embodiment, codon optimisation was based on lightly expressed mammalian genes. The third and sometimes the second and third base may be changed.

Due to the degenerate nature of the Genetic Code, it will be appreciated that numerous *gag-pol* sequences can be achieved by a skilled worker. Also there are many retroviral variants described which can be used as a starting point for generating a codon optimised *gag-pol* sequence. Lentiviral genomes can be quite variable. For example there are many quasi-species of HIV-1 which are still functional. This is also the case for EIAV. These variants may be used to enhance particular parts of the transduction process. Examples of HIV-1 variants may be found at http://hiv-web.lanl.gov. Details of EIAV clones may be found at the NCBI database: http://www.ncbi.nlm.nih.gov.

The strategy for codon optimised *gag-pol* and TetR sequences can be used in relation to any retrovirus. This would apply to all lentiviruses, including EIAV, FIV, BIV, CAEV, VMR, SIV, HIV-1 and HIV-2. In addition this method could be used to increase expression of genes from HTLV-1, HTLV-2, HFV, HSRV and human endogenous retroviruses (HERV), MLV and other retroviruses.

Codon optimisation can render *gag-pol* expression Rev independent. In order to enable the use of anti-*rev* or RRE factors in the retroviral vector, however, it would be necessary to render the viral vector generation system totally Rev/RRE independent Thus, the genome also needs to be modified. This is achieved by optimising vector genome components in accordance with the present invention. Advantageously, these modifications also lead to the production of a safer system absent of all additional proteins both in the producer and in the transduced cell.

As described above, the packaging components for a retroviral vector include expression products of *gag, pol* and *env* genes. In addition, efficient packaging depends on a short sequence of 4 stem loops followed by a partial sequence from *gag* and *env* (the "packaging signal"). Thus, inclusion of a deleted *gag* sequence in the retroviral vector genome (in addition to the full *gag* sequence on the packaging construct) will optimise vector titre. To date efficient packaging has been reported to require from 255 to 360 nucleotides of *gag* in vectors that still retain *env* sequences, or about 40 nucleotides of *gag* in a particular combination of splice donor mutation, *gag* and *env* deletions. It has surprisingly been found that a deletion of all but the N-terminal 360 or so nucleotides in *gag* leads to an increase in vector titre. Thus, preferably, the retroviral vector genome includes a *gag* sequence which comprises one or more deletions, more preferably the gag sequence comprises about 360 nucleotides derivable from the N-terminus.

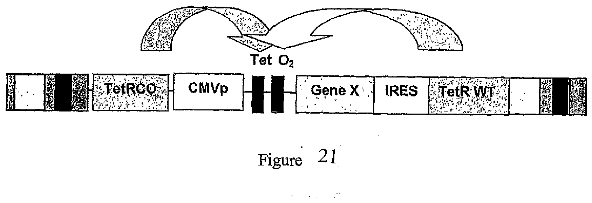

The TetR sequence may be usefully codon optimised using the codons shown in Figure 21.

NOIs

In the present invention, the term NOI (nucleotide sequence of interest) relates to nucleotide sequences useful in the treatment of a neurodegenerative disease. Also disclosed is any suitable nucleotide sequence, which need not necessarily be a complete naturally occurring DNA or RNA sequence. Thus, the NOI can be, for example, a synthetic RNA/DNA sequence, a recombinant RNA/DNA sequence (i.e. prepared by use of recombinant DNA techniques), a cDNA sequence or a partial genomic DNA sequence, including combinations thereof. The sequence need not be a coding region. If it is a coding region, it need not be an entire coding region. In addition, the RNA/DNA sequence can be in a sense orientation or in an anti-sense orientation. Preferably, it is in a sense orientation. Preferably, the sequence is, comprises, or is transcribed from cDNA.

The NOI(s), also referred to as "heterologous sequence(s)", "heterologous gene(s)" or "transgene(s)", may be any one or more of, for example, a selection gene(s), marker gene(s) and therapeutic gene(s).

The NOI may be a candidate gene which is of potential significance in a disease process. Thus the vector system of the present invention may, for example, be used for target validation purposes.

The NOI has a therapeutic application. Suitable NOIs include, but are not limited to: sequences encoding enzymes, cytokines, chemokines, hormones, antibodies, anti-oxidant molecules, engineered immunoglobulin-like molecules, a single chain antibody, fusion proteins, immune co-stimulatory molecules, immunomodulatory molecules, antisense RNA, a transdominant negative mutant of a target protein, a toxin, a conditional toxin, an antigen, a tumour suppresser protein and growth factors, membrane proteins, vasoactive proteins and peptides, anti-viral proteins and ribozymes, and derivatives thereof (such as with an associated reporter group). The NOIs may also encode pro-drug activating enzymes.

The NOI coding sequence may encode a segment of a coding sequence.

The NOI is useful in the treatment of a neurodegenerative disorder.

Preferably the NOI is useful in the treatment of Parkinson's disease.

The NOI may encode an enzyme involved in dopamine synthesis. For example, the enzyme may be one of the following: Tyrosine Hydroxylase, GTP-cyclohydrolase I and/or Aromatic Amino Acid Dopa Decarboxylase. The sequences of all three genes are available: Accession Nos. X05290, U19523 and M76180 respecively.

Alternatively the NOI may encode the vesicular monoamine transporter 2 (VMAT2). In a preferred embodiment the viral genome comprises an NOI encoding Aromatic Amino Acid Dopa Decarboxylase and an NOI encoding VMAT 2. Such a genome may be used in the treatment of Parkinson's disease, in particular in conjunction with peripheral administration of L-DOPA.

Alternatively the NOI may encode a factor capable of blocking or inhibiting degeneration in the nigrostriatal system. An example of such a factor is a neurotrophic factor. For example the NOI may encode glial cell-line derived neurotrophic factor (GDNF) or brain-derived neurotrophic factor (BDNF).

Alternatively the NOI may encode a neuroprotective factor. In particular, the NOI(s) may encode molecules which prevent TH-positive neurons from dying or which stimulate regeneration and functional recovery in the damaged nigrostriatal system.

The NOI may encode all or part of the protein of interest ("POI"), or a mutant, homologue or variant thereof. For example, the NOI may encode a fragment of the POI which is capable of functioning in vivo in an analogous manner to the wild-type protein.

In a highly preferred embodiment, one of the NOIs comprises a truncated form of the TH gene, lacking the regulatory domain. Such an NOI avoids feed-back inhibition by dopamine which may limit expression of the full-length enzyme.

The term "mutant" includes POIs which include one or more amino acid variations from the wild-type sequence. For example, a mutant may comprise one or more amino acid additions, deletions or substitutions. A mutant may arise naturally, or may be created artificially (for example by site-directed mutagenesis).

INTERNAL RIBOSOME ENTRY SITE (IRES)

The viral genome of the first aspect of the invention comprises one or more NOIs. In order for more than one of the NOIs to be expressed, there may be two or more transcription units within the vector genome, one for each NOI. However, it is clear from the literature that retroviral vectors achieve the highest titres and most potent gene expression properties if they are kept genetically simple (PCT/GB96/01230; Bowtell et al., 1988 J.Virol. 62, 2464; Correll et al., 1994 Blood 84, 1812; Emerman and Temin 1984 Cell 39, 459; Ghattas et al., 1991 Mol.Cell.Biol. 11, 5848; Hantzopoulos et al., 1989 PNAS 86, 3519; Hatzoglou et al., 1991 J.Biol.Chem 266, 8416; Hatzoglou et al., 1988 J.Biol.Chem 263, 17798; Li et al., 1992 Hum.Gen.Ther. 3, 381; McLachlin et al., 1993 Virol. 195, 1; Overell et al., 1988 Mol.Cell Biol. 8, 1803; Scharfman et al., 1991 PNAS 88, 4626; Vile et al., 1994 Gene Ther 1, 307; Xu et al., 1989 Virol. 171, 331; Yee et al., 1987 PNAS 84, 5197) and so it is preferable to use an internal ribosome entry site (IRES) to initiate translation of the second (and subsequent) coding sequence(s) in a poly-cistronic message (Adam et al 1991 J.Virol. 65, 4985).

Insertion of IRES elements into retroviral vectors is compatible with the retroviral replication cycle and allows expression of multiple coding regions from a single promoter (Adam et al (as above); Koo et al (1992) Virology 186:669-675; Chen et al 1993 J. Virol 67:2142-2148). IRES elements were first found in the non-translated 5' ends of picomaviruses where they promote cap-independent translation of viral proteins (Jang et al (1990) Enzyme 44: 292-309). When located between open reading frames in an RNA, IRES elements allow efficient translation of the downstream open reading frame by promoting entry of the ribosome at the IRES element followed by downstream initiation of translation.

A review on IRES is presented by Mountford and Smith (TIG May 1995 vol 11, No 5:179-184). A number of different IRES sequences are known including those from encephalomyocarditis virus (EMCV) (Ghattas, I.R., et al., Mol. Cell. Biol., 11:5848-5859 (1991); BiP protein [Macejak and Samow, Nature 353:91 (1991)]; the *Antennapedia* gene of drosphilia (exons d and e) [Oh, et al., Genes & Development, 6:1643-1653 (1992)] as well as those in polio virus (PV) [Pelletier and Sonenberg, Nature 334: 320-325 (1988); see also Mountford and Smith, TIG 11, 179-184 (1985)].

According to WO-A-97/14809, IRES sequences are typically found in the 5' non-coding region of genes. In addition to those in the literature they can be found empirically by looking for genetic sequences that affect expression and then determining whether that sequence affects the DNA (i.e. acts as a promoter or enhancer) or only the RNA (acts as an IRES sequence).

IRES elements from PV, EMCV and swine vesicular disease virus have previously been used in retroviral vectors (Coffin et al, as above).

The term "IRES" includes any sequence or combination of sequences which work as or improve the function of an IRES.

The IRES(s) may be of viral origin (such as EMCV IRES or PV IRES) or cellular origin.

In order for the IRES to be capable of initiating translation of each NOI, it should be located between NOIs in the vector genome. In other words there will always be one fewer IRES sequences than NOIs. For example, for a multicistronic sequence containing n NOIs, the genome may be as follows:

$$[(NOI_{1-n-1}) - (IRES_{1-n-1})] - NOI_n$$

For bi and tricistronic sequences, the order may be as follows:

$$NOI_1-IRES_1-NOI_2$$

$$NOI_1-IRES_1-NOI_2-IRES_2-NOI_3$$

The one or more NOIs are operably linked to an internal promoter. A discussion on promoters is given below. It is possible to use a combination of promoters and IRESs.

TRANSDUCED CELLS

The present invention also relates to a cell which has been transduced with a vector system comprising a viral genome according to the first aspect of the invention.

Transduction with the vector system of the present invention may confer or increase the ability of the cell to produce catecholamines. It may, for example, confer or increase the ability of the cell to convert tyrosine to L-dopa and/or L-dopa to dopamine..Release of catecholamines can be measured by techniques known in the art, for example by using an electrochemical detector connected to an analytical cell. In addition of the catecholamines themselves, biproducts associated with catecholamine release (such as DOPAC, a specific degradation product of dopamine) may also be detected.

The cell may be transduced *in vivo, in vitro* or *ex vivo.* For example, if the cell is a cell from a mammalian subject, the cell may be removed from the subject and transduced ready for reimplantation into the subject (*ex vivo* transduction). Alternatively the cell may be transduced by direct gene transfer *in vivo,* using the vector system of the present invention in accordance with standard techniques (such as via injection of vector stocks expressing the NOIs). If the cell is part of a cell line which is stable in culture (i.e. which can survive numerous passages and can multiple *in vitro*) then it may be transduced in vitro by standard techniques, for example by exposure of the cell to viral supernatants comprising vectors expressing the NOIs.

The cell may be any cell which is susceptible to transduction. If the vector system is capable of transducing non-dividing cells (for example if it is a lentiviral system) then the cell may be a non-dividing cell such as a neuron.

In a preferred embodiment the transduced cell forms part of a genetically modified neuronal cell line. Such a cell line may, for example, be transplanted into the brain for the treatment of Parkinson's disease.

In a further embodiment the cell is a neuronal stem cell. Such a cell line may, for example, be transplanted into the brain for the treatment of Parkinson's disease.

In a further embodiment the cell is a cell in the striatum of a subject, such as a neuron or glial cell. Direct gene transfer in vivo to such a cell may, for example, convert it into a dopamine-producer cell.

CASSETTES.

We also describe multicistronic cassettes comprising and ORF and two or more NOIs operably linked by a regulatory element such as an IRES or internal promoter. These cassettes may be used in a method for producing the vector genome in a producer cell.

We also describe an expression vector comprising such a cassette. Transfection of a suitable cell with such an expression vector should result is a cell which expresses each POI encoded by the NOI in the cassette.

Cloning of the cassette into an expression vector and transfection of cells with the vector (to give expression of the cassette) can be carried out by techniques well known in the art (such as those described in Sambrook et al. (Molecular Cloning: A Laboratory Manual, 2nd Edition, Cold Spring Harbor Laboratory press (1989)), and other laboratory textbooks).

The cassette may comprise a promoter. The cassette may be bicistronic or tricistronic and comprise the following elements:

$$LTR - ORF - Promoter \quad -(NOI_1)-(IRES_1)-(NOI_2)$$
$$LTR - ORF - Promoter \quad -(NOI_1)-(IRES_1)-(NOI_2)-(IRES_2)-(NOI_3)$$

The cassette may be bicistronic and comprise an NOI encoding tyrosine hydroxylase (or a mutant, variant or homologue thereof) and an NOI encoding GTP-cyclohydrolase I (or a mutant, variant or homologue thereof).

The cassette may be tricistronic and comprise an NOI encoding tyrosine hydroxylase (or a mutant, variant or homologue thereof), an NOI encoding GTP-cyclohydrolase I (or a mutant, variant or homologue thereof) and an NOI encoding Aromatic Amino Acid Dopa Decarboxylase (or a mutant, variant or homologue thereof).

VECTOR

As it is well known in the art, a vector is a tool that allows or facilates the transfer of an entity from one environment to another. In accordance with the present invention, and by way of example, some vectors used in recombinant DNA techniques allow entities, such as a segment of DNA (such as a heterologous DNA segment, such as a heterologous cDNA segment), to be transferred into a host cell for the purpose of replicating the vectors comprising a segment of DNA. Examples of vectors used in recombinant DNA techniques include but are not limited to plasmids, chromosomes, artificial chromosomes or viruses.

The term "vector" includes expression vectors and/or transformation vectors.

The term "expression vector" means a construct capable of *in vivo* or *in vitro*/*ex vivo* expression.

The term "transformation vector" means a construct capable of being transferred from one species to another.

LENTIVIRAL VECTOR

The viral vector capable of transducing a target non-dividing or slowly dividing cell is a lentiviral vector.

Lentivirus vectors are part of a larger group of retroviral vectors.

The vector of the present invention may be delivered to a target site by a viral or a non-viral vector.

Non-viral delivery systems include but are not limited to DNA transfection methods. Here, transfection includes a process using a non-viral vector to deliver a gene to a target mammalian cell.

Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (Nature Biotechnology 1996 14; 556), and combinations thereof.

Viral delivery systems include but are not limited to adenovirus vector, an adeno-associated viral (AAV) vector, a herpes viral vector, retroviral vector, lentiviral vector, baculoviral vector. Other examples of vectors include *ex vivo* delivery systems, which include but are not limited to DNA transfection methods such as electroporation, DNA biolistics, lipid-mediated transfection, compacted DNA-mediated transfection.

The delivery of one or more therapeutic genes by a vector system according to the present invention may be used alone or in combination with other treatments or components of the treatment.

OPERABLY LINKED

The term "operably linked" means that the components described are in a relationship permitting them to function in their intended manner.

PROMOTERS

The term promoter is well-known in the art and is used in the normal sense of the art, e.g. as an RNA polymerase binding site. The term encompasses nucleic acid regions ranging in size and complexity from minimal promoters to promoters including upstream elements and enhancers.

The promoter is typically selected from promoters which are functional in mammalian, cells, although promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase).

Preferably the promoter is a modified H5 or sE/L promoter (see Carroll, MW, GW Wilkinson & K Lunstrom 2001 Mammalian expression systems and vaccination Genetically Engineered Viruses Ed CJ Ring & ED Blair pp107-157 BIOS Scientific Oxford UK).

Preferably the promoter is an early late promoter used for maximum protein production and which allows optimal sensitivity during antibody identification process.

One preferred promoter-enhancer combination is a human cytomegalovirus (hCMV) major immediate early (MIE) promoter/enhancer combination.

Preferably the promoter is designed using data in Davison & Moss (J. Mol. Biol. 1989 210:749-769).

The level of expression of a nucleotide sequence(s) under the control of a particular promoter may be modulated by manipulating the promoter region. For example, different domains within a promoter region may possess different gene regulatory activities. The roles of these different regions are typically assessed using vector constructs having different variants of the promoter with specific regions deleted (that is, deletion analysis).

Preferably the promoter is a hypoxia regulated promoter.

Preferably the hypoxia regulated promoter is a cell-specific hypoxia regulated promoter.

Preferably the hypoxia regulated promoter is derived from a combination of known DNA-binding moieties optimal for a particular cell type.

The enhancer and/or promoter may be preferentially active in a hypoxic or ischaemic or low glucose environment, when the host cell is cultivated under certain conditions such as ischaemic conditions. The enhancer element or other elements conferring regulated expression may be present in multiple copies. The enhancer and/or promoter may be preferentially active in a hypoxic or ischaemic or low glucose environment, when the host cell is cultivated under certain conditions such as ischaemic conditions. The enhancer element or other elements conferring regulated expression may be present in multiple copies.

Likewise, or in addition, the enhancer and/or promoter may be preferentially active in one or more specific host cell types - such as any one or more of macrophages, endothelial cells or combinations thereof.

Preferably the tissue specific promoters are cardiomyocyte promoters.

Preferably the tissue specific promoters are macrophage promoters.

Examples of suitable tissue restricted promoters/enhancers include but are not limited to those which are highly active in tumour cells such as a promoter/enhancer from a *MUC*1 gene, a *CEA* gene or a *5T4* antigen gene. Examples of temporally restricted promoters/enhancers are those which are responsive to ischaemia and/or hypoxia, such as hypoxia response elements or the promoter/enhancer of a *grp*78 or a *grp*94 gene. The alpha fetoprotein (AFP) promoter is also a tumour-specific promoter.

Preferably the promoters are tissue specific.

The term "tissue specific" means a promoter which is not restricted in activity to a single tissue type but which nevertheless shows selectivity in that they may be active in one group of tissues and less active or silent in another group. A desirable characteristic of the promoters is that they posess a relatively low activity in the absence of activated hypoxia-regulated enhancer elements. One means of achieving this is to use "silencer" elements which suppress the activity of a selected promoter in the absence of hypoxia.

The level of expression of one or more nucleotide sequence(s) of interest under the control of a particular promoter may be modulated by manipulating the promoter region. For example, different domains within a promoter region may possess different gene regulatory activities. The roles of these different regions are typically assessed using vector constructs having different variants of the promoter with specific regions deleted (that is, deletion analysis). This approach may be used to identify, for example, the smallest region capable of conferring tissue specificity or the smallest region conferring hypoxia sensitivity.

A number of tissue specific promoters, described above, may be particularly advantageous in practising the present invention. In most instances, these promoters may be isolated as convenient restriction digestion fragments suitable for cloning in a selected vector. Alternatively, promoter fragments may be isolated using the polymerase chain reaction. Cloning of the amplified fragments may be facilitated by incorporating restriction sites at the 5' end of the primers.

Preferably the ischaemic responsive promoter is a tissue restricted ischaemic responsive promoter.

Preferably the tissue restricted ischaemic responsive promoter is a macrophage specific promoter restricted by repression.

Preferably the tissue restricted ischaemic responsive promoter is an endothelium specific promoter.

Preferably the tissue restricted ischaemic responsive promoter is an ILRE responsive promoter.

For example, the glucose-regulated proteins (grp's) such as grp78 and grp94 are highly conserved proteins known to be induced by glucose deprivation (Attenello and Lee 1984 Science 226 187-190). The grp 78 gene is expressed at low levels in most normal healthy tissues under the influence of basal level promoter elements but has at least two critical "stress inducible regulatory elements" upstream of the TATA element (Attenello 1984 ibid; Gazit et al 1995 Cancer Res 55: 1660-1663). Attachment to a truncated 632 base pair sequence of the 5'end of the grp78 promoter confers high inducibility to glucose deprivation on reporter genes *in vitro* (Gazit *et al* 1995 *ibid*). Furthermore, this promoter sequence in retroviral vectors was capable of driving a high level expression of a reporter gene in tumour cells in murine fibrosarcomas, particularly in central relatively ischaemic/fibrotic sites (Gazit *et al* 1995 *ibid*).

Preferably the selected physiologically regulated promoter elements are heat shock promoter elements.

Preferably the selected physiologically regulated promoter elements are radiation elements.

Preferably the selected physiologically regulated promoter elements are ischaemia elements.

ENHANCER

In addition, any of these promoters may be modified by the addition of further regulatory sequences, for example enhancer sequences. Chimeric promoters may also be used comprising sequence elements from two or more different promoters described above.

The term "enhancer" includes a DNA sequence which binds to other protein components of the transcription initiation complex and thus facilitates the initiation of transcription directed by its associated promoter.

Other examples of suitable tissue restricted promoters/enhancers are those which are highly active in tumour cells such as a promoter/enhancer from a *MUC*1 gene, a *CEA* gene or a *5T4* antigen gene. The alpha fetoprotein (AFP) promoter is also a tumour-specific promoter. One preferred promoter-enhancer combination is a human cytomegalovirus (hCMV) major immediate early (MIE) promoter/enhancer combination.

A preferred enhancer is an is an hypoxia response element (HRE).

HRE

As mentioned above, hypoxia is a powerful regulator of gene expression in a wide range of different cell types and acts by the induction of the activity of hypoxia-inducible transcription factors such as hypoxia inducible factor-1 (HIF-1; Wang & Semenza 1993 Proc Natl Acad Sci 90:430), which bind to cognate DNA recognition sites, the hypoxia-responsive elements (HREs) on various gene promoters. Dachs et al (1997 Nature Med 5: 515) have used a multimeric form of the HRE from the mouse phosphoglycerate kinase-1 (PGK-1) gene (Firth et al 1994 Proc Natl Acad Sci 91:6496-6500) to control expression of both marker and therapeutic genes by human fibrosarcoma cells in response to hypoxia *in vitro* and within solid tumours *in vivo* (Dachs *et al ibid*).

Hypoxia response enhancer elements (HREEs) have also been found in association with a number of genes including the erythropoietin (EPO) gene (Madan et al 1993 Proc Natl Acad Sci 90: 3928; Semenza and Wang 1992 Mol Cell Biol 1992 12: 5447-5454). Other HREEs have been isolated from regulatory regions of both the muscle glycolytic enzyme pyrivate kinase (PKM) gene (Takenaka et al 1989 J Biol Chem 264: 2363-2367), the human muscle-specific β-enolase gene (ENO3; Peshavaria and Day 1991 Biochem J 275: 427-433 ) and the endothelin-1 (ET-1) gene (Inoue et al 1989 J Biol Chem 264: 14954-14959).

Preferably the HRE selected from, for example, the erythropoietin HRE element (HREE1), muscle pyruvate kinase (PKM), HRE element, phosphoglycerate kinase (PGK) HRE, β-enolase (enolase 3; ENO3) HRE element, endothelin-1 (ET-1) HRE element and metallothionein II (MTII) HRE element.

PHARMACEUTICAL COMPOSITIONS

In one aspect, the present invention provides a pharmaceutical composition as defined in the claims, which comprises the vector

and a pharmaceutically acceptable carrier, diluent or excipient (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the pharmaceutical composition is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose or chalk, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

ADMINISTRATION

Typically, a physician will determine the actual dosage which will be most suitable for an individual subject and it will vary with the age, weight and response of the particular patient and severity of the condition. The dosages below are exemplary of the average case. There can, of course, be individual instances where higher or lower dosage ranges are merited.

The compositions (or component parts thereof) of the present invention may be administered orally. In addition or in the alternative the compositions (or component parts thereof) of the present invention may be administered by direct injection. In addition or in the alternative the compositions (or component parts thereof) of the present invention may be administered topically. In addition or in the alternative the compositions (or component parts thereof) of the present invention may be administered by inhalation. In addition or in the alternative the compositions (or component parts thereof) of the present invention may also be administered by one or more of: parenteral, mucosal, intramuscular, intravenous, subcutaneous, intraocular or transdermal administration means, and are formulated for such administration.

By way of further example, the pharmaceutical composition of the present invention may be administered in accordance with a regimen of 1 to 10 times per day, such as once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The term "administered" also includes but is not limited to delivery by a mucosal route, for example, as a nasal spray or aerosol for inhalation or as an ingestable solution; a parenteral route where delivery is by an injectable form, such as, for example, an intravenous, intramuscular or subcutaneous route.

Hence, the pharmaceutical composition of the present invention may be administered by one or more of the following routes: oral administration, injection (such as direct injection), topical, inhalation, parenteral administration, mucosal administration, intramuscular administration, intravenous administration, subcutaneous administration, intraocular administration or transdermal administration.

DISEASES

Pharmaceutical compositions comprising an effective amount of the vector may be used in the treatment of neurodegenerative
disorders such as those listed in WO-A-98/09985. For ease of reference, part of that list is now provided: :

disease, complication and/or side effects from treatment of Parlcinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems,

FIGURES

The invention is described only by way of example in which reference is made to the following Figures:

Figure 1 is a schematic representation of EIAV genomes;

Figure 2 gives the total plasmid sequence of pONY8.1G;

Figure 3 gives the total plasmid sequence of pONY8.4ZCG;

Figure 4 gives the total plasmid sequence of pONY8.4GCZ;

Figure 5. is a schematic representation of the hybrid U3 region.

Figure 6 gives the sequence of the hybrid LTR;

Figure 7 gives the sequence of pONY8. 1Zhyb;

Figure 8 is a schematic representation of pONY8.1Zhyb;

Figure 9 is a schematic representation of pONY8.1ZCG and pONY8.0G;

Figure 10 is graphical representation showing the relative tranduction efficiency of pONY8.1ZCG and pONY8.0G in the presence and absence of Rev;

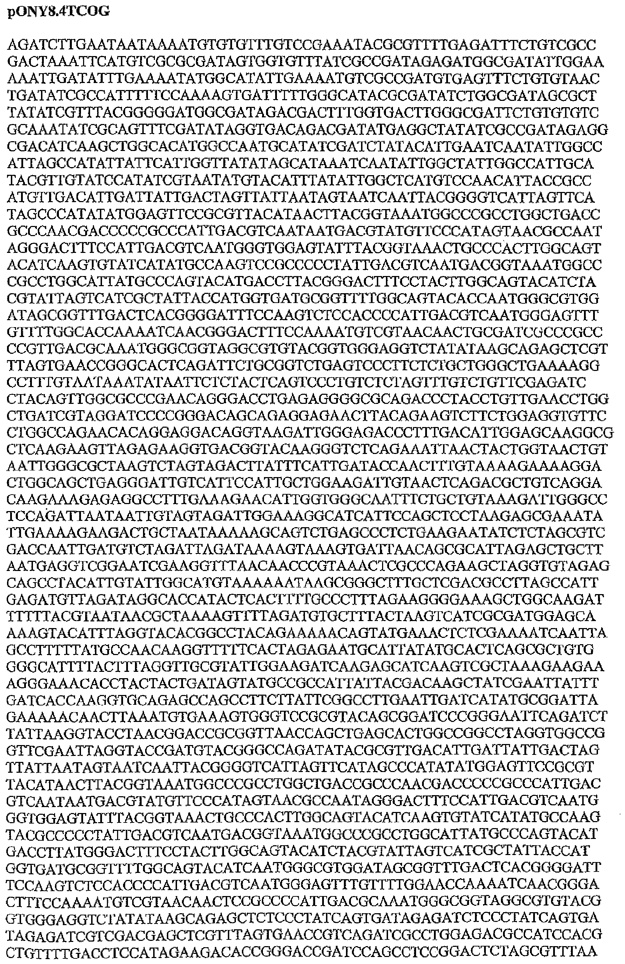

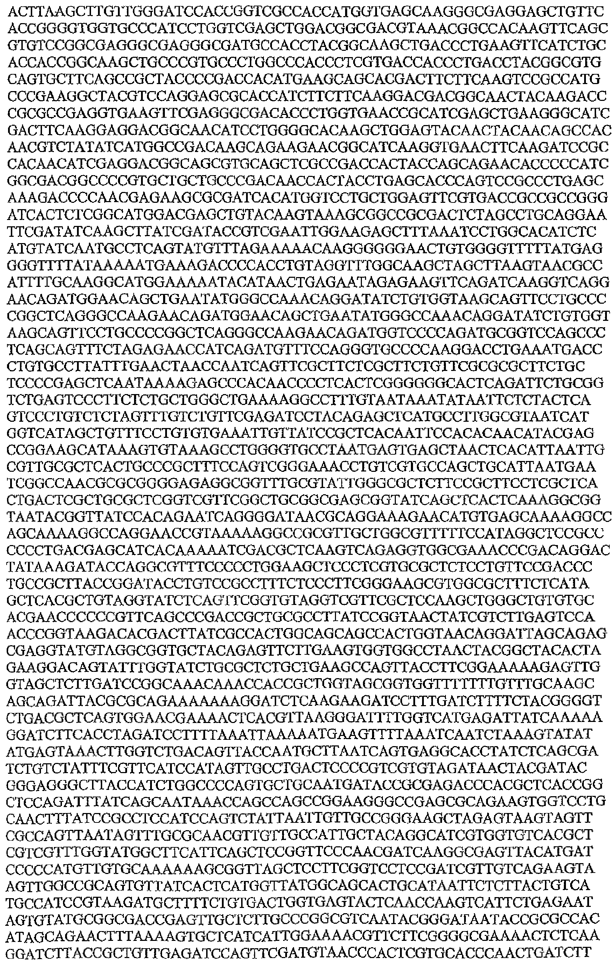

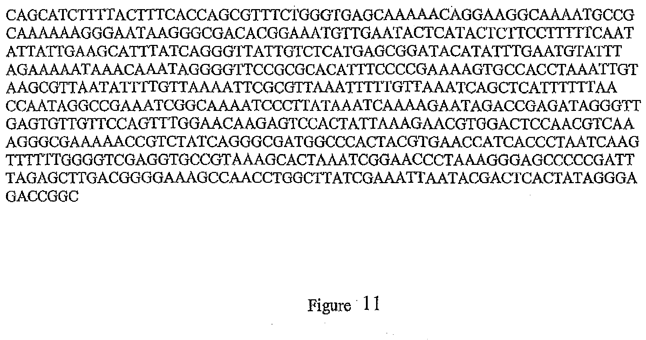

Figure 11 shows the full sequence of pONY8.4TCOG

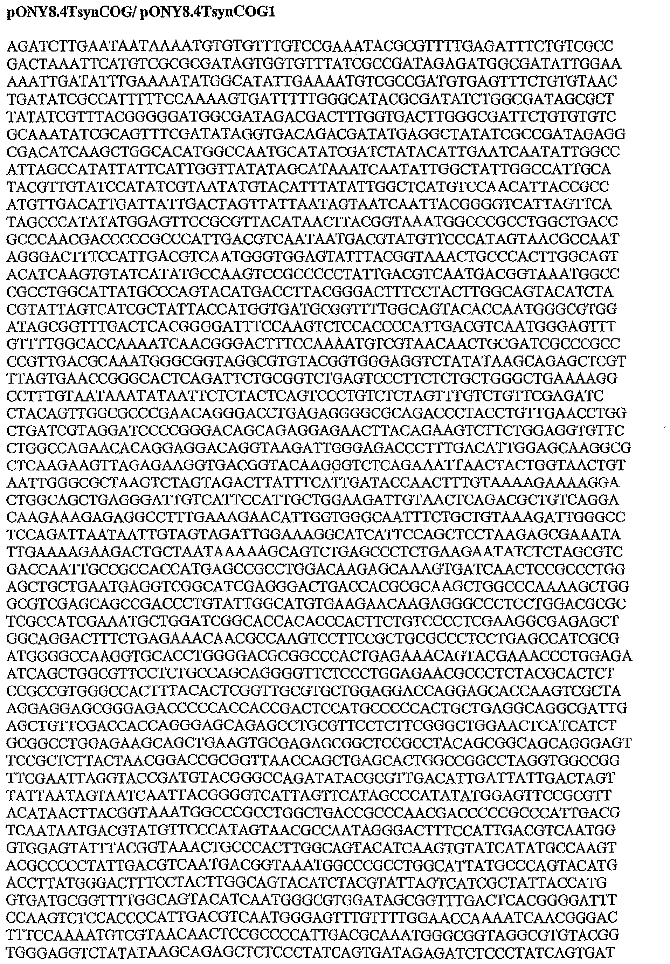

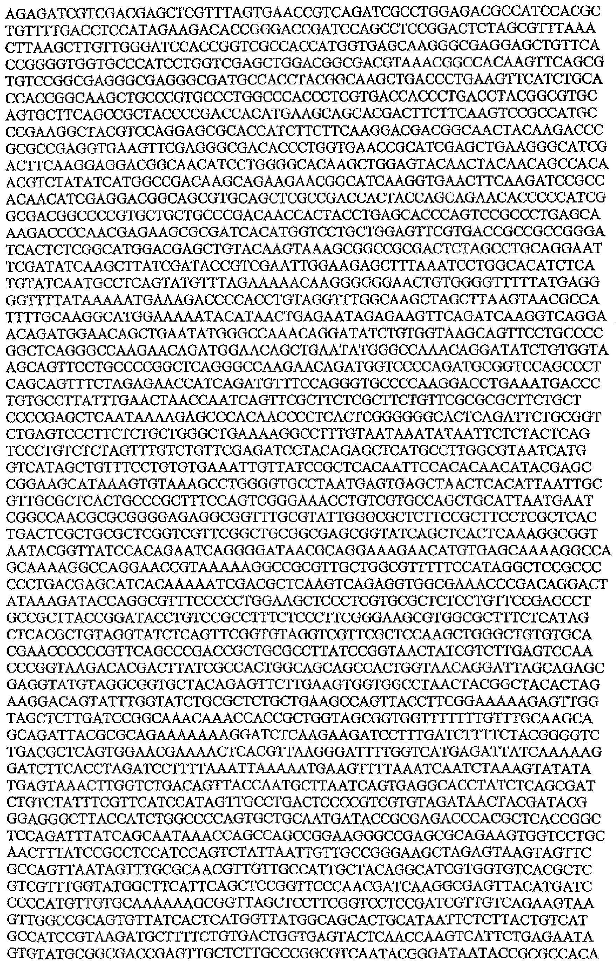

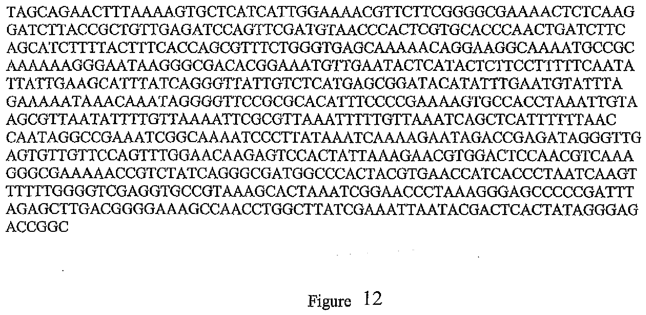

Figure 12 shows the full sequence of pONY8.4TsynCOG/pONY8.4TsynCOG1

Figure 13 is a schematic representation of pONY8.4TsynCOG/pONY8.4TsynCOG1

Figure 14 is schematic representaion of an example of a bicistronic tetracycline-regulated vector;

Figure 15 shows the analysis of codon usage of the TetR gene. MH = Mammalian highly expressed genes, WT = wild type TetR gene, CO = codon optimised TetR gene,The codon use has been altered such that it more closely resembles that of highly expressed mammalian genes;

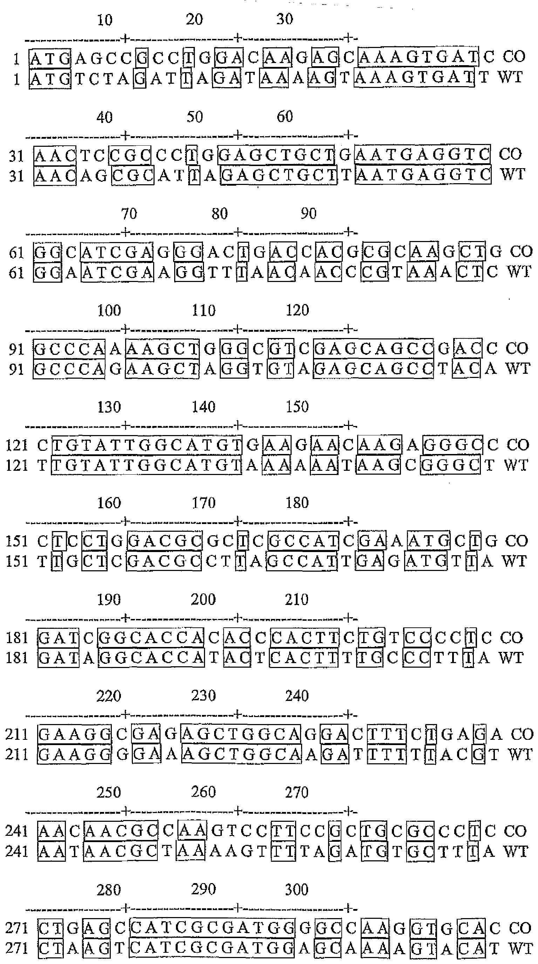

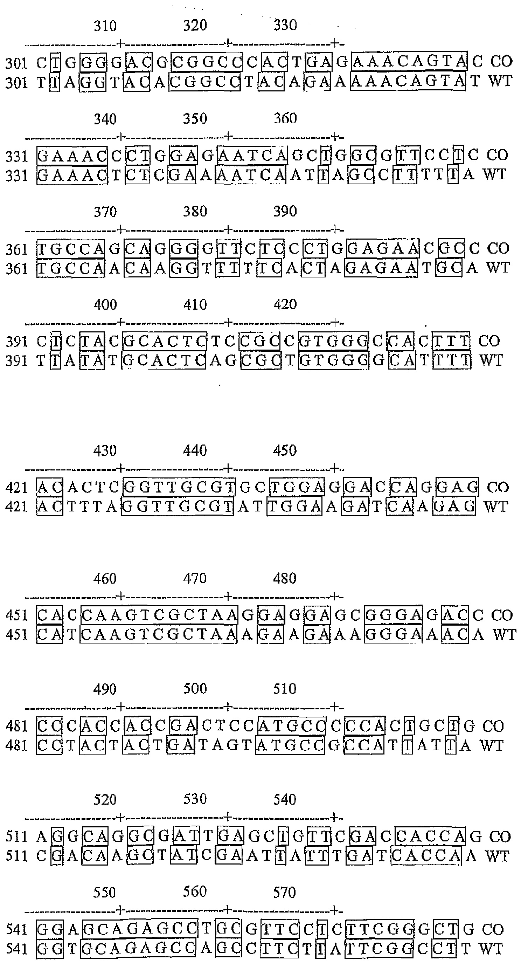

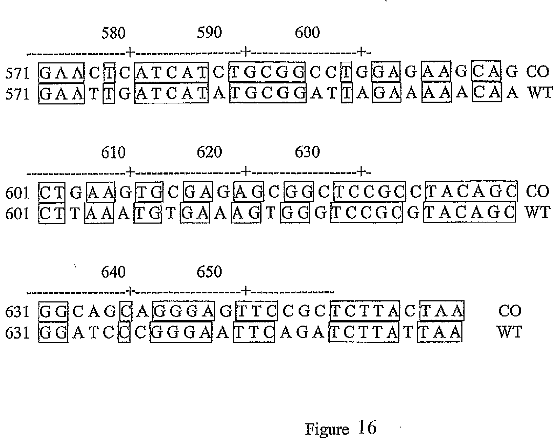

Figure 16 shows alignment of wild type and codon-optimised Tet Repressor gene sequences.
The sequences are 71% identical at the nucleotide level (longest run of homology 13bp);

Figure 17 shows that with codon optimised TetR allows GFP expression in the in the absence of dox 10 times lower than in the presence of dox. This difference is significantly larger than when using other vectors, suggesting that pONY8.4TsynCOG allow better control of repression;

Figure 18 shows the reversibility of Tet-regulated gene expression from vectors containing codon optimised TetR;

Figure 19 shows a time course for the expression of GFP after the addition of doxycycline;

Figure 20 shows expression from alternative LTRs;

Figure 21 is a schematic of the modification of the bicistronic Tet-regulated vector incorporating a second copy of the Tet repressor gene; and

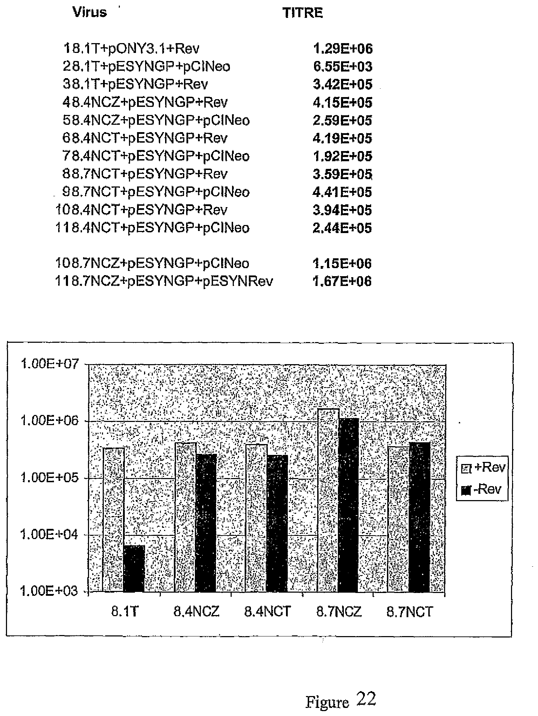

Figure 22 shows the viral titres of pONY8.4 and pONY8.7 vectors with and without Rev present. Expression of pONY8.4 and pONY8.7 is Rev independent.

EXAMPLE 1 -pONY 8.4

pONY 8.4 series of vectors has a number of modifications which enable it to function as part of a transient or stable vector system totally independent of accessory proteins, with no detrimental effect on titre. Conventionally lentiviral vector genomes have required the presence of the viral protein rev in producer cells (transient or stable) in order to obtain adequate titres. This includes current HIV vector systems as well as earlier EIAV vectors. There are 4 modifications when compared with the pONY 8.1 series of vector genomes, these are:

a) All the ATG motifs which are derived from gag and form part of the packaging signal have been modified to read ATTG. This allows the insertion of an open reading frame which can be driven by a promoter in the LTR.
b) The length of the genome i.e. distance between the R regions is closer to that seen in the wt virus (7.9kb).
c) The 3' U3 region has been modified to include sequences from the moloney luekemia virus (MLV) U3 region, so upon transduction it can drive second open reading frame (ORF) in addition to the internal cassette, In this example we have MLV but this could be any promoter.
d) The vector contains a nucleotide sequence operably linked to the viral LTR and wherein said nucleotide sequence is upstream of an internal promoter and wherein said nucleotide sequence encodes a polypeptide or fragment thereof.

Together these modifications allow production of viral delivery system without the need for accessory proteins and only 10% of the original viral sequence is integrated into the target cell. These factors are important for future safety considerations in terms of an immune response and probability of the generation of replication competent viruses. Further details on modifying LTRs can be found in our WO96/37623 and WO98/17816.

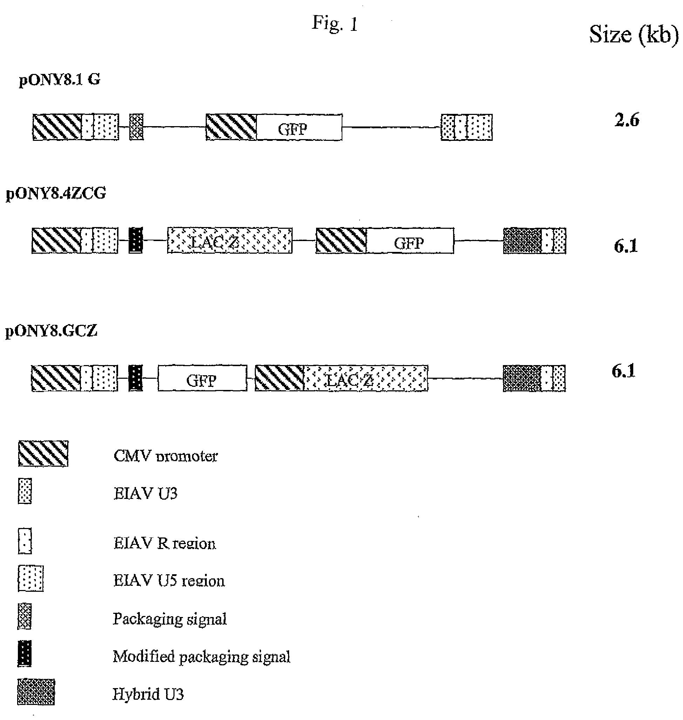

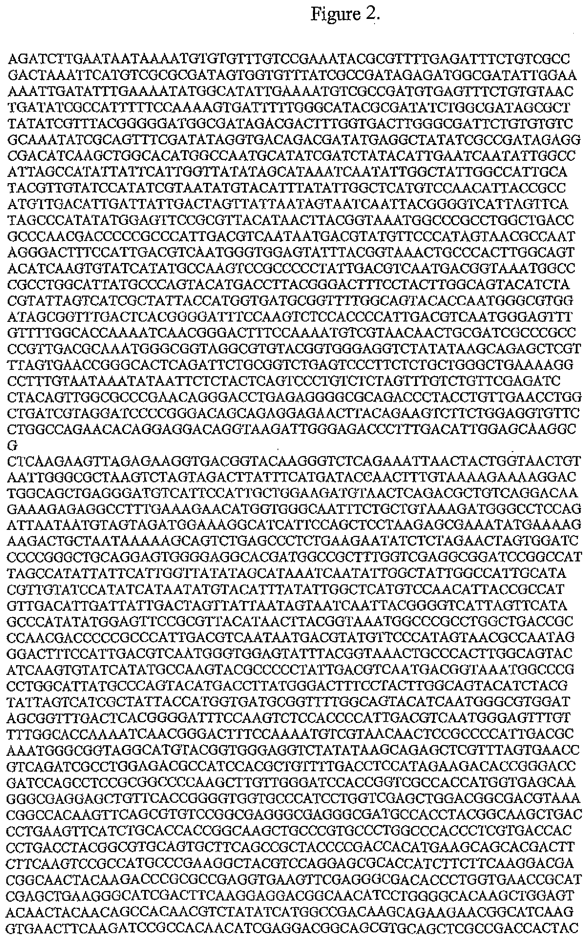

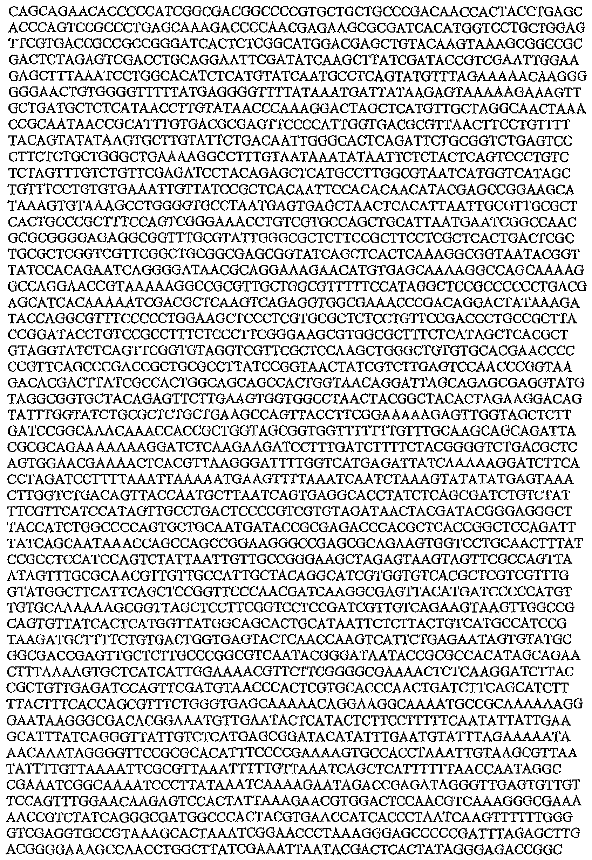

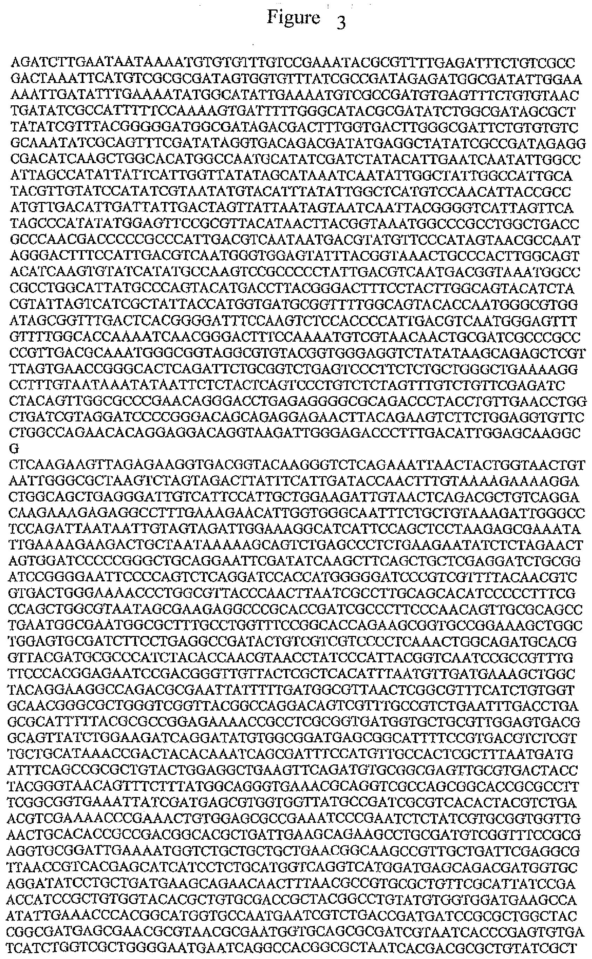

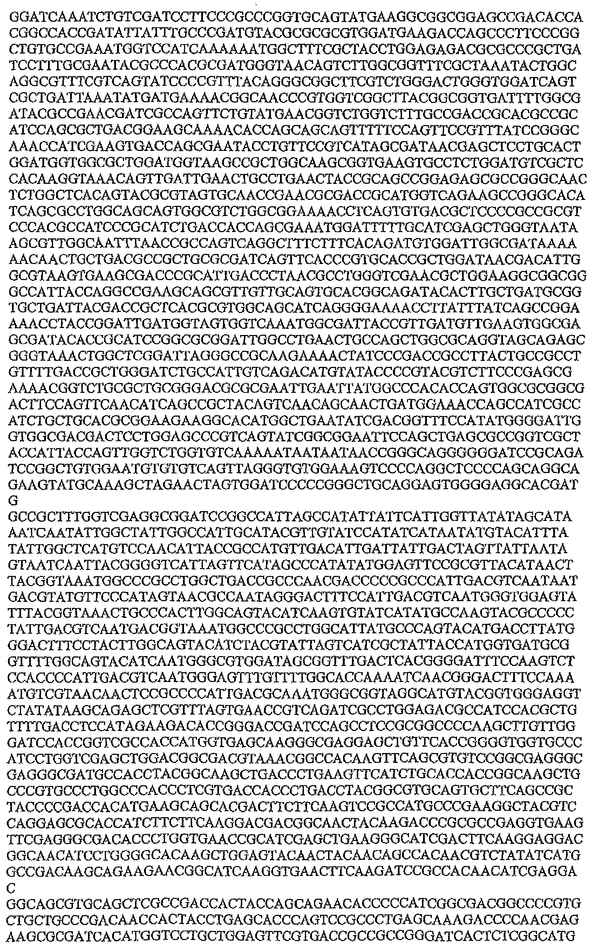

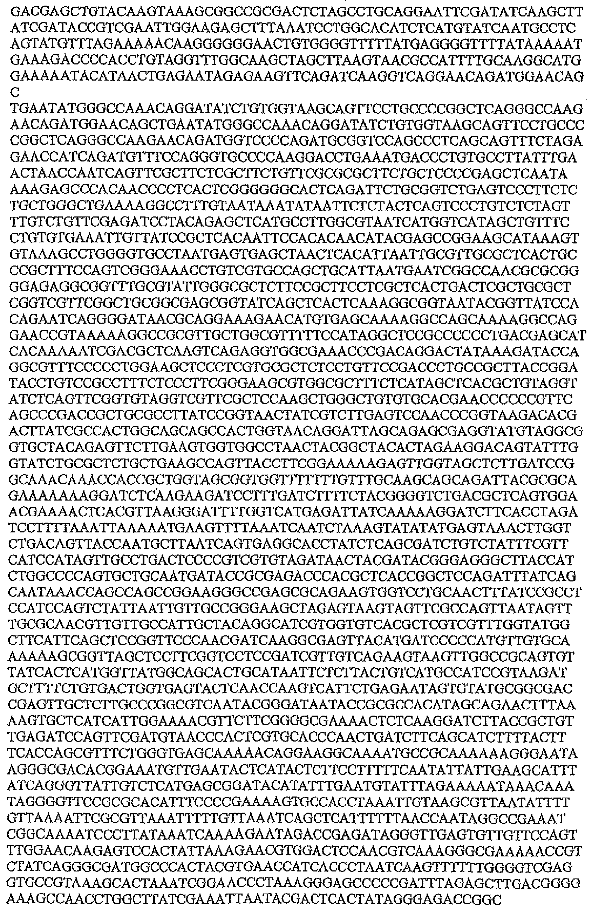

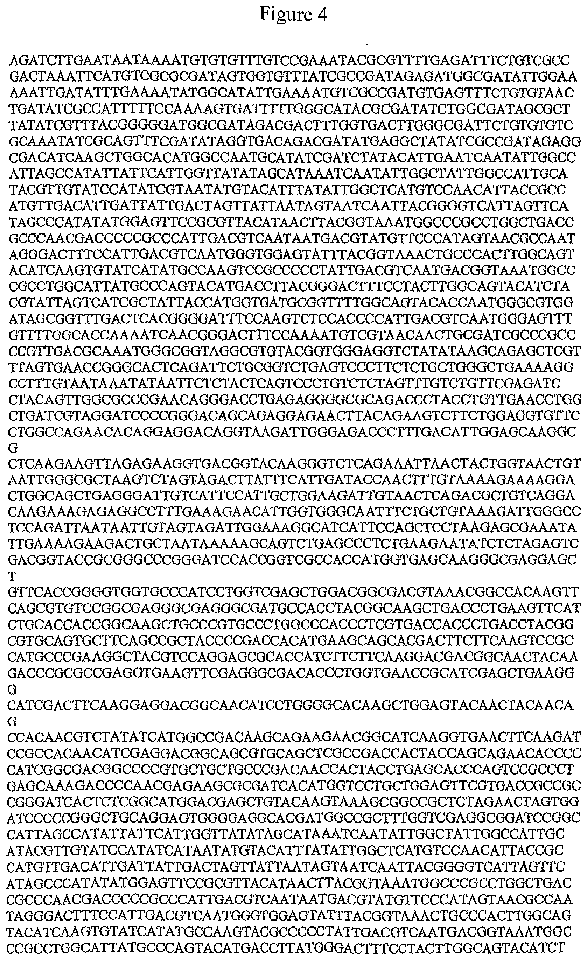

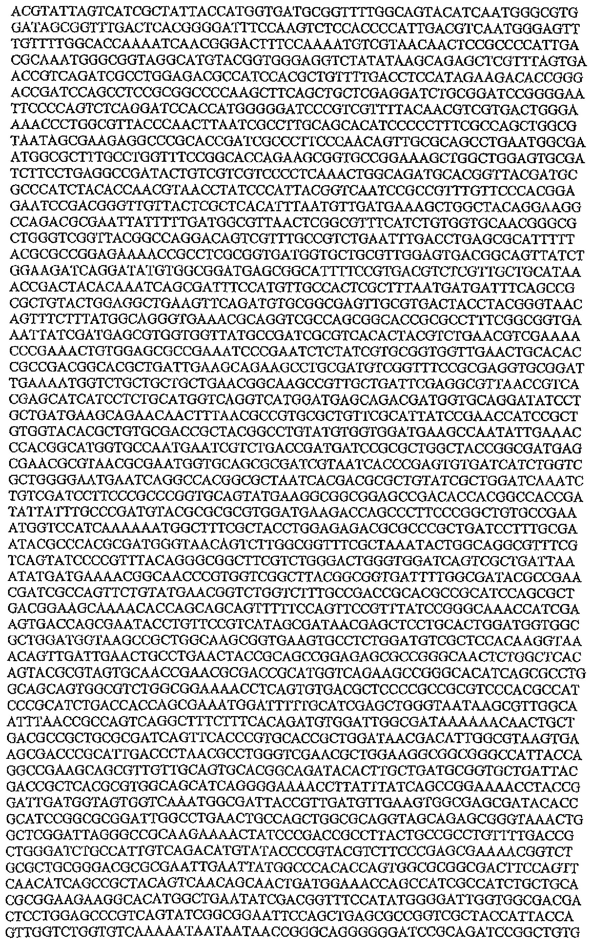

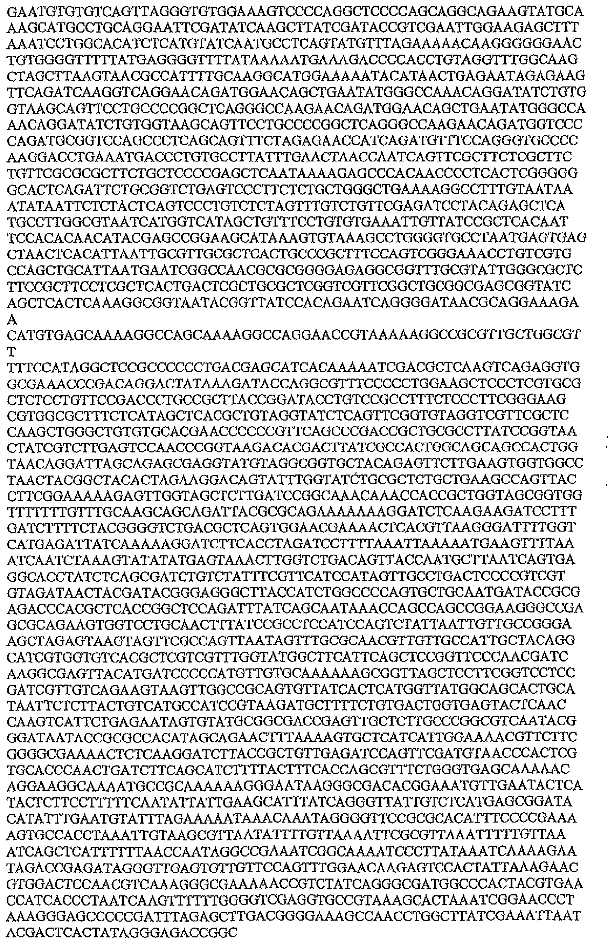

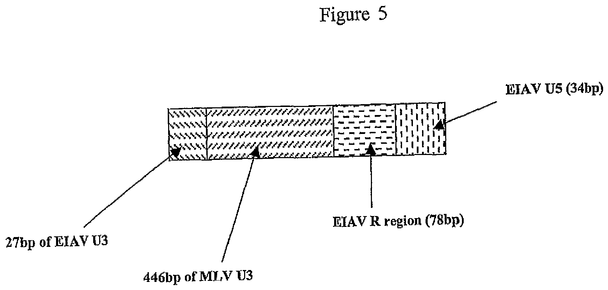

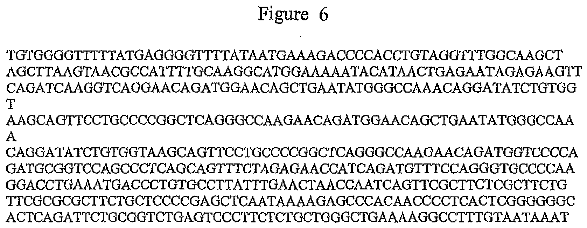

Figure 1 is a schematic representation of EIAV genomes. These may be used for transfection in accordance with the method of the present inveniton. Upon transfcetion the 3' LTR will be copied to the 5' LTR. Figure 2 gives the total plasmid sequence of pONY8.1G. Figure 3 gives the total plasmid sequence of pONY8.4ZCG, Figure 4 gives the total plasmid sequence of pONY8.4GCZ. Figure 5 is a schematic representation of the hybrid U3 region. Figure 6 gives the sequence of the hybrid LTR.

EXAMPLE 2 - The construction of EIAV/MLV Hybrid LTR Vectors

PCR was carried out as follows:

$$Product\ A = primers\ KM001 + KM003,$$

with the pONY8.1Z as target

$$Product\ B = primers\ KM004 + KM005,$$

with the pHTT111 as target.

$$Product\ C = primers\ KM006 + KM002,$$

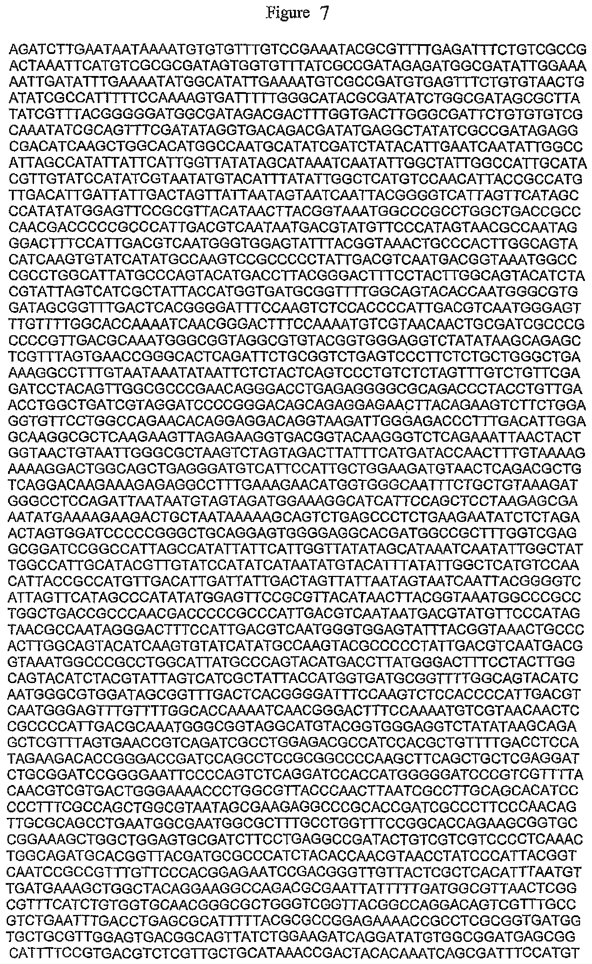

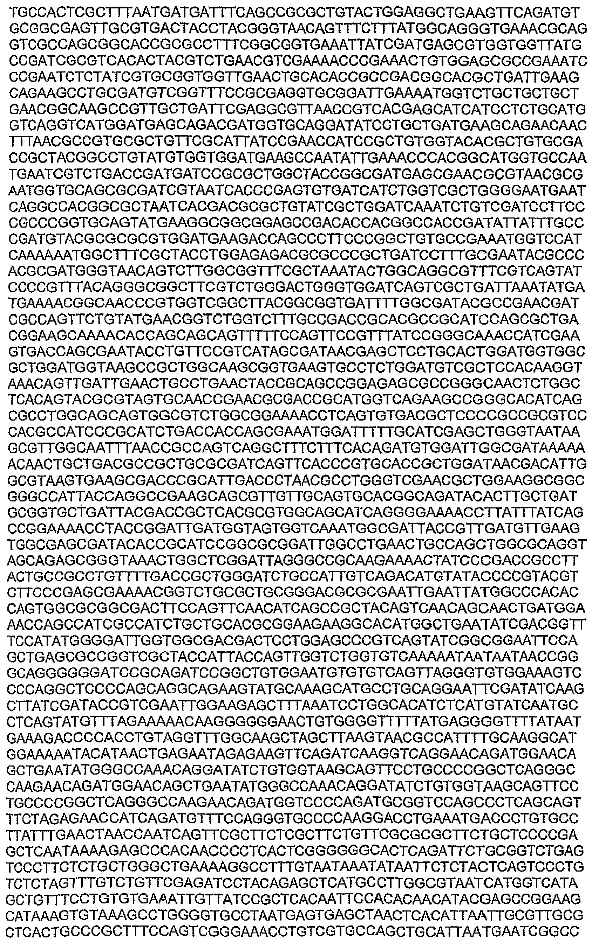

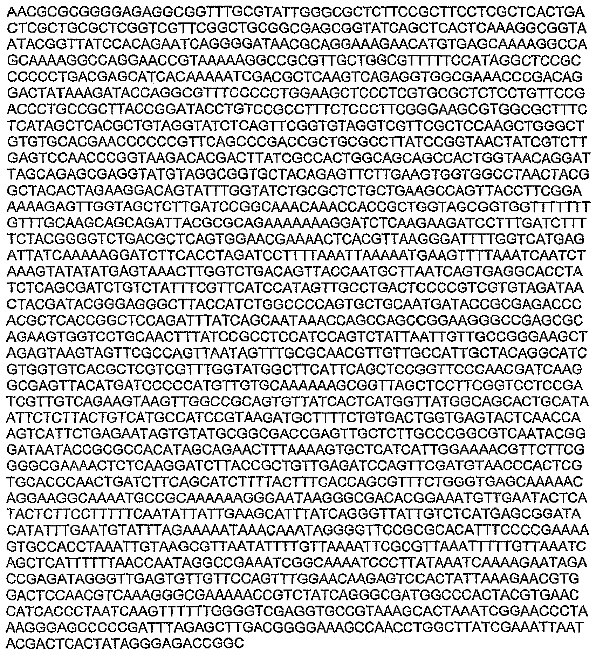

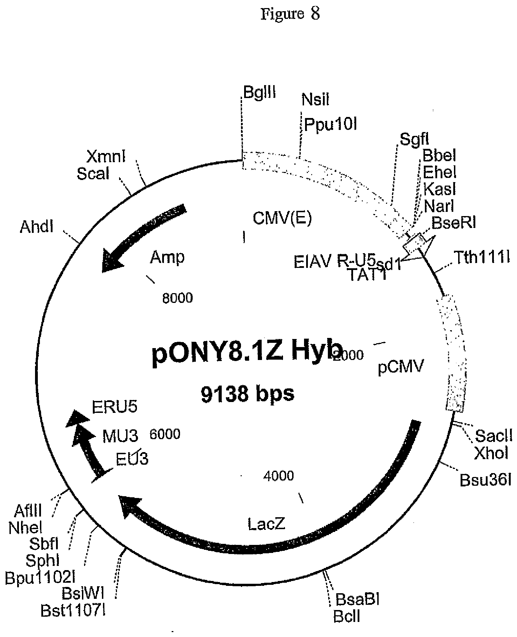

with the pONY8.1Z as target.
The PCR products (A, B and C) were gel purified. A PCR reaction was set up using Product A and B (with primers KM001 and KM005) to give Product D. A PCR reaction was set up using Product B and C (with primers KM004 and KM002) to give Product E. Product D and E were gel purified and used in a PCR reaction, as targets with primers KM001 and KM002 to give Product F. The PCR Product F was gel purified (approximately 1 kb). This was then cut with Sap I and subcloned into pONY8.1Z cut with Sap I. This gave the vector pONY8.1Zhyb shown in Figures 7 and 8. The 3' LTR of EIAV has now been replaced with an EIAV/MLV hybrid LTR. The EIAV U3 has been almost replaced with the MLV U3 region. The EIAV 5' U3 sequences of the 3'LTR have been retained as these comprise the *att* site, that is the sequences needed for integration.
The primer sequences are shown below:

EIAV/MLV hybrid U3

**KM001**
CAAAGCATGCCTGCAGGAATTCG

**KM002**
GAGCGCAGCGAGTCAGTGAGCGAG

**KM003**

GCCAAACCTACAGGTGGGGTC

TTTCATTATAAAACCCCTCATAAAAACCCCACAG

**KM004**

CTGTGGGGTTTTTATGAGGGGTTTTATAATGAAAGACCCCACCTGTAGGTTTGG

C

**KM005**

GAAGGGACTCAGACCGCAGAATCTGAGTGCCCCCCGAGTGAGGGGTTGTGGGC

TCT

**KM006**

AGAGCCCACAACCCCTCACTCGGGGG

GCACTCAGATTCTGCGGTCTGAGTCCCTTC

Sequence of final PCR product.

**EIAV PPT/U3**

CAAAGCATGCCTGCAGGAATTCGATATCAAGCTTATCGATACCGTCGAATTGGA

AGAGCTTTAAATCCTGGCACATCTCATGTATCAATGCCTCAGTATGTTTAGAAA

AACAAGGGGGGAACTGTGGGGTTTTTATGAGGGGTTTTATAA

**MLVU3**

TGAAAGACCCCACCTGTAGGTTTGGCAAGCTAGCTTAAGTAACGCCATTTTGCA

AGGCATGGAAAAATACATAACTGAGAATAGAGAAGTTCAGATCAAGGTCAGGA

ACAGATGGAACAGCTGAATATGGGCCAAACAGGATATCTGTGGTAAGCAGTTC

CTGCCCCGGCTCAGGGCCAAGAACAGATGGAACAGCTGAATATGGGCCAAACA

GGATATCTGTGGTAAGCAGTTCCTGCCCCGGCTCAGGGCCAAGAACAGATGGTC

CCCAGATGCGGTCCAGCCCTCAGCAGTTTCTAGAGAACCATCAGATGTTTCCAG

GGTGCCCCAAGGACCTGAAATGACCCTGTGCCTTATTTGAACTAACCAATCAGT

TCGCTTCTCGCTTCTGTTCGCGCGCTTCTGCTCCCCGAGCTCAATAAAAGAGCCC

ACAACCCCTCACTCGGG

**MLV U3/EIAV R/U5**

GGGCACTCAGATTCTGCGGTCTGAGTCCCTTCTCTGCTGGGCTGAAAAGGCCTT

TGTAATAAATATAATTCTCTACTCAGTCCCTGTCTCTAGTTTGTCTGTTCGAGAT

CCTACAGAGCTCATGCCTTGGCGTAATCATGGTCATAGCTGTTTCCTGTGTGAA

ATTGTTATCCGCTCACAATTCCACACAACATACGAGCCGGAAGCATAAAGTGTA

AAGCCTGGGGTGCCTAATGAGTGAGCTAACTCACATTAATTGCGTTGCGCTCAC

TGCCCGCTTTCCAGTCGGGAAACCTGTCGTGCCAGCTGCATTAATGAATCGGCC

AACGCGCGGGGAGAGGCGGTTTGCGTATTGGGCGCTCTTCCGCTTCCTCGCTCA

CTGACTCGCTGCGCTC

EXAMPLE 3 - Parkinson's Disease - Identification of regulators of tyrosine hydroxylase

Tyrosine hydroxylase (TH) is an enzyme which is required for dopamine synthesis. Nerve growth factor is known to induce TH in rat sympathetic ganglia, adrenal medulla, sympathetic neurons and chromaffin cells.

A development in molecular biology with therapeutic potential for treating Parkinson's disease is the cloning of genes encoding neurotrophins and growth factors. For example, glial-derived neurotrophic factor (GDNF) has been reported to halt the progression of degeneration in the substantia nigra induced by the neurotoxin MPTP (1-methyl-4-phenyl 1,2,3,6-tetrahydropyridine) and GDNF infusion has been reported to reduce symptoms in primate parkinsonian models. The signalling molecule Sonic hedgehog, known to have important roles in a number developmental contexts, is able to induce neurons of the developing midbrain to differentiate with a dopaminergic phenotype *in vitro.* These results suggest new therapeutic strategies could be to identify molecules that promote the differentiation or survival of dopaminergic neurons.

An alternative hypothesis is that dopaminergic neurons simply cease expression of genes involved in dopamine production. In response to GDNF the dopaminergic cells may in fact be activating expression of these genes. If this is the case then the identification of molecules that activate expression of these genes would be of considerable therapeutic function. Thus these molecules would lead to the therapeutic effect seen with GDNF without the side effects of cellular proliferation.

One approach to identify molecules which regulate the expression of tyrosine hydroxylase is:

Strategy

1. The tyrosine hydroxylase promoter is cloned such that its activity is monitored by a suitable reporter gene such as green fluorescent protein (GFP), low affinity growth factor receptor (LNGFR) or thymidine kinase (TK).

2. The promoter-reporter cassette is introduced into a dopaminergic cell line such as 1RB3AN27 or primary neurons which may or may not normally express tyrosine hydroxylase.

3. The reporter cells are transduced with a suitable library (such as cDNA or ribozyne) in an EIAV, vector.

4. Optionally the cells are grown in the presence of GDNF or other growth factor.

5. Cells in which reporter gene expression is altered are isolated, and the modulating moiety isolated from the library vector by sequencing.

6. Identify the target moiety from the sequence of the library insert.

EXAMPLE 4

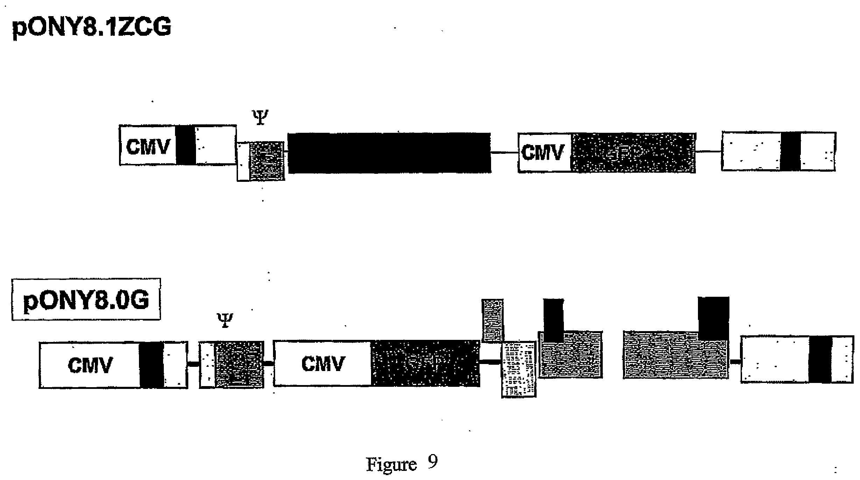

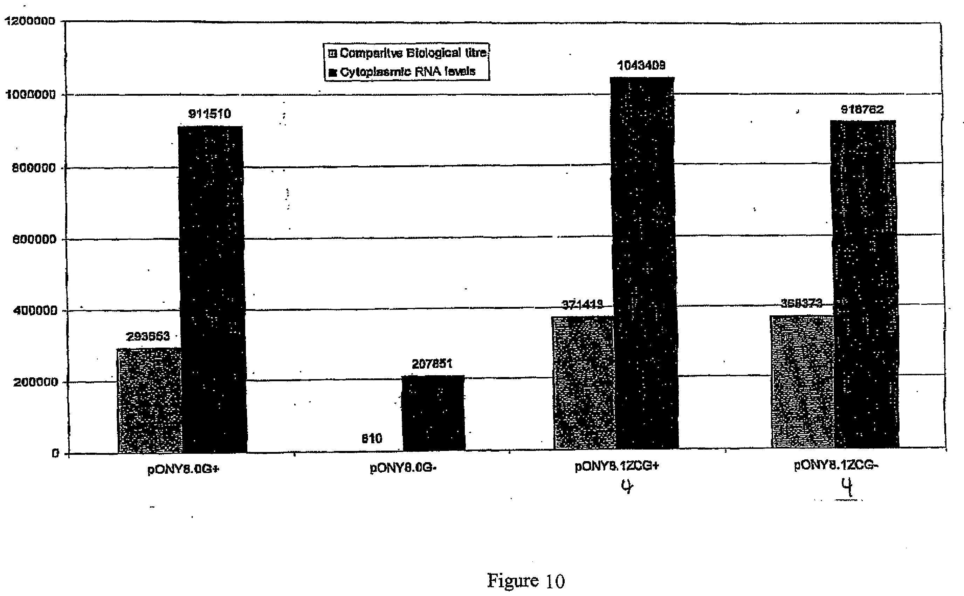

The relative REV dependancies of pONY8.1ZCG and pONY8.0G were determined by evaluating transduction efficiencies in the presence and absence of REV (see Figure 10). This was achieved using the pESYNREV vector. pESYNREV and pONY8.0Z are described in WO 0179518, pONY8.0G is derived form pONY8.0Z with GFP replacing lacZ. pONY8.1ZCG is derived from pONY8.4ZCG but does not have the hybrid LTR or complete ATTG sequence. A schematic of pONY8.1ZCG and pONY8.0G is shown in Figure 9. Transduction efficiencies of pONY8.1ZCG and pONY8.0G were evaluated using standard cell lines in the presence and absence of REV.

Cytoplasmic RNA levels in the producer cells were measured using quantative PCR and were normalised to a 100 copies of cellular β-martin. The measurements were taken at the time of viral harvesting and show that the levels of RNA with the EIAV packaging signal are reduced in the pONY8.0G producer cells in the absence of REV. This difference is not seen in the pONY8.1ZCG producer cells. The data shows the pONY8.0G (+/- pESYNREV) construct to be REV dependent, whilst pONY8.1ZCG transduction efficiency is independent of REV.

EXAMPLE 5

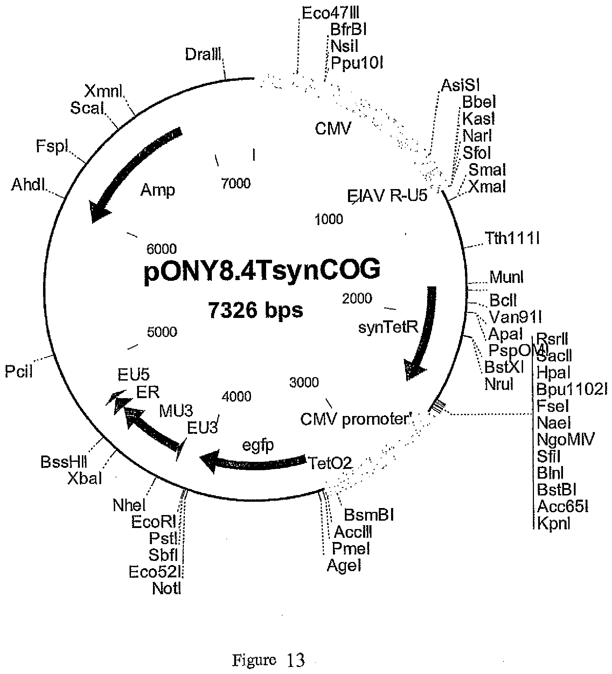

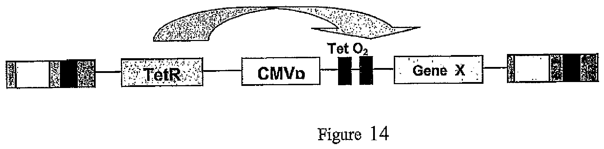

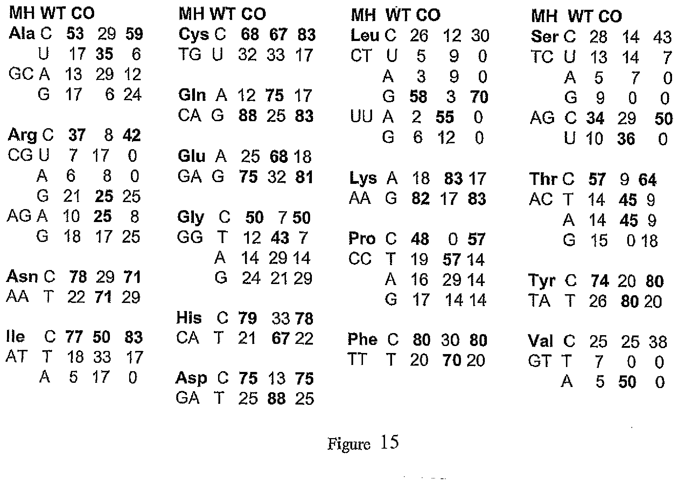

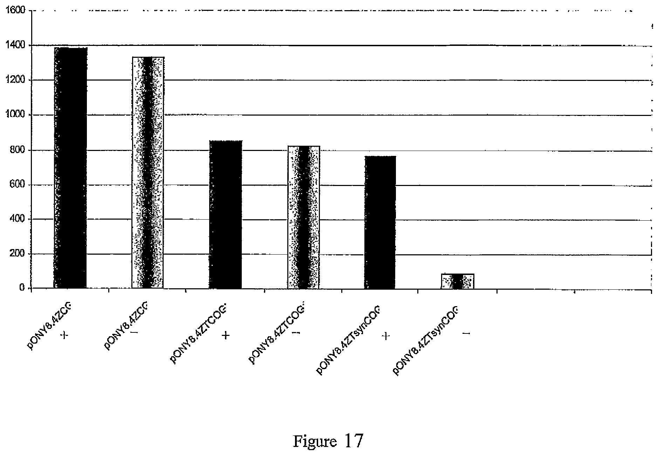

Tetracycline regulated vectors were created using pONY8.4 EIAV vector and incorporating the T-Rex tetracycline regulation system to make PONY8.4TsynCoG1 (Figures 13 and 14). The Tet $O_2$ promoter was codon optimised for expression on mammalian cells (Figure 15). GFP was used as a reporter gene to measure expression. The expression levels of pONY8.4TsynCO G1 (Figure 17), was shown to be 10 times higher in the presence of Doxycyline (dox), which binds TetR and relieves the repression of $TetO_2$ than in the absence of it. Comparison of GFP expression with the parent plasmids pONY8.4ZCG and pONY8.4TCOG1 shows that repression in the absence of dox is only achieved with pONY8.4ZTsynCOG1.

EXAMPLE 6

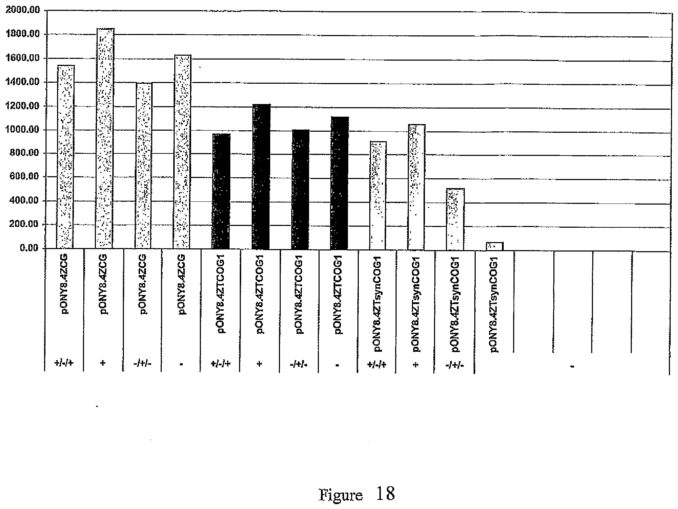

Cells transduced with pONY8.4ZCG derived vectors were cultured in media with or without dox, then switched to induce or repress expression as appropriate, then switched back again. The 'reversibility' of the Tet-regulated gene expression from the vectors containing codon optimised TetR was confirmed. The GFP expression seen in the switched cells as shown in Figure 18 has yet to reach maximal / minimal levels observed in cells which have been continually grown either + or - dox.

EXAMPLE 7

The pONY8.4TsynnlsCOG1 was made in which TetR contained a Nuclear Localisation signal (NLS) :

$$5' \text{ GAC CCC AAG AAG AAG CGC AAG GTG TAA}$$
$$D \quad P \quad K \quad K \quad K \quad R \quad K \quad V \quad stop$$

at its C terminus. The NLS is reported to improve the rate of transcriptional repression threefold (Ogueta *et al*) by increasing the concentration of TetR in the nucleus. The NLS was attached to the C terminal end so that it did not interfere with the N- terminus of TetR, which is important for binding $TetO_2$.

A time course was carried out to determine how quickly the expression occurs following the addition of dox (decrease in expression following removal of dox as measured by GFP is influenced by protein turnover). Figure 18 shows that the expression is near maximal within 24hr of the addition of the dox and that the NLS-tagged codon optimised TetR did not have a lower basal expression level than the untagged version.

EXAMPLE 8

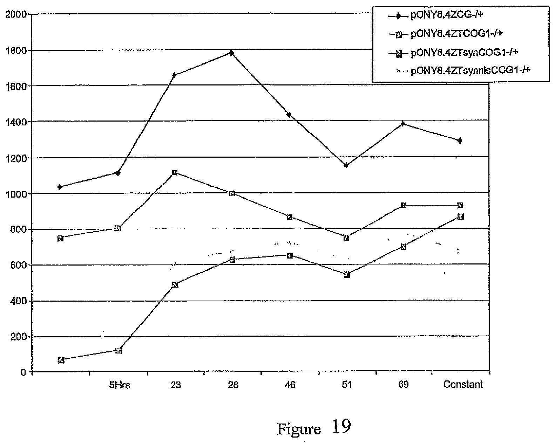

Figure 19 shows the expression of GFP from the alternative LTRs PGK and RSV in pONY8.3 vectors, proving that alternative hybrid LTRs can also be used in the pONY8.4 configured vectors.

EXAMPLE 9

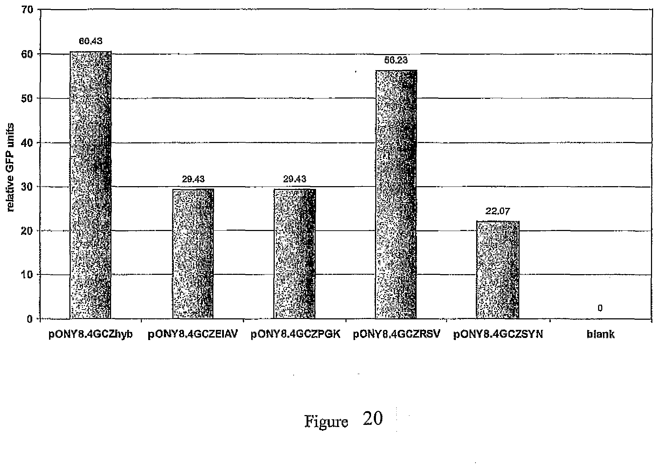

The bicistronic Tet-regulated vector may be modified to incorporate a second copy of the Tet repressor gene (Figure 20). The second copy is downstream of an IRES. This will be actively expressed when a cell is first transduced and initially contains no TetR protein: as protein levels build up, transcription of gene X and the TetR located downstream of the IRES is shut down (no tetracycline or doxycycline present). This repression will be more rapid in the modified construct shown here.

In the absence of the LTR driven TetR there would be a feedback loop resulting in cycling of transcription of gene X depending on the threshold levels of TetR The presence of a constitutively transcribed copy of TetR avoids this by maintaining a constant level of TetR in the cell. In this example the TetR gene downstream of the IRES is the wild type version minimising the possibility of undesirable recombination occurring, however, any combination of gene sequences coding for the TetR protein can be envisaged (the NOI can also be located downstream of the IRES).

EXAMPLE 10

The expression from the pONY8.4 and pONY 8.7 vectors was checked in the presence and absence of Rev by assaying viral titres from infected cells. The virus data shown is unconcentrated and from a single plate. pONY8.7NCZ was tested on a different week to the other vectors, hence slightly higher titres. The absence of Rev did not lead to a significant difference in the titre pONY8.4 and pONY8.7 based vectors, showing that they are rev independent.

Various modifications and variations of the described methods and system of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in molecular biology or related fields are intended to be within the scope of the following claims.

References

Bieri et al., (1999) Nat. Biotechnol., 17: 1105-1108

Li et al., (2000) Nucleic Acids Research, 28(13): 2605-2612

Beger et al., (2001) PNAS, 98(1): 130-135

Kestler et al., (1999) Human Gene Ther., 10(10): 1619-32

Winter et al., (1994) Annu Rev Immunol., 12: 433-55

Hoogenboom, (1997) Trends Biotechnol., 15: 62-70

Parmley and Smith, (1988) Gene, 73: 305-18

Lowman et al., (1991) Biochemistry, 30: 10832-8

Nissim et al., (1994) Embo J., 13: 692-8

Yelamos et al., (1995) Nature, 376: 225-9

Zaccolo et al., (1996) J. Mo.1 Biol., 255: 589-603

Leung et al., (1989) Technique, 1: 11-15

Kowalczykowski et al., (1994) Microbiol. Rev., 58: 401-65

Stemmer, (1994) Nature, 370: 389-91

Stemmer, (1994) Proc. Natl. Acad. Sci. USA, 91: 10747-51

Goodchild, JVK, (1991) Arch. Biochem. Biophys., 284: 386-391

"Molecular Biology and Biotechnology" (Ed. RA Meyers 1995 VCH Publishers Inc p831-8320)

"Retroviruses" (Ed. JM Coffin, et al 1997 Cold Spring Harbour Laboratory Press pp 683)

"Retroviruses" 1997 Cold Spring Harbour Laboratory Press Eds: JM Coffin, SM Hughes, HE Varmus pp 758-763

Lewis et al., (1992) EMBO. J., 11: 3053-3058

Lewis et al., (1992) EMBO. J., 11: 3053-3058

Lewis and Emerman (1994) J. Virol., 68: 510-516

Yu et al., (1986) Proc. Natl. Acad. Sci., 83: 3194-3198

Dougherty and Temin (1987) Proc. Natl. Acad. Sci., 84: 1197-1201

Hawley et al., (1987) Proc. Natl. Acad. Sci., 84: 2406-2410

Yee et al., (1987) Proc. Natl. Acad. Sci., 91: 9564-9568

Jolly et al., (1983) Nucleic Acids Res., 11: 1855-1872

Emerman and Temin (1984) Cell, 39: 449-467

Herman and Coffin (1987) Science 236: 845-848

Clabough, et al., (1991) J. Virol., 65: 6242-51

Derse and Newbold (1993) Virology, 194: 530-6

Maury, et al., (1994) Virology, 200: 632-42

Martarano et al.,(1994) J. Virol. 68: 3102-11

Senter et al., (1988) Proc. Natl. Acad. Sci., 85: 4842-4846

Mullen et al., (1994) Cancer Res., 54: 1503-1506

Kerr et al., (1990) Cancer Immunol. Immunother., 31: 202-206

Borrelli et al., (1988) Proc. Natl. Acad. Sci., 85: 7572-7576

Friedlos et al., (1997) J. Med. Chem. 40: 1270-1275

Chen et al., (1996) Cancer Res., 56: 1331-1340

Nature Biotechnology (1996) 14: 556

Landau & Littman (1992) J. Virol., 66: 5110

Soneoka et al., (1995) Nucleic Acids Res., 23: 628-633

Tomko et al., (1997) Proc. Natl. Acad. Sci., 94: 3352-2258

Wickham et al., (1993) Cell, 73: 309-319

Wickham et al., (1994) J. Cell Biol., 127: 257-264

Hong et al., (1997) EMBO. 16: 2294-2306

Meyer, et al., (1991) J. Gen. Virol.. 72: 1031-1038

Smith and Moss (1983) Gene, 25: 21-28

Upton, et al., (1986) J. Virology, 60: 920

Gershon, et al., (1989) J. Gen. Virol., 70: 525

Weir, et al., (1983) J. Virol., 46: 530

Esposito, et al., (1984) Virology, 135: 561

Hruby, et al., (1983) PNAS, 80: 3411

Kilpatrick, et al., (1985) Virology 143: 399

Binns, et al., (1988) J. Gen. Virol., 69: 1275

Boyle, et al., (1987) Virology, 156: 355

Schnitzlein, et al., (1988) J. Virological Method, 20: 341

Lytvyn, et al., (1992) J. Gen. Virol., 73: 3235-3240

Moss, B. (1991), Science, 252: 1662-7

Merchlinsky, M. et al., (1992) Virology, 190: 522-526

Scheiflinger, F. et al., (1992) Proc. Natl. Acad. Sci., USA. 89: 9977-9981

Merchlinsky, M. et al., (1992) Virology, 190: 522-526

Scheiflinger, F. et al., (1992), Proc.Natl. Acad. Sci. USA, 89: 9977-9981

Pfleiderer et al., (1995) J. General Virology, 76: 2957-2962

Marshall (1998) Nature Biotechnology, 16: 129

Wang and Sememnza (1993) Proc. Natl. Acad. Sci. USA, 90: 4304

Firth et al., (1994) Proc. Natl. Acad. Sci. USA, 91: 6496

Kallio, et al., (1998) Embo J., 17: 6573-86

Semenza, G et al., (1994) J. Biol. Chem., 269: 23757-63

Royds et al., (1998) Mol. Pathol., 51: 55-61

Matsui et al., (1999) Cardiovasc. Res., 42: 104-12

Yan, et al., (1997) J. Biol. Chem., 272: 4287-94

Estes et al., (1995) Exp. Cell Res., 220: 47-54

Gazit et al., (1995) Cancer Res., 55: 1660

Matthias, et al., (1989) NAR, 17: 6418

Carroll, MW, GW Wilkinson & K Lunstrom 2001 Mammalian expression systems and vaccination Genetically Engineered Viruses Ed: CJ Ring & ED Blair pp107-157 BIOS Scientific Oxford UK

Davison & Moss (1989) J. Mol. Biol., 210: 749-769

Attenello and Lee (1984) Science, 226: 187-190

Gazit et al., (1995) Cancer Res., 55: 1660-1663

Wang & Semenza (1993) Proc. Natl. Acad. Sci., 90: 430

Dachs et al., (1997) Nature Med., 5: 515

Firth et al., (1994) Proc. Natl. Acad. Sci., 91: 6496-6500

Madan et al., (1993) Proc. Natl. Acad. Sci., 90: 3928

Semenza and Wang (1992) Mol. Cell Biol., 12: 5447-5454

Takenaka et al., (1989) J. Biol. Chem., 264: 2363-2367

Peshavaria and Day (1991) Biochem. J. 275: 427-433

Inoue et al., (1989) J. Biol. Chem. 264: 14954-14959

O'Hare, M. J. et al., Proc. Natl. Acad. Sci. USA, 98(2): 646-51

Gillett et al., (1994) Cancer Res., 54(7): 1812-7

Yu et al., (2001) Nature, 411(6841): 1017-21

Stirling & Chung (2000) Eur. Respir. J., 16(6): 1158-74

Weltman & Karim (2000) Expert Opin. Investig. Drugs 9(3): 491-6

Cai, H. et al., (2001) Nat. Neurosci., 4(3): 233-4

Vigneri & Wang (2001) Nat. Med., 7(2): 228-34

Gorre et al., (Aug 3 2001) Science; 293(5531): 876-80

McCormick (Jul 19 2001) Nature;412(6844): 281-2

Jahagirdar et al., (May 2001) Exp. Hematol.; 29(5): 543-56.

Sillaber et al., (Mar 15 2000) Blood.; 95(6): 2118-25

Liu et al., (Mar 1996) Mol. Cell Biol.; 16(3): 998-1005

Arlinghaus (1998) Crit. Rev. Oncog., 9(1): 1-18

Shah et al., (Apr 1991) Mol Cell Biol.; 11(4): 1854-60

Zhu et al., (Dec 11 1990) Nucleic Acids Res.; 18(23): 7119-7125

Collins et al., (Aug 1987) Mol. Cell Biol.; 7(8): 2870-2876

Voncken et al., (Nov 1998) Int. J. Mol. Med.; 2(5): 577-583

Voncken et al., (Mar 10 1995) Cell; 80(5): 719-728

Voncken et al., (Apr 16 1998) Oncogene; 16(15): 2029-2032

Wetzler et al., (Oct 1993) J. Clin. Invest; 92(4): 1925-1939

Perkins et al., (Feb 1 2000) Blood; 95(3): 1014-22

Porosnicu et al., (May 2001) Leukemia,;15(5): 772-778

Kwong & Todd, (May 1 1997) Blood; 89(9): 3487-8

Puccetti et al., (Jul 1 2000) Cancer Res. 60(13): 3409-3413

Andrews & Collins, (Oct 1987) Leukemia; 1(10): 718-24

Dalgaard (1957) Acta, Med, Scand., 328: 1-255

Bacolla et al., (May 25 2001) J. Biol. Chem.; 276(21): 18597-604

Arnaout (2001) Annu. Rev. Med., 52: 93-123

Calvet & Grantham (Mar 2001) Semin Nephrol. 21(2): 107-23

Boletta et al., (Nov 2000) MoL Cell.; 6(5): 1267-73

Grantham (Jul 2001) Curr Opin Nephrol Hypertens.; 10(4): 533-42

Arnould et al., (May 1999) Mol. Cell Biol.; 19(5): 3423-34

Pugh et al., (Mar 1995) Kidney Int.; 47(3): 774-81

Richard et al., (Mar 11 998) J. Clin. Invest.; 101(5): 935-9

Ibraghimov-Beskrovnaya et al., (Jun 10 1997) Proc Natl Acad Sci USA., 94(12): 6397-402

Kim (Feb 15 2000) Proc. Natl. Acad. Sci. USA. 97(4): 1731-6

Pey et al., (Nov-Dec 1999) In Vitro Cell Dev. Biol. Anim. 35(10): 571-9

Miskin et al., (1998 & 2000) Science, 281: 562-565; J. Virol., 74: 9412-9420

Powell et al., J. Virol., 70: 8527-33

Tait et al., J. Biol. Chem., 275: 34656-64

O'Hare, M. J. et al., (2001) Proc. Natl. Acad. Sci. U S A., 98(2): 646-51

Cai, H. et al., (2001) Nat. Neurosci., 4(3): 233-4

Emmons SW, Somlo S. (23 Septmeber 1999) Nature, 401(6751): 339-40

## Claims

1. A multicistronic vector genome derivable from a lentivirus, for use in a *rev*independent lentiviral production system for producing a lentivirus-derived vector particle, said genome comprising a packaging signal, a heterologous open reading frame (ORF) downstream of a viral LTR and upstream of a promotor, wherein the promoter controls the expression of at least one downstream nucleotide of interest (NOI) useful in the treatment of a neurodegenerative disorder, and wherein the heterologous ORF is operably linked to the LTR; wherein the nucleic acid sequence encoding the auxiliary gene *rev* is disrupted such that said auxiliary gene is incapable of encoding the functional auxiliary protein, or is removed from the lentiviral vector genome, and wherein the Rev Response element (RRE) is disrupted such that said RRE is non-functional, or is removed from the lentiviral vector genome, and wherein the lentiviral vector is a self-inactivating lentiviral vector.

**2.** The vector genome of claim 1 wherein the NOI is useful in the treatment of Parkinson's disease.

**3.** The vector genome of claim 2 wherein the NOI is involved in dopamine synthesis.

**4.** The vector genome according to any one of claims 1 to 3 wherein the NOI encodes one or more of Tyrosine Hydroxylase, GTP-cyclohydrolase I, Aromatic Amino Acid Dopa Decarboxylase or vesicular monoamine transporter 2 (VMAT2).

**5.** The vector genome according to claim 1 wherein the NOI encodes a factor capable of blocking or inhibiting degeneration in the nigrostriatal system.

**6.** The vector genome according to claim 5 wherein the factor is a neurotrophic factor.

**7.** The vector genome according to claim 5 wherein the factor is a glial cell-line derived neurotrophic factor (GDNF) or a brain-derived neurotrophic factor (BDNF).

**8.** The vector genome according to claim 1 wherein the NOI encodes a neuroprotective factor.

**9.** The vector genome of any preceding claim wherein the heterologous ORF is a reporter moiety or selectable marker.

**10.** The vector genome of any preceding claim wherein the heterologous ORF encodes a modulator gene.

**11.** The vector genome of claim 10 wherein the modulator gene is selected from tetracycline repressors, such as TetR, and tetracycline-controlled transactivators, such as tTA and rtTA.

**12.** The vector genome according to claim 11 wherein the tetracycline repressor is codon optimised for expression in a mammalian cell.

**13.** The vector genome according to any preceding claim wherein the vector genome is derivable from a non-primate lentivirus vector.

**14.** The vector genome according to any preceding claim wherein the vector genome is derivable from a lentivirus selected from the group consisting of HIV-1, HIV-2, SIV, FIV, BIV, EIAV, CAEV, MVV and visna lentivirus vector.

**15.** The vector genome according to any preceding claim wherein the vector genome comprises a central polypurine tract (cPPT) sequence.

**16.** The vector genome according to any preceding claim wherein the vector genome comprises a post-transcriptional regulatory element or a translational element.

**17.** The vector genome according to any preceding claim wherein all ATG motifs which are derived from *gag* and form part of the packaging signal have been modified to read ATTG.

**18.** The vector genome according to any preceding claim wherein the vector genome is bi-, tricistronic or quadcistronic.

**19.** A lentiviral vector production system for producing a lentivirus-derived vector particle, which system comprises a set of nucleic acid sequences encoding the components of the vector genome including the vector genome of any one of claims 1 to 18, Gag and Pol proteins, and Env protein or a functional substitute therefor and wherein a nucleic acid sequence encoding the auxiliary gene *rev,* is disrupted such that said auxiliary gene is incapable of encoding the functional auxiliary protein, or is not present in the lentiviral vector production system, and is not supplied *in trans,* and RRE is disrupted such that RRE is non-functional, or is not present in the lentiviral vector production system, and is not supplied *in trans.*

**20.** The production system according to claim 19 wherein the set of nucleic acid sequences comprises three DNA constructs encoding, (i) the lentiviral vector genome, (ii) Gag and Pol, and (iii) Env.

**21.** The production system according to claim 19 or 20 wherein the nucleic acid sequence encoding Gag and Pol is codon optimised.

**22.** The production system according to any one of claims 19 to 21 wherein the nucleic acid sequences encoding at least one of the auxiliary genes *vpr, vif, tat* and *nef,* or analogous auxiliary genes, from the lentivirus from which said particles are derived, are also disrupted such that said auxiliary genes are incapable of encoding the functional auxiliary proteins, or removed from the system.

**23.** A DNA construct for use in the system according to any one of claims 19 to 22, said DNA construct encoding a packagable RNA vector genome as defined in any one of claims 1 to 18.

**24.** A set of DNA constructs comprising the DNA construct according to claim 23 and a DNA construct encoding Gag and Pol as defined in any one of claims 19 to 21.

**25.** The set of DNA constructs according to claim 24 further comprising a DNA construct encoding Env or a functional substitute thereof.

**26.** A process for preparing a lentiviral vector particle comprising introducing a set of nucleic acid sequences or DNA constructs as defined in any one of claims 23 to 25 into a host cell, and obtaining the lentiviral vector particle.

**27.** A lentiviral vector particle produced by the system, DNA construct or set of DNA constructs or process according to any one of claims 19 to 22, 25 and 26.

**28.** A lentiviral-derived vector particle comprising a vector genome as defined in any one of claims 1 to 18, Gag and Pol proteins, and Env protein, or a functional substitute therefor.

**29.** A cell transduced with the lentiviral vector particle of claim 27 or 28 or DNA construct or set of DNA constructs of any one of claims 23 to 25.

**30.** A lentiviral vector particle of claim 27 or 28 or a DNA construct or set of DNA constructs of any one of claims 23 to 25 or a cell of claim 29 for use in medicine.

**31.** A pharmaceutical composition comprising the vector genome of any one of claims 1 to 18, the system of any one of claims 19 to 22, the DNA construct or set of DNA constructs of any one of claims 23 to 25, a particle of claim 27 or 28 or a cell of claim 29, together with a pharmaceutically acceptable carrier or diluent.

**32.** Use of a lentiviral vector particle of claim 27 or 28 or a DNA construct or a set of DNA constructs of any one of claims 23 to 25 or a cell of claim 29 for the preparation of a medicament to deliver an NOI to a target site in need of same.

**33.** The use of claim 32 wherein the medicament is for the treatment of a neurodegenerative disease, such as Parkinson's disease.

**34.** A delivery system in the form of a lentiviral vector particle of claim 27 or 28 or a DNA construct or set of DNA constructs of any one of claims 23 to 25 or a cell of claim 29 for use in medicine.

**Patentansprüche**

**1.** Multicistronisches Vektorgenom, ableitbar von einem Lentivirus, zur Verwendung in einem *rev*unabhängigen lentiviralen Produktionssystem zur Herstellung eines von Lentivirus abgeleiteten Vektorpartikels, wobei das Genom ein Verpackungssignal, ein heterologes offenes Leseraster (ORF) stromabwärts eines viralen LTR und stromaufwärts eines Promotors umfasst, wobei der Promotor die Expression wenigstens eines stromabwärts liegenden Nukleotids von Interesse (NOI), das sich für die Behandlung einer neurodegenerativen Erkrankung eignet, kontrolliert, und wobei das heterologe ORF in operativer Verknüpfung mit dem LTR steht; wobei die für das Hilfsgen *rev* codierende Nukleinsäuresequenz unterbrochen ist, so dass das Hilfsgen nicht für das funktionelle Hilfsprotein codieren kann, oder aus dem lentiviralen Vektorgenom entfernt ist und wobei das Rev-Response-Element (RRE) unterbrochen ist, so dass das RRE nicht funktionsfähig ist, oder aus dem lentiviralen Vektorgenom entfernt ist und wobei es sich bei dem lentiviralen Vektor um einen selbstinaktivierenden lentiviralen Vektor handelt.

**2.** Vektorgenom nach Anspruch 1, wobei sich das NOI für die Behandlung von Morbus Parkinson eignet.

3. Vektorgenom nach Anspruch 2, wobei das NOI an der Dopamin-Synthese beteiligt ist.

4. Vektorgenom gemäß einem der Ansprüche 1 bis 3, wobei das NOI für ein oder mehrere von Tyrosin-Hydroxylase, GTP-Cyclohydrolase I, Aromatische-Aminosäure-Dopa-Decarboxylase oder vesikulärem Monoamin-Transporter 2 (VMAT2) codiert.

5. Vektorgenom gemäß Anspruch 1, wobei das NOI für einen Faktor codiert, der zur Blockierung oder Hemmung von Degeneration im nigrostriatalen System fähig ist.

6. Vektorgenom gemäß Anspruch 5, wobei es sich bei dem Faktor um einen neurotrophen Faktor handelt.

7. Vektorgenom gemäß Anspruch 5, wobei es sich bei dem Faktor um einen aus einer Gliazelllinie stammenden neurotrophen Faktor (GDNF) oder einen aus Gehirn stammenden neurotrophen Faktor (BDNF) handelt.

8. Vektorgenom gemäß Anspruch 1, wobei das NOI für einen neuroprotektiven Faktor codiert.

9. Vektorgenom nach einem der vorhergehenden Ansprüche, wobei es sich bei dem heterologen ORF um eine Repertergruppierung oder einen selektionierbaren Marker handelt.

10. Vektorgenom nach einem der vorhergehenden Ansprüche, wobei das heterologe ORF für ein Modulator-Gen codiert.

11. Vektorgenom nach Anspruch 10, wobei das Modulator-Gen aus Tetracyclin-Repressoren, wie etwa TetR, und tetracyclin-kontrollierten Transaktivatoren, wie etwa tTA und rtTA, ausgewählt ist.

12. Vektorgenom gemäß Anspruch 11, wobei der Tetracyclin-Repressor für die Expression in einer Säugerzelle codonoptimiert ist.

13. Vektorgenom gemäß einem der vorhergehenden Ansprüche, wobei das Vektorgenom von einem Nichtprimaten-Lentivirus-Vektor ableitbar ist.

14. Vektorgenom gemäß einem der vorhergehenden Ansprüche, wobei das Vektorgenom von einem aus der aus HIV-1, HIV-2, SIV, FIV, BIV, EIAV, CAEV, MVV und Visna-Lentivirus-Vektor bestehenden Gruppe ausgewählten Lentivirus ableitbar ist.

15. Vektorgenom gemäß einem der vorhergehenden Ansprüche, wobei das Vektorgenom eine cPPT(central polypurine tract)-Sequenz umfasst.

16. Vektorgenom gemäß einem der vorhergehenden Ansprüche, wobei das Vektorgenom ein posttranskriptionales Regulationselement oder ein translationales Element umfasst.

17. Vektorgenom gemäß einem der vorhergehenden Ansprüche, wobei alle ATG-Motive, die von *gag* abgeleitet sind und einen Teil des Verpackungssignals bilden, so modifiziert sind, dass sie als ATTG gelesen werden.

18. Vektorgenom gemäß einem der vorhergehenden Ansprüche, wobei das Vektorgenom bi-, tricistronisch oder quadcistronisch ist.

19. Lentivirus-Vektor-Produktionssystem zur Herstellung eines von Lentivirus abgeleiteten Vektorpartikels, wobei das System einen Satz Nukleinsäuresequenzen umfasst, die für die Komponenten des Vektorgenoms, einschließlich des Vektorgenoms nach einem der Ansprüche 1 bis 18, Gag- und Pol-Proteine sowie Env-Protein oder einen funktionellen Ersatz dafür codieren, und wobei eine für das Hilfsgen *rev* codierende Nukleinsäuresequenz unterbrochen ist, so dass das Hilfsgen nicht für das funktionelle Hilfsprotein codieren kann, oder nicht in dem Lentivirus-Vektor-Produktionssystem vorhanden ist und nicht *in trans* bereitgestellt wird, und RRE unterbrochen ist, so dass RRE nicht funktionsfähig ist, oder nicht in dem Lentivirus-Vektor-Produktionssystem vorhanden ist und nicht *in trans* bereitgestellt wird.

20. Produktionssystem gemäß Anspruch 19, wobei der Satz Nukleinsäuresequenzen drei DNA-Konstrukte umfasst, die für (i) das lentivirale Vektorgenom, (ii) Gag und Pol und (iii) Env codieren.

**21.** Produktionssystem gemäß Anspruch 19 oder 20, wobei die für Gag und Pol codierende Nukleinsäuresequenz codonoptimiert ist.

**22.** Produktionssystem gemäß einem der Ansprüche 19 bis 21, wobei die für wenigstens eines der Hilfsgene vpr, vif, tat und nef, oder analoge Hilfsgene aus dem Lentivirus, von dem die Partikel abgeleitet sind, codierenden Nukleinsäuresequenzen ebenfalls unterbrochen sind, so dass die Hilfsgene nicht für die funktionellen Hilfsproteine codieren können, oder aus dem System entfernt sind.

**23.** DNA-Konstrukt zur Verwendung in dem System gemäß einem der Ansprüche 19 bis 22, wobei das DNA-Konstrukt für ein verpackbares RNA-Vektorgenom mit der in einem der Ansprüche 1 bis 18 angegebenen Bedeutung codiert.

**24.** Satz von DNA-Konstrukten, umfassend das DNA-Konstrukt gemäß Anspruch 23 sowie ein DNA-Konstrukt, das für Gag und Pol mit der in einem der Ansprüche 19 bis 21 angegebenen Bedeutung codiert.

**25.** Satz von DNA-Konstrukten gemäß Anspruch 24, ferner umfassend ein DNA-Konstrukt, das für Env oder einen funktionellen Ersatz davon codiert.

**26.** Verfahren zur Herstellung eines lentiviralen Vektorpartikels, wobei man einen Satz Nukleinsäuresequenzen oder DNA-Konstrukte mit der in einem der Ansprüche 23 bis 25 angegebenen Bedeutung in eine Wirtszelle einführt und das lentivirale Vektorpartikel gewinnt.

**27.** Lentivirales Vektorpartikel, hergestellt mit dem System, DNA-Konstrukt oder Satz DNA-Konstrukte oder Verfahren gemäß einem der Ansprüche 19 bis 22, 25 und 26.

**28.** Von Lentivirus abgeleitetes Vektorpartikel, umfassend ein Vektorgenom mit der in einem der Ansprüche 1 bis 18 angegebenen Bedeutung, Gag- und Pol-Proteine und Env-Protein oder einen funktionellen Ersatz dafür.

**29.** Zelle, transduziert mit dem lentiviralen Vektorpartikel nach Anspruch 27 oder 28 oder dem DNA-Konstrukt oder Satz DNA-Konstrukte nach einem der Ansprüche 23 bis 25.

**30.** Lentivirales Vektorpartikel nach Anspruch 27 oder 28 oder DNA-Konstrukt oder Satz DNA-Konstrukte nach einem der Ansprüche 23 bis 25 oder Zelle nach Anspruch 29 zur Verwendung in der Medizin.

**31.** Pharmazeutische Zusammensetzung, umfassend das Vektorgenom nach einem der Ansprüche 1 bis 18, das System nach einem der Ansprüche 19 bis 22, das DNA-Konstrukt oder den Satz DNA-Konstrukte nach einem der Ansprüche 23 bis 25, ein Partikel nach Anspruch 27 oder 28 oder eine Zelle nach Anspruch 29 zusammen mit einem pharmazeutisch unbedenklichen Träger- oder Verdünnungsmittel.

**32.** Verwendung eines lentiviralen Vektorpartikels nach Anspruch 27 oder 28 oder eines DNA-Konstrukts oder eines Satzes DNA-Konstrukte nach einem der Ansprüche 23 bis 25 oder einer Zelle nach Anspruch 29 zur Herstellung eines Arzneimittels zur Zuführung eines NOI an eine Zielstelle, die dieses benötigt.

**33.** Verwendung nach Anspruch 32, wobei das Arzneimittel zur Behandlung einer neurogenerativen Erkrankung wie Morbus Parkinson vorgesehen ist.

**34.** Zuführungssystem in Form eines lentiviralen Vektorpartikels nach Anspruch 27 oder 28 oder eines DNA-Konstrukts oder Satzes DNA-Konstrukte nach einem der Ansprüche 23 bis 25 oder einer Zelle nach Anspruch 29 zur Verwendung in der Medizin.

## Revendications

**1.** Génome de vecteur multicistronique pouvant dériver d'un lentivirus, pour utilisation dans un système de production lentiviral *rev*-indépendant, pour la production d'une particule de vecteur dérivant d'un lentivirus, ledit génome comprenant un signal d'encapsidation, un cadre ouvert de lecture (ORF) hétérologue en aval d'un LTR viral et en amont d'un promoteur, dans lequel le promoteur contrôle l'expression d'au moins un nucléotide d'intérêt (NOI) aval utile dans le traitement d'un trouble neurodégénératif, et dans lequel l'ORF hétérologue est lié de manière opérationnelle au LTR ; dans lequel la séquence d'acide nucléique codant pour le gène auxiliaire *rev* est interrompue de telle sorte que ledit gène auxiliaire soit incapable de coder pour la protéine auxiliaire fonctionnelle, ou est éliminée du génome du vecteur lentiviral, et dans lequel l'élément de réponse à Rev (RRE) est interrompu de telle sorte que ledit RRE soit non fonctionnel, ou soit éliminé du génome du vecteur lentiviral, et dans lequel le vecteur lentiviral est un vecteur lentiviral à auto-inactivation.

2. Génome de vecteur de la revendication 1, dans lequel le NOI est utile dans le traitement de la maladie de Parkinson.

3. Génome de vecteur de la revendication 2 dans lequel le NOI est impliqué dans la synthèse de dopamine.

4. Génome de vecteur selon l'une quelconque des revendications 1 à 3 dans lequel le NOI code pour l'un ou plusieurs parmi la tyrosine hydroxylase, la GTP-cyclohydrolase I, l'acide aminé aromatique Dopa décarboxylase ou le transporteur de monoamine vésiculaire 2 (VMAT2).

5. Génome de vecteur selon la revendication 1 dans lequel le NOI code pour un facteur capable de bloquer ou inhiber la dégénérescence dans le système nigrostriatal.

6. Génome de vecteur selon la revendication 5 dans lequel le facteur est un facteur neurotrophique.

7. Génome de vecteur selon la revendication 5 dans lequel le facteur est un facteur neurotrophique dérivé de lignée cellulaire gliale (GDNF) ou un facteur neurotrophique dérivé de cerveau (BDNF).

8. Génome de vecteur selon la revendication 1, dans lequel le NOI code pour un facteur neuroprotecteur.

9. Génome de vecteur de l'une quelconque des revendications précédentes, dans lequel l'ORF hétérologue est un fragment rapporteur ou un marqueur sélectionnable.

10. Génome de vecteur de l'une quelconque des revendications précédentes, dans lequel l'ORF hétérologue code pour un gène modulateur.

11. Génome de vecteur de la revendication 10, dans lequel le gène modulateur est choisi parmi les répresseurs de la tétracycline tels que TetR, et les transactivateurs contrôlés par la tétracycline, tels que tTA et rtTA.

12. Génome de vecteur selon la revendication 11, dans lequel le répresseur de la tétracycline est à codon optimisé pour expression dans une cellule de mammifère.

13. Génome de vecteur selon l'une quelconque des revendications précédentes, le génome de vecteur pouvant dériver d'un vecteur lentiviral de non-primate.

14. Génome de vecteur selon l'une quelconque des revendications précédentes, le génome de vecteur pouvant dériver d'un lentivirus choisi dans le groupe consistant en les virus VIH-1, VIH-2, SIV, FIV, BIV, EIAV, CAEV, MVV, et le vecteur lentiviral visna.

15. Génome de vecteur selon l'une quelconque des revendications précédentes, le génome de vecteur comprenant une séquence polypurine tract centrale (cPPT).

16. Génome de vecteur selon l'une quelconque des revendications précédentes, le génome de vecteur comprenant un élément régulateur post-transcriptionnel ou un élément traductionnel.

17. Génome de vecteur selon l'une quelconque des revendications précédentes, dans lequel tous les motifs ATG qui dérivent de *gag* et font partie du signal d'encapsidation ont été modifiés pour lire ATTG.

18. Génome de vecteur selon l'une quelconque des revendications précédentes, le génome de vecteur étant bi-, tricistronique ou quadricistronique.

19. Système de production d'un vecteur lentiviral pour la production d'une particule de vecteur dérivant d'un lentivirus, ledit système comprenant un ensemble de séquences d'acides nucléiques codant pour les composants du génome de vecteur comprenant le génome de vecteur de l'une quelconque des revendications 1 à 18, les protéines Gag et Pol et la protéine Env ou un substitut fonctionnel de cette dernière, et dans lequel une séquence d'acide nucléique codant pour le gène auxiliaire *rev* est interrompue de telle sorte que ledit gène auxiliaire soit incapable de coder pour la protéine auxiliaire fonctionnelle, ou ne soit pas présent dans le système de production d'un vecteur lentiviral, et ne soit pas fourni en *trans,* et le RRE est interrompu de telle sorte que le RRE soit non fonctionnel, ou ne soit pas présent dans le système de production d'un vecteur lentiviral, et ne soit pas fourni en *trans.*

20. Système de production selon la revendication 19, dans lequel l'ensemble de séquences d'acides nucléiques comprend trois constructions d'ADN codant (i) pour le génome du vecteur lentiviral, (ii) pour Gag et Pol, et (iii) pour Env.

21. Système de production selon la revendication 19 ou 20, dans lequel la séquence d'acide nucléique codant pour Gag et Pol est à codon optimisé.

22. Système de production selon l'une quelconque des revendications 19 à 21, dans lequel les séquences d'acides nucléiques codant pour au moins l'un des gènes auxiliaires *vpr, vif, tat* et *nef,* ou des gènes auxiliaires analogues, provenant du lentivirus dont dérivent lesdites particules, sont elles aussi interrompues de telle sorte que lesdits gènes auxiliaires soient incapables de coder pour les protéines auxiliaires fonctionnelles, ou sont enlevées du système.

23. Construction d'ADN pour utilisation dans le système selon l'une quelconque des revendications 19 à 22, ladite construction d'ADN codant pour un génome de vecteur ARN encapsidable tel que défini dans l'une quelconque des revendications 1 à 18.

24. Ensemble de constructions d'ADN comprenant la construction d'ADN selon la revendication 23 et une construction d'ADN codant pour Gag et Pol, telle que définie dans l'une quelconque des revendications 19 à 21.

25. Ensemble de constructions d'ADN selon la revendication 24, comprenant en outre une construction d'ADN codant pour Env ou un substitut fonctionnel de ce dernier.

26. Procédé de préparation d'une particule d'un vecteur lentiviral, comprenant l'introduction, dans une cellule hôte, d'un ensemble de séquences d'acides nucléiques ou de constructions d'ADN telles que définies dans l'une quelconque des revendications 23 à 25, et l'obtention de la particule du vecteur lentiviral.

27. Particule de vecteur lentiviral produite par le système, la construction d'ADN ou l'ensemble de constructions d'ADN ou par le procédé de l'une quelconque des revendications 19 à 22, 25 et 26.

28. Particule de vecteur dérivant d'un lentivirus, comprenant un génome de vecteur tel que défini dans l'une quelconque des revendications 1 à 18, les protéines Gag et Pol et une protéine Env, ou un substitut fonctionnel de cette dernière.

29. Cellule transduite avec la particule de vecteur lentiviral de la revendication 27 ou 28 ou la construction d'ADN ou l'ensemble de constructions d'ADN de l'une quelconque des revendications 23 à 25.

30. Particule de vecteur lentiviral de la revendication 27 ou 28 ou construction d'ADN ou ensemble de constructions d'ADN de l'une quelconque des revendications 23 à 25 ou cellule de la revendication 29, pour utilisation en médecine.

31. Composition pharmaceutique comprenant le génome de vecteur de l'une quelconque des revendications 1 à 18, le système de l'une quelconque des revendications 19 à 22, la construction d'ADN ou l'ensemble de constructions d'ADN de l'une quelconque des revendications 23 à 25, une particule de la revendication 27 ou 28 ou une cellule de la revendication 29, en même temps qu'un support ou un diluant pharmaceutiquement acceptable.

32. Utilisation d'une particule de vecteur lentiviral de la revendication 27 ou 28 ou d'une construction d'ADN ou d'un ensemble de constructions d'ADN de l'une quelconque des revendications 23 à 25 ou d'une cellule de la revendication 29 pour la préparation d'un médicament destiné à délivrer un NOI sur un site cible qui en a besoin.

33. Utilisation de la revendication 32, dans laquelle le médicament est destiné au traitement d'un trouble neurodégénératif, tel que la maladie de Parkinson.

34. Système d'administration sous forme d'une particule de vecteur lentiviral de la revendication 27 ou 28 ou d'une construction d'ADN ou d'un ensemble de constructions d'ADN de l'une quelconque des revendications 23 à 25 ou d'une cellule de la revendication 29 pour utilisation en médecine.

Fig. 1

Size (kb)

pONY8.1 G

2.6

pONY8.4ZCG

6.1

pONY8.GCZ

6.1

CMV promoter

EIAV U3

EIAV R region

EIAV U5 region

Packaging signal

Modified packaging signal

Hybrid U3

# Figure 2.

```
AGATCTTGAATAATAAAATGTGTGTTTGTCCGAAATACGCGTTTTGAGATTTCTGTCGCC
GACTAAATTCATGTCGCGCGATAGTGGTGTTTATCGCCGATAGAGATGGCGATATTGGAA
AAATTGATATTTGAAAATATGGCATATTGAAAATGTCGCCGATGTGAGTTTCTGTGTAAC
TGATATCGCCATTTTTCCAAAAGTGATTTTTGGGCATACGCGATATCTGGCGATAGCGCT
TATATCGTTTACGGGGGATGGCGATAGACGACTTTGGTGACTTGGGCGATTCTGTGTGTC
GCAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCA
TACGTTGTATCCATATCGTAATATGTACATTTATATTGGCTCATGTCCAACATTACCGCC
ATGTTGACATTGATTATTGACTAGTTATTAATAGTAATCAATTACGGGGTCATTAGTTCA
TAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAGT
ACATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTGG
ATAGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTGCGATCGCCCGCC
CCGTTGACGCAAATGGGCGGTAGGCGTGTACGGTGGGAGGTCTATATAAGCAGAGCTCGT
TTAGTGAACCGGGCACTCAGATTCTGCGGTCTGAGTCCCTTCTCTGCTGGGCTGAAAAGG
CCTTTGTAATAAATATAATTCTCTACTCAGTCCCTGTCTCTAGTTTGTCTGTTCGAGATC
CTACAGTTGGCGCCCGAACAGGGACCTGAGAGGGGCGCAGACCCTACCTGTTGAACCTGG
CTGATCGTAGGATCCCCGGGACAGCAGAGGAGAACTTACAGAAGTCTTCTGGAGGTGTTC
CTGGCCAGAACACAGGAGGACAGGTAAGATTGGGAGACCCTTTGACATTGGAGCAAGGC
G
CTCAAGAAGTTAGAGAAGGTGACGGTACAAGGGTCTCAGAAATTAACTACTGGTAACTGT
AATTGGGCGCTAAGTCTAGTAGACTTATTTCATGATACCAACTTTGTAAAAGAAAAGGAC
TGGCAGCTGAGGGATGTCATTCCATTGCTGGAAGATGTAACTCAGACGCTGTCAGGACAA
GAAAGAGAGGCCTTTGAAAGAACATGGTGGGCAATTTCTGCTGTAAAGATGGGCCTCCAG
ATTAATAATGTAGTAGATGGAAAGGCATCATTCCAGCTCCTAAGAGCGAAATATGAAAAG
AAGACTGCTAATAAAAAGCAGTCTGAGCCCTCTGAAGAATATCTCTAGAACTAGTGGATC
CCCCGGGCTGCAGGAGTGGGGAGGCACGATGGCCGCTTTGGTCGAGGCGGATCCGGCCAT
TAGCCATATTATTCATTGGTTATATAGCATAAATCAATATTGGCTATTGGCCATTGCATA
CGTTGTATCCATATCATAATATGTACATTTATATTGGCTCATGTCCAACATTACCGCCAT
GTTGACATTGATTATTGACTAGTTATTAATAGTAATCAATTACGGGGTCATTAGTTCATA
GCCCATATATGGAGTTCCGCGTTACATAACTTACGGTAAATGGCCCGCCTGGCTGACCGC
CCAACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAGTAC
ATCAAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTACG
TATTAGTCATCGCTATTACCATGGTGATGCGGTTTTGGCAGTACATCAATGGGCGTGGAT
AGCGGTTTGACTCACGGGGATTTCCAAGTCTCCACCCCATTGACGTCAATGGGAGTTTGT
TTTGGCACCAAAATCAACGGGACTTTCCAAAATGTCGTAACAACTCCGCCCCATTGACGC
AAATGGGCGGTAGGCATGTACGGTGGGAGGTCTATATAAGCAGAGCTCGTTTAGTGAACC
GTCAGATCGCCTGGAGACGCCATCCACGCTGTTTTGACCTCCATAGAAGACACCGGGACC
GATCCAGCCTCCGCGGCCCCAAGCTTGTTGGGATCCACCGGTCGCCACCATGGTGAGCAA
GGGCGAGGAGCTGTTCACCGGGGTGGTGCCCATCCTGGTCGAGCTGGACGGCGACGTAAA
CGGCCACAAGTTCAGCGTGTCCGGCGAGGGCGAGGGCGATGCCACCTACGGCAAGCTGAC
CCTGAAGTTCATCTGCACCACCGGCAAGCTGCCCGTGCCCTGGCCCACCCTCGTGACCAC
CCTGACCTACGGCGTGCAGTGCTTCAGCCGCTACCCCGACCACATGAAGCAGCACGACTT
CTTCAAGTCCGCCATGCCCGAAGGCTACGTCCAGGAGCGCACCATCTTCTTCAAGGACGA
CGGCAACTACAAGACCCGCGCCGAGGTGAAGTTCGAGGGCGACACCCTGGTGAACCGCAT
CGAGCTGAAGGGCATCGACTTCAAGGAGGACGGCAACATCCTGGGGCACAAGCTGGAGT
ACAACTACAACAGCCACAACGTCTATATCATGGCCGACAAGCAGAAGAACGGCATCAAG
GTGAACTTCAAGATCCGCCACAACATCGAGGACGGCAGCGTGCAGCTCGCCGACCACTAC
```

CAGCAGAACACCCCCATCGGCGACGGCCCCGTGCTGCTGCCCGACAACCACTACCTGAGC
ACCCAGTCCGCCCTGAGCAAAGACCCCAACGAGAAGCGCGATCACATGGTCCTGCTGGAG
TTCGTGACCGCCGCCGGGATCACTCTCGGCATGGACGAGCTGTACAAGTAAAGCGGCCGC
GACTCTAGAGTCGACCTGCAGGAATTCGATATCAAGCTTATCGATACCGTCGAATTGGAA
GAGCTTTAAATCCTGGCACATCTCATGTATCAATGCCTCAGTATGTTTAGAAAAACAAGGG
GGGAACTGTGGGGTTTTTATGAGGGGTTTTATAAATGATTATAAGAGTAAAAAGAAAGTT
GCTGATGCTCTCATAACCTTGTATAACCCAAAGGACTAGCTCATGTTGCTAGGCAACTAAA
CCGCAATAACCGCATTTGTGACGCGAGTTCCCCATTGGTGACGCGTTAACTTCCTGTTTT
TACAGTATATAAGTGCTTGTATTCTGACAATTGGGCACTCAGATTCTGCGGTCTGAGTCC
CTTCTCTGCTGGGCTGAAAAGGCCTTTGTAATAAATATAATTCTCTACTCAGTCCCTGTC
TCTAGTTTGTCTGTTCGAGATCCTACAGAGCTCATGCCTTGGCGTAATCATGGTCATAGC
TGTTTCCTGTGTGAAATTGTTATCCGCTCACAATTCCACACAACATACGAGCCGGAAGCA
TAAAGTGTAAAGCCTGGGGTGCCTAATGAGTGAGCTAACTCACATTAATTGCGTTGCGCT
CACTGCCCGCTTTCCAGTCGGGAAACCTGTCGTGCCAGCTGCATTAATGAATCGGCCAAC
GCGCGGGGAGAGGCGGTTTGCGTATTGGGCGCTCTTCCGCTTCCTCGCTCACTGACTCGC
TGCGCTCGGTCGTTCGGCTGCGGCGAGCGGTATCAGCTCACTCAAAGGCGGTAATACGGT
TATCCACAGAATCAGGGGATAACGCAGGAAAGAACATGTGAGCAAAAGGCCAGCAAAAG
GCCAGGAACCGTAAAAAGGCCGCGTTGCTGGCGTTTTTCCATAGGCTCCGCCCCCCTGACG
AGCATCACAAAAATCGACGCTCAAGTCAGAGGTGGCGAAACCCGACAGGACTATAAAGA
TACCAGGCGTTTCCCCCTGGAAGCTCCCTCGTGCGCTCTCCTGTTCCGACCCTGCCGCTTA
CCGGATACCTGTCCGCCTTTCTCCCTTCGGGAAGCGTGGCGCTTTCTCATAGCTCACGCT
GTAGGTATCTCAGTTCGGTGTAGGTCGTTCGCTCCAAGCTGGGCTGTGTGCACGAACCCC
CCGTTCAGCCCGACCGCTGCGCCTTATCCGGTAACTATCGTCTTGAGTCCAACCCGGTAA
GACACGACTTATCGCCACTGGCAGCAGCCACTGGTAACAGGATTAGCAGAGCGAGGTATG
TAGGCGGTGCTACAGAGTTCTTGAAGTGGTGGCCTAACTACGGCTACACTAGAAGGACAG
TATTTGGTATCTGCGCTCTGCTGAAGCCAGTTACCTTCGGAAAAAGAGTTGGTAGCTCTT
GATCCGGCAAACAAACCACCGCTGGTAGCGGTGGTTTTTTTGTTTGCAAGCAGCAGATTA
CGCGCAGAAAAAAAGGATCTCAAGAAGATCCTTTGATCTTTTCTACGGGGTCTGACGCTC
AGTGGAACGAAAACTCACGTTAAGGGATTTTGGTCATGAGATTATCAAAAAGGATCTTCA
CCTAGATCCTTTTAAATTAAAAATGAAGTTTTAAATCAATCTAAAGTATATATGAGTAAA
CTTGGTCTGACAGTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCT
TACCATCTGGCCCCAGTGCTGCAATGATACCGCGAGACCCACGCTCACCGGCTCCAGATT
TATCAGCAATAAACCAGCCAGCCGGAAGGGCCGAGCGCAGAAGTGGTCCTGCAACTTTAT
CCGCCTCCATCCAGTCTATTAATTGTTGCCGGGAAGCTAGAGTAAGTAGTTCGCCAGTTA
ATAGTTTGCGCAACGTTGTTGCCATTGCTACAGGCATCGTGGTGTCACGCTCGTCGTTTG
GTATGGCTTCATTCAGCTCCGGTTCCCAACGATCAAGGCGAGTTACATGATCCCCCATGT
TGTGCAAAAAAGCGGTTAGCTCCTTCGGTCCTCCGATCGTTGTCAGAAGTAAGTTGGCCG
CAGTGTTATCACTCATGGTTATGGCAGCACTGCATAATTCTCTTACTGTCATGCCATCCG
TAAGATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCAGAA
CTTTAAAAGTGCTCATCATTGGAAAACGTTCTTCGGGGCGAAAACTCTCAAGGATCTTAC
CGCTGTTGAGATCCAGTTCGATGTAACCCACTCGTGCACCCAACTGATCTTCAGCATCTT
TTACTTTCACCAGCGTTTCTGGGTGAGCAAAAACAGGAAGGCAAAATGCCGCAAAAAAGG
GAATAAGGGCGACACGGAAATGTTGAATACTCATACTCTTCCTTTTTCAATATTATTGAA
GCATTTATCAGGGTTATTGTCTCATGAGCGGATACATATTTGAATGTATTTAGAAAAATA
AACAAATAGGGGTTCCGCGCACATTTCCCCGAAAAGTGCCACCTAAATTGTAAGCGTTAA
TATTTTGTTAAAATTCGCGTTAAATTTTTGTTAAATCAGCTCATTTTTTAACCAATAGGC
CGAAATCGGCAAAATCCCTTATAAATCAAAAGAATAGACCGAGATAGGGTTGAGTGTTGT
TCCAGTTTGGAACAAGAGTCCACTATTAAAGAACGTGGACTCCAACGTCAAAGGGCGAAA
AACCGTCTATCAGGGCGATGGCCCACTACGTGAACCATCACCCTAATCAAGTTTTTTGGG
GTCGAGGTGCCGTAAAGCACTAAATCGGAACCCTAAAGGGAGCCCCGATTTAGAGCTTG
ACGGGGAAAGCCAACCTGGCTTATCGAAATTAATACGACTCACTATAGGGAGACCGGC

## Figure 3

AGATCTTGAATAATAAAATGTGTGTTTGTCCGAAATACGCGTTTTGAGATTTCTGTCGCC
GACTAAATTCATGTCGCGCGATAGTGGTGTTATCGCCGATAGAGATGGCGATATTGGAA
AAATTGATATTTGAAAATATGGCATATTGAAAATGTCGCCGATGTGAGTTTCTGTGTAAC
TGATATCGCCATTTTTCCAAAAGTGATTTTTGGGCATACGCGATATCTGGCGATAGCGCT
TATATCGTTTACGGGGGATGGCGATAGACGACTTTGGTGACTTGGGCGATTCTGTGTGTC
GCAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCA
TACGTTGTATCCATATCGTAATATGTACATTTATATTGGCTCATGTCCAACATTACCGCC
ATGTTGACATTGATTATTGACTAGTTATTAATAGTAATCAATTACGGGGTCATTAGTTCA
TAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAGT
ACATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTGG
ATAGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTGCGATCGCCCGCC
CCGTTGACGCAAATGGGCGGTAGGCGTGTACGGTGGGAGGTCTATATAAGCAGAGCTCGT
TTAGTGAACCGGGCACTCAGATTCTGCGGTCTGAGTCCCTTCTCTGCTGGGCTGAAAAGG
CCTTTGTAATAAATATAATTCTCTACTCAGTCCCTGTCTCTAGTTTGTCTGTTCGAGATC
CTACAGTTGGCGCCCGAACAGGGACCTGAGAGGGGCGCAGACCCTACCTGTTGAACCTGG
CTGATCGTAGGATCCCCGGGACAGCAGAGGAGAACTTACAGAAGTCTTCTGGAGGTGTTC
CTGGCCAGAACACAGGAGGACAGGTAAGATTGGGAGACCCTTTGACATTGGAGCAAGGC
G
CTCAAGAAGTTAGAGAAGGTGACGGTACAAGGGTCTCAGAAATTAACTACTGGTAACTGT
AATTGGGCGCTAAGTCTAGTAGACTTATTTCATTGATACCAACTTTGTAAAAGAAAAGGA
CTGGCAGCTGAGGGATTGTCATTCCATTGCTGGAAGATTGTAACTCAGACGCTGTCAGGA
CAAGAAAGAGAGGCCTTTGAAAGAACATTGGTGGGCAATTTCTGCTGTAAAGATTGGGCC
TCCAGATTAATAATTGTAGTAGATTGGAAAGGCATCATTCCAGCTCCTAAGAGCGAAATA
TTGAAAAGAAGACTGCTAATAAAAAGCAGTCTGAGCCCTCTGAAGAATATCTCTAGAACT
AGTGGATCCCCCGGGCTGCAGGAATTCGATATCAAGCTTCAGCTGCTCGAGGATCTGCGG
ATCCGGGGAATTCCCCAGTCTCAGGATCCACCATGGGGGATCCCGTCGTTTTACAACGTC
GTGACTGGGAAAACCCTGGCGTTACCCAACTTAATCGCCTTGCAGCACATCCCCCTTTCG
CCAGCTGGCGTAATAGCGAAGAGGCCCGCACCGATCGCCCTTCCCAACAGTTGCGCAGCC
TGAATGGCGAATGGCGCTTTGCCTGGTTTCCGGCACCAGAAGCGGTGCCGGAAAGCTGGC
TGGAGTGCGATCTTCCTGAGGCCGATACTGTCGTCGTCCCCTCAAACTGGCAGATGCACG
GTTACGATGCGCCCATCTACACCAACGTAACCTATCCCATTACGGTCAATCCGCCGTTTG
TTCCCACGGAGAATCCGACGGGTTGTTACTCGCTCACATTTAATGTTGATGAAAGCTGGC
TACAGGAAGGCCAGACGCGAATTATTTTTGATGGCGTTAACTCGGCGTTTCATCTGTGGT
GCAACGGGCGCTGGGTCGGTTACGGCCAGGACAGTCGTTTGCCGTCTGAATTTGACCTGA
GCGCATTTTTACGCGCCGGAGAAAACCGCCTCGCGGTGATGGTGCTGCGTTGGAGTGACG
GCAGTTATCTGGAAGATCAGGATATGTGGCGGATGAGCGGCATTTTCCGTGACGTCTCGT
TGCTGCATAAACCGACTACACAAATCAGCGATTTCCATGTTGCCACTCGCTTTAATGATG
ATTTCAGCCGCGCTGTACTGGAGGCTGAAGTTCAGATGTGCGGCGAGTTGCGTGACTACC
TACGGGTAACAGTTTCTTTATGGCAGGGTGAAACGCAGGTCGCCAGCGGCACCGCGCCTT
TCGGCGGTGAAATTATCGATGAGCGTGGTGGTTATGCCGATCGCGTCACACTACGTCTGA
ACGTCGAAAACCCGAAACTGTGGAGCGCCGAAATCCCGAATCTCTATCGTGCGGTGGTTG
AACTGCACACCGCCGACGGCACGCTGATTGAAGCAGAAGCCTGCGATGTCGGTTTCCGCG
AGGTGCGGATTGAAAATGGTCTGCTGCTGCTGAACGGCAAGCCGTTGCTGATTCGAGGCG
TTAACCGTCACGAGCATCATCCTCTGCATGGTCAGGTCATGGATGAGCAGACGATGGTGC
AGGATATCCTGCTGATGAAGCAGAACAACTTTAACGCCGTGCGCTGTTCGCATTATCCGA
ACCATCCGCTGTGGTACACGCTGTGCGACCGCTACGGCCTGTATGTGGTGGATGAAGCCA
ATATTGAAACCCACGGCATGGTGCCAATGAATCGTCTGACCGATGATCCGCGCTGGCTAC
CGGCGATGAGCGAACGCGTAACGCGAATGGTGCAGCGCGATCGTAATCACCCGAGTGTGA
TCATCTGGTCGCTGGGGAATGAATCAGGCCACGGCGCTAATCACGACGCGCTGTATCGCT

GGATCAAATCTGTCGATCCTTCCCGCCCGGTGCAGTATGAAGGCGGCGGAGCCGACACCA
CGGCCACCGATATTATTTGCCCGATGTACGCGCGCGTGGATGAAGACCAGCCCTTCCCGG
CTGTGCCGAAATGGTCCATCAAAAAATGGCTTTCGCTACCTGGAGAGACGCGCCCGCTGA
TCCTTTGCGAATACGCCCACGCGATGGGTAACAGTCTTGGCGGTTTCGCTAAATACTGGC
AGGCGTTTCGTCAGTATCCCCGTTACAGGGCGGCTTCGTCTGGGACTGGGTGGATCAGT
CGCTGATTAAATATGATGAAAACGGCAACCCGTGGTCGGCTTACGGCGGTGATTTTGGCG
ATACGCCGAACGATCGCCAGTTCTGTATGAACGGTCTGGTCTTTGCCGACCGCACGCCGC
ATCCAGCGCTGACGGAAGCAAAACACCAGCAGCAGTTTTTCCAGTTCCGTTTATCCGGGC
AAACCATCGAAGTGACCAGCGAATACCTGTTCCGTCATAGCGATAACGAGCTCCTGCACT
GGATGGTGGCGCTGGATGGTAAGCCGCTGGCAAGCGGTGAAGTGCCTCTGGATGTCGCTC
CACAAGGTAAACAGTTGATTGAACTGCCTGAACTACCGCAGCCGGAGAGCGCCGGGCAAC
TCTGGCTCACAGTACGCGTAGTGCAACCGAACGCGACCGCATGGTCAGAAGCCGGGCACA
TCAGCGCCTGGCAGCAGTGGCGTCTGGCGGAAAACCTCAGTGTGACGCTCCCCGCCGCGT
CCCACGCCATCCCGCATCTGACCACCAGCGAAATGGATTTTGCATCGAGCTGGGTAATA
AGCGTTGGCAATTTAACCGCCAGTCAGGCTTTCTTTCACAGATGTGGATTGGCGATAAAA
AACAACTGCTGACGCCGCTGCGCGATCAGTTCACCCGTGCACCGCTGGATAACGACATTG
GCGTAAGTGAAGCGACCCGCATTGACCCTAACGCCTGGGTCGAACGCTGGAAGGCGGCGG
GCCATTACCAGGCCGAAGCAGCGTTGTTGCAGTGCACGGCAGATACACTTGCTGATGCGG
TGCTGATTACGACCGCTCACGCGTGGCAGCATCAGGGGAAAACCTTATTTATCAGCCGGA
AAACCTACCGGATTGATGGTAGTGGTCAAATGGCGATTACCGTTGATGTTGAAGTGGCGA
GCGATACACCGCATCCGGCGCGGATTGGCCTGAACTGCCAGCTGGCGCAGGTAGCAGAGC
GGGTAAACTGGCTCGGATTAGGGCCGCAAGAAAACTATCCCGACCGCCTTACTGCCGCCT
GTTTTGACCGCTGGGATCTGCCATTGTCAGACATGTATACCCCGTACGTCTTCCCGAGCG
AAAACGGTCTGCGCTGCGGGACGCGCGAATTGAATTATGGCCCACACCAGTGGCGCGGCG
ACTTCCAGTTCAACATCAGCCGCTACAGTCAACAGCAACTGATGGAAACCAGCCATCGCC
ATCTGCTGCACGCGGAAGAAGGCACATGGCTGAATATCGACGGTTTCCATATGGGGATTG
GTGGCGACGACTCCTGGAGCCCGTCAGTATCGGCGGAATTCCAGCTGAGCGCCGGTCGCT
ACCATTACCAGTTGGTCTGGTGTCAAAAATAATAATAACCGGGCAGGGGGGATCCGCAGA
TCCGGCTGTGGAATGTGTGTCAGTTAGGGTGTGGAAAGTCCCCAGGCTCCCCAGCAGGCA
GAAGTATGCAAAGCTAGAACTAGTGGATCCCCCGGGCTGCAGGAGTGGGGAGGCACGAT
G
GCCGCTTTGGTCGAGGCGGATCCGGCCATTAGCCATATTATTCATTGGTTATATAGCATA
AATCAATATTGGCTATTGGCCATTGCATACGTTGTATCCATATCATAATATGTACATTTA
TATTGGCTCATGTCCAACATTACCGCCATGTTGACATTGATTATTGACTAGTTATTAATA
GTAATCAATTACGGGGTCATTAGTTCATAGCCCATATATGGAGTTCCGCGTTACATAACT
TACGGTAAATGGCCCGCCTGGCTGACCGCCCAACGACCCCCGCCCATTGACGTCAATAAT
GACGTATGTTCCCATAGTAACGCCAATAGGGACTTTCCATTGACGTCAATGGGTGGAGTA
TTTACGGTAAACTGCCCACTTGGCAGTACATCAAGTGTATCATATGCCAAGTACGCCCCC
TATTGACGTCAATGACGGTAAATGGCCCGCCTGGCATTATGCCCAGTACATGACCTTATG
GGACTTTCCTACTTGGCAGTACATCTACGTATTAGTCATCGCTATTACCATGGTGATGCG
GTTTTGGCAGTACATCAATGGGCGTGGATAGCGGTTTGACTCACGGGGATTTCCAAGTCT
CCACCCCATTGACGTCAATGGGAGTTTGTTTTGGCACCAAAATCAACGGGACTTTCCAAA
ATGTCGTAACAACTCCGCCCCATTGACGCAAATGGGCGGTAGGCATGTACGGTGGGAGGT
CTATATAAGCAGAGCTCGTTTAGTGAACCGTCAGATCGCCTGGAGACGCCATCCACGCTG
TTTTGACCTCCATAGAAGACACCGGGACCGATCCAGCCTCCGCGGCCCCAAGCTTGTTGG
GATCCACCGGTCGCCACCATGGTGAGCAAGGGCGAGGAGCTGTTCACCGGGGTGGTGCCC
ATCCTGGTCGAGCTGGACGGCGACGTAAACGGCCACAAGTTCAGCGTGTCCGGCGAGGGC
GAGGGCGATGCCACCTACGGCAAGCTGACCCTGAAGTTCATCTGCACCACCGGCAAGCTG
CCCGTGCCCTGGCCCACCCTCGTGACCACCCTGACCTACGGCGTGCAGTGCTTCAGCCGC
TACCCCGACCACATGAAGCAGCACGACTTCTTCAAGTCCGCCATGCCCGAAGGCTACGTC
CAGGAGCGCACCATCTTCTTCAAGGACGACGGCAACTACAAGACCCGCGCCGAGGTGAAG
TTCGAGGGCGACACCCTGGTGAACCGCATCGAGCTGAAGGGCATCGACTTCAAGGAGGAC
GGCAACATCCTGGGGCACAAGCTGGAGTACAACTACAACAGCCACAACGTCTATATCATG
GCCGACAAGCAGAAGAACGGCATCAAGGTGAACTTCAAGATCCGCCACAACATCGAGGA
C
GGCAGCGTGCAGCTCGCCGACCACTACCAGCAGAACACCCCCATCGGCGACGGCCCCGTG
CTGCTGCCCGACAACCACTACCTGAGCACCCAGTCCGCCCTGAGCAAAGACCCCAACGAG
AAGCGCGATCACATGGTCCTGCTGGAGTTCGTGACCGCCGCCGGGATCACTCTCGGCATG

GACGAGCTGTACAAGTAAAGCGGCCGCGACTCTAGCCTGCAGGAATTCGATATCAAGCTT
ATCGATACCGTCGAATTGGAAGAGCTTTAAATCCTGGCACATCTCATGTATCAATGCCTC
AGTATGTTTAGAAAAACAAGGGGGGAACTGTGGGGTTTTTATGAGGGGTTTTATAAAAAT
GAAAGACCCCACCTGTAGGTTTGGCAAGCTAGCTTAAGTAACGCCATTTTGCAAGGCATG
GAAAAATACATAACTGAGAATAGAGAAGTTCAGATCAAGGTCAGGAACAGATGGAACAG
C
TGAATATGGGCCAAACAGGATATCTGTGGTAAGCAGTTCCTGCCCCGGCTCAGGGCCAAG
AACAGATGGAACAGCTGAATATGGGCCAAACAGGATATCTGTGGTAAGCAGTTCCTGCCC
CGGCTCAGGGCCAAGAACAGATGGTCCCCAGATGCGGTCCAGCCCTCAGCAGTTTCTAGA
GAACCATCAGATGTTTCCAGGGTGCCCCAAGGACCTGAAATGACCCTGTGCCTTATTTGA
ACTAACCAATCAGTTCGCTTCTCGCTTCTGTTCGCGCGCTTCTGCTCCCCGAGCTCAATA
AAAGAGCCCACAACCCCTCACTCGGGGGGCACTCAGATTCTGCGGTCTGAGTCCCTTCTC
TGCTGGGCTGAAAAGGCCTTTGTAATAAATATAATTCTCTACTCAGTCCCTGTCTCTAGT
TTGTCTGTTCGAGATCCTACAGAGCTCATGCCTTGGCGTAATCATGGTCATAGCTGTTTC
CTGTGTGAAATTGTTATCCGCTCACAATTCCACACAACATACGAGCCGGAAGCATAAAGT
GTAAAGCCTGGGGTGCCTAATGAGTGAGCTAACTCACATTAATTGCGTTGCGCTCACTGC
CCGCTTTCCAGTCGGGAAACCTGTCGTGCCAGCTGCATTAATGAATCGGCCAACGCGCGG
GGAGAGGCGGTTTGCGTATTGGGCGCTCTTCCGCTTCCTCGCTCACTGACTCGCTGCGCT
CGGTCGTTCGGCTGCGGCGAGCGGTATCAGCTCACTCAAAGGCGGTAATACGGTTATCCA
CAGAATCAGGGGATAACGCAGGAAAGAACATGTGAGCAAAAGGCCAGCAAAAGGCCAG
GAACCGTAAAAAGGCCGCGTTGCTGGCGTTTTTCCATAGGCTCCGCCCCCCTGACGAGCAT
CACAAAAATCGACGCTCAAGTCAGAGGTGGCGAAACCCGACAGGACTATAAAGATACCA
GGCGTTTCCCCCTGGAAGCTCCCTCGTGCGCTCTCCTGTTCCGACCCTGCCGCTTACCGGA
TACCTGTCCGCCTTTCTCCCTTCGGGAAGCGTGGCGCTTTCTCATAGCTCACGCTGTAGGT
ATCTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACACG
ACTTATCGCCACTGGCAGCAGCCACTGGTAACAGGATTAGCAGAGCGAGGTATGTAGGCG
GTGCTACAGAGTTCTTGAAGTGGTGGCCTAACTACGGCTACACTAGAAGGACAGTATTTG
GTATCTGCGCTCTGCTGAAGCCAGTTACCTTCGGAAAAAGAGTTGGTAGCTCTTGATCCG
GCAAACAAACCACCGCTGGTAGCGGTGGTTTTTTTGTTTGCAAGCAGCAGATTACGCGCA
GAAAAAAAGGATCTCAAGAAGATCCTTTGATCTTTTCTACGGGGTCTGACGCTCAGTGGA
ACGAAAACTCACGTTAAGGGATTTTGGTCATGAGATTATCAAAAAGGATCTTCACCTAGA
TCCTTTTAAATTAAAAATGAAGTTTTAAATCAATCTAAAGTATATATGAGTAAACTTGGT
CTGACAGTTACCAATGCTTAATCAGTGAGGCACCTATCTCAGCGATCTGTCTATTTCGTT
CATCCATAGTTGCCTGACTCCCCGTCGTGTAGATAACTACGATACGGGAGGGCTTACCAT
CTGGCCCCAGTGCTGCAATGATACCGCGAGACCCACGCTCACCGGCTCCAGATTTATCAG
CAATAAACCAGCCAGCCGGAAGGGCCGAGCGCAGAAGTGGTCCTGCAACTTTATCCGCCT
CCATCCAGTCTATTAATTGTTGCCGGGAAGCTAGAGTAAGTAGTTCGCCAGTTAATAGTT
TGCGCAACGTTGTTGCCATTGCTACAGGCATCGTGGTGTCACGCTCGTCGTTTGGTATGG
CTTCATTCAGCTCCGGTTCCCAACGATCAAGGCGAGTTACATGATCCCCCATGTTGTGCA
AAAAAGCGGTTAGCTCCTTCGGTCCTCCGATCGTTGTCAGAAGTAAGTTGGCCGCAGTGT
TATCACTCATGGTTATGGCAGCACTGCATAATTCTCTTACTGTCATGCCATCCGTAAGAT
GCTTTTCTGTGACTGGTGAGTACTCAACCAAGTCATTCTGAGAATAGTGTATGCGGCGAC
CGAGTTGCTCTTGCCCGGCGTCAATACGGGATAATACCGCGCCACATAGCAGAACTTTAA
AAGTGCTCATCATTGGAAAACGTTCTTCGGGGCGAAAACTCTCAAGGATCTTACCGCTGT
TGAGATCCAGTTCGATGTAACCCACTCGTGCACCCAACTGATCTTCAGCATCTTTTACTT
TCACCAGCGTTTCTGGGTGAGCAAAAACAGGAAGGCAAAATGCCGCAAAAAAGGGAATA
AGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAA
TAGGGGTTCCGCGCACATTTCCCCGAAAAGTGCCACCTAAATTGTAAGCGTTAATATTTT
GTTAAAATTCGCGTTAAATTTTTGTTAAATCAGCTCATTTTTTAACCAATAGGCCGAAAT
CGGCAAAATCCCTTATAAATCAAAAGAATAGACCGAGATAGGGTTGAGTGTTGTTCCAGT
TTGGAACAAGAGTCCACTATTAAAGAACGTGGACTCCAACGTCAAAGGGCGAAAAACCGT
CTATCAGGGCGATGGCCCACTACGTGAACCATCACCCTAATCAAGTTTTTTGGGGTCGAG
GTGCCGTAAAGCACTAAATCGGAACCCTAAAGGGAGCCCCCGATTTAGAGCTTGACGGGG
AAAGCCAACCTGGCTTATCGAAATTAATACGACTCACTATAGGGAGACCGGC

## Figure 4

AGATCTTGAATAATAAAATGTGTGTTTGTCCGAAATACGCGTTTTGAGATTTCTGTCGCC
GACTAAATTCATGTCGCGCGATAGTGGTGTTTATCGCCGATAGAGATGGCGATATTGGAA
AAATTGATATTTGAAAATATGGCATATTGAAAATGTCGCCGATGTGAGTTTCTGTGTAAC
TGATATCGCCATTTTTCCAAAAGTGATTTTTGGGCATACGCGATATCTGGCGATAGCGCT
TATATCGTTTACGGGGGATGGCGATAGACGACTTTGGTGACTTGGGCGATTCTGTGTGTC
GCAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCA
TACGTTGTATCCATATCGTAATATGTACATTTATATTGGCTCATGTCCAACATTACCGCC
ATGTTGACATTGATTATTGACTAGTTATTAATAGTAATCAATTACGGGGTCATTAGTTCA
TAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAGT
ACATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTGG
ATAGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTGCGATCGCCCGCC
CCGTTGACGCAAATGGGCGGTAGGCGTGTACGGTGGGAGGTCTATATAAGCAGAGCTCGT
TTAGTGAACCGGGCACTCAGATTCTGCGGTCTGAGTCCCTTCTCTGCTGGGCTGAAAAGG
CCTTTGTAATAAATATAATTCTCTACTCAGTCCCTGTCTCTAGTTTGTCTGTTCGAGATC
CTACAGTTGGCGCCCGAACAGGGACCTGAGAGGGGCGCAGACCCTACCTGTTGAACCTGG
CTGATCGTAGGATCCCCGGGACAGCAGAGGAGAACTTACAGAAGTCTTCTGGAGGTGTTC
CTGGCCAGAACACAGGAGGACAGGTAAGATTGGGAGACCCTTTGACATTGGAGCAAGGC
G
CTCAAGAAGTTAGAGAAGGTGACGGTACAAGGGTCTCAGAAATTAACTACTGGTAACTGT
AATTGGGCGCTAAGTCTAGTAGACTTATTTCATTGATACCAACTTTGTAAAAGAAAAGGA
CTGGCAGCTGAGGGATTGTCATTCCATTGCTGGAAGATTGTAACTCAGACGCTGTCAGGA
CAAGAAAGAGAGGCCTTTGAAAGAACATTGGTGGGCAATTTCTGCTGTAAAGATTGGGCC
TCCAGATTAATAATTGTAGTAGATTGGAAAGGCATCATTCCAGCTCCTAAGAGCGAAATA
TTGAAAAGAAGACTGCTAATAAAAAGCAGTCTGAGCCCTCTGAAGAATATCTCTAGAGTC
GACGGTACCGCGGGCCCGGGATCCACCGGTCGCCACCATGGTGAGCAAGGGCGAGGAGC
T
GTTCACCGGGGTGGTGCCCATCCTGGTCGAGCTGGACGGCGACGTAAACGGCCACAAGTT
CAGCGTGTCCGGCGAGGGCGAGGGCGATGCCACCTACGGCAAGCTGACCCTGAAGTTCAT
CTGCACCACCGGCAAGCTGCCCGTGCCCTGGCCCACCCTCGTGACCACCCTGACCTACGG
CGTGCAGTGCTTCAGCCGCTACCCCGACCACATGAAGCAGCACGACTTCTTCAAGTCCGC
CATGCCCGAAGGCTACGTCCAGGAGCGCACCATCTTCTTCAAGGACGACGGCAACTACAA
GACCCGCGCCGAGGTGAAGTTCGAGGGCGACACCCTGGTGAACCGCATCGAGCTGAAGG
G
CATCGACTTCAAGGAGGACGGCAACATCCTGGGGCACAAGCTGGAGTACAACTACAACA
G
CCACAACGTCTATATCATGGCCGACAAGCAGAAGAACGGCATCAAGGTGAACTTCAAGAT
CCGCCACAACATCGAGGACGGCAGCGTGCAGCTCGCCGACCACTACCAGCAGAACACCCC
CATCGGCGACGGCCCCGTGCTGCTGCCCGACAACCACTACCTGAGCACCCAGTCCGCCCT
GAGCAAAGACCCCAACGAGAAGCGCGATCACATGGTCCTGCTGGAGTTCGTGACCGCCGC
CGGGATCACTCTCGGCATGGACGAGCTGTACAAGTAAAGCGGCCGCTCTAGAACTAGTGG
ATCCCCCGGGCTGCAGGAGTGGGGAGGCACGATGGCCGCTTTGGTCGAGGCGGATCCGGC
CATTAGCCATATTATTCATTGGTTATATAGCATAAATCAATATTGGCTATTGGCCATTGC
ATACGTTGTATCCATATCATAATATGTACATTTATATTGGCTCATGTCCAACATTACCGC
CATGTTGACATTGATTATTGACTAGTTATTAATAGTAATCAATTACGGGGTCATTAGTTC
ATAGCCCATATATGGAGTTCCGCGTTACATAACTTACGGTAAATGGCCCGCCTGGCTGAC
CGCCCAACGACCCCCGCCCATTGACGTCAATAATGACGTATGTTCCCATAGTAACGCCAA
TAGGGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATGGC
CCGCCTGGCATTATGCCCAGTACATGACCTTATGGGACTTTCCTACTTGGCAGTACATCT

ACGTATTAGTCATCGCTATTACCATGGTGATGCGGTTTTGGCAGTACATCAATGGGCGTG
GATAGCGGTTTGACTCACGGGGATTTCCAAGTCTCCACCCCATTGACGTCAATGGGAGTT
TGTTTTGGCACCAAAATCAACGGGACTTTCCAAAATGTCGTAACAACTCCGCCCCATTGA
CGCAAATGGGCGGTAGGCATGTACGGTGGGAGGTCTATATAAGCAGAGCTCGTTTAGTGA
ACCGTCAGATCGCCTGGAGACGCCATCCACGCTGTTTTGACCTCCATAGAAGACACCGGG
ACCGATCCAGCCTCCGCGGCCCCAAGCTTCAGCTGCTCGAGGATCTGCGGATCCGGGGAA
TTCCCCAGTCTCAGGATCCACCATGGGGGATCCCGTCGTTTACAACGTCGTGACTGGGA
AAACCCTGGCGTTACCCAACTTAATCGCCTTGCAGCACATCCCCCTTTCGCCAGCTGGCG
TAATAGCGAAGAGGCCCGCACCGATCGCCCTTCCCAACAGTTGCGCAGCCTGAATGGCGA
ATGGCGCTTTGCCTGGTTTCCGGCACCAGAAGCGGTGCCGGAAAGCTGGCTGGAGTGCGA
TCTTCCTGAGGCCGATACTGTCGTCGTCCCCTCAAACTGGCAGATGCACGGTTACGATGC
GCCCATCTACACCAACGTAACCTATCCCATTACGGTCAATCCGCCGTTTGTTCCCACGGA
GAATCCGACGGGTTGTTACTCGCTCACATTTAATGTTGATGAAAGCTGGCTACAGGAAGG
CCAGACGCGAATTATTTTTGATGGCGTTAACTCGGCGTTTCATCTGTGGTGCAACGGGCG
CTGGGTCGGTTACGGCCAGGACAGTCGTTTGCCGTCTGAATTTGACCTGAGCGCATTTTT
ACGCGCCGGAGAAAACCGCCTCGCGGTGATGGTGCTGCGTTGGAGTGACGGCAGTTATCT
GGAAGATCAGGATATGTGGCGGATGAGCGGCATTTTCCGTGACGTCTCGTTGCTGCATAA
ACCGACTACACAAATCAGCGATTTCCATGTTGCCACTCGCTTTAATGATGATTTCAGCCG
CGCTGTACTGGAGGCTGAAGTTCAGATGTGCGGCGAGTTGCGTGACTACCTACGGGTAAC
AGTTTCTTTATGGCAGGGTGAAACGCAGGTCGCCAGCGGCACCGCGCCTTTCGGCGGTGA
AATTATCGATGAGCGTGGTGGTTATGCCGATCGCGTCACACTACGTCTGAACGTCGAAAA
CCCGAAACTGTGGAGCGCCGAAATCCCGAATCTCTATCGTGCGGTGGTTGAACTGCACAC
CGCCGACGGCACGCTGATTGAAGCAGAAGCCTGCGATGTCGGTTTCCGCGAGGTGCGGAT
TGAAAATGGTCTGCTGCTGCTGAACGGCAAGCCGTTGCTGATTCGAGGCGTTAACCGTCA
CGAGCATCATCCTCTGCATGGTCAGGTCATGGATGAGCAGACGATGGTGCAGGATATCCT
GCTGATGAAGCAGAACAACTTTAACGCCGTGCGCTGTTCGCATTATCCGAACCATCCGCT
GTGGTACACGCTGTGCGACCGCTACGGCCTGTATGTGGTGGATGAAGCCAATATTGAAAC
CCACGGCATGGTGCCAATGAATCGTCTGACCGATGATCCGCGCTGGCTACCGGCGATGAG
CGAACGCGTAACGCGAATGGTGCAGCGCGATCGTAATCACCCGAGTGTGATCATCTGGTC
GCTGGGGAATGAATCAGGCCACGGCGCTAATCACGACGCGCTGTATCGCTGGATCAAATC
TGTCGATCCTTCCCGCCCGGTGCAGTATGAAGGCGGCGGAGCCGACACCACGGCCACCGA
TATTATTTGCCCGATGTACGCGCGCGTGGATGAAGACCAGCCCTTCCCGGCTGTGCCGAA
ATGGTCCATCAAAAAATGGCTTTCGCTACCTGGAGAGACGCGCCCGCTGATCCTTTGCGA
ATACGCCCACGCGATGGGTAACAGTCTTGGCGGTTTCGCTAAATACTGGCAGGCGTTTCG
TCAGTATCCCCGTTTACAGGGCGGCTTCGTCTGGGACTGGGTGGATCAGTCGCTGATTAA
ATATGATGAAAACGGCAACCCGTGGTCGGCTTACGGCGGTGATTTTGGCGATACGCCGAA
CGATCGCCAGTTCTGTATGAACGGTCTGGTCTTTGCCGACCGCACGCCGCATCCAGCGCT
GACGGAAGCAAAACACCAGCAGCAGTTTTTCCAGTTCCGTTTATCCGGGCAAACCATCGA
AGTGACCAGCGAATACCTGTTCCGTCATAGCGATAACGAGCTCCTGCACTGGATGGTGGC
GCTGGATGGTAAGCCGCTGGCAAGCGGTGAAGTGCCTCTGGATGTCGCTCCACAAGGTAA
ACAGTTGATTGAACTGCCTGAACTACCGCAGCCGGAGAGCGCCGGGCAACTCTGGCTCAC
AGTACGCGTAGTGCAACCGAACGCGACCGCATGGTCAGAAGCCGGGCACATCAGCGCCTG
GCAGCAGTGGCGTCTGGCGGAAAACCTCAGTGTGACGCTCCCCGCCGCGTCCCACGCCAT
CCCGCATCTGACCACCAGCGAAATGGATTTTTGCATCGAGCTGGGTAATAAGCGTTGGCA
ATTTAACCGCCAGTCAGGCTTTCTTTCACAGATGTGGATTGGCGATAAAAAACAACTGCT
GACGCCGCTGCGCGATCAGTTCACCCGTGCACCGCTGGATAACGACATTGGCGTAAGTGA
AGCGACCCGCATTGACCCTAACGCCTGGGTCGAACGCTGGAAGGCGGCGGGCCATTACCA
GGCCGAAGCAGCGTTGTTGCAGTGCACGGCAGATACACTTGCTGATGCGGTGCTGATTAC
GACCGCTCACGCGTGGCAGCATCAGGGGAAAACCTTATTTATCAGCCGGAAAACCTACCG
GATTGATGGTAGTGGTCAAATGGCGATTACCGTTGATGTTGAAGTGGCGAGCGATACACC
GCATCCGGCGCGGATTGGCCTGAACTGCCAGCTGGCGCAGGTAGCAGAGCGGGTAAACTG
GCTCGGATTAGGGCCGCAAGAAAACTATCCCGACCGCCTTACTGCCGCCTGTTTTGACCG
CTGGGATCTGCCATTGTCAGACATGTATACCCCGTACGTCTTCCCGAGCGAAAACGGTCT
GCGCTGCGGGACGCGCGAATTGAATTATGGCCCACACCAGTGGCGCGGCGACTTCCAGTT
CAACATCAGCCGCTACAGTCAACAGCAACTGATGGAAACCAGCCATCGCCATCTGCTGCA
CGCGGAAGAAGGCACATGGCTGAATATCGACGGTTTCCATATGGGGATTGGTGGCGACGA
CTCCTGGAGCCCGTCAGTATCGGCGGAATTCCAGCTGAGCGCCGGTCGCTACCATTACCA
GTTGGTCTGGTGTCAAAAATAATAATAACCGGGCAGGGGGGATCCGCAGATCCGGCTGTG

```
GAATGTGTGTCAGTTAGGGTGTGGAAAGTCCCCAGGCTCCCCAGCAGGCAGAAGTATGCA
AAGCATGCCTGCAGGAATTCGATATCAAGCTTATCGATACCGTCGAATTGGAAGAGCTTT
AAATCCTGGCACATCTCATGTATCAATGCCTCAGTATGTTTAGAAAAACAAGGGGGGAAC
TGTGGGGTTTTTATGAGGGGTTTTATAAAAATGAAAGACCCCACCTGTAGGTTTGGCAAG
CTAGCTTAAGTAACGCCATTTTGCAAGGCATGGAAAAATACATAACTGAGAATAGAGAAG
TTCAGATCAAGGTCAGGAACAGATGGAACAGCTGAATATGGGCCAAACAGGATATCTGTG
GTAAGCAGTTCCTGCCCCGGCTCAGGGCCAAGAACAGATGGAACAGCTGAATATGGGCCA
AACAGGATATCTGTGGTAAGCAGTTCCTGCCCCGGCTCAGGGCCAAGAACAGATGGTCCC
CAGATGCGGTCCAGCCCTCAGCAGTTTCTAGAGAACCATCAGATGTTTCCAGGGTGCCCC
AAGGACCTGAAATGACCCTGTGCCTTATTTGAACTAACCAATCAGTTCGCTTCTCGCTTC
TGTTCGCGCGCTTCTGCTCCCCGAGCTCAATAAAAGAGCCCACAACCCCTCACTCGGGGG
GCACTCAGATTCTGCGGTCTGAGTCCCTTCTCTGCTGGGCTGAAAAGGCCTTTGTAATAA
ATATAATTCTCTACTCAGTCCCTGTCTCTAGTTTGTCTGTTCGAGATCCTACAGAGCTCA
TGCCTTGGCGTAATCATGGTCATAGCTGTTTCCTGTGTGAAATTGTTATCCGCTCACAAT
TCCACACAACATACGAGCCGGAAGCATAAAGTGTAAAGCCTGGGGTGCCTAATGAGTGAG
CTAACTCACATTAATTGCGTTGCGCTCACTGCCCGCTTTCCAGTCGGGAAACCTGTCGTG
CCAGCTGCATTAATGAATCGGCCAACGCGCGGGGAGAGGCGGTTTGCGTATTGGGCGCTC
TTCCGCTTCCTCGCTCACTGACTCGCTGCGCTCGGTCGTTCGGCTGCGGCGAGCGGTATC
AGCTCACTCAAAGGCGGTAATACGGTTATCCACAGAATCAGGGGATAACGCAGGAAAGA
A
CATGTGAGCAAAAGGCCAGCAAAAGGCCAGGAACCGTAAAAAGGCCGCGTTGCTGGCGT
T
TTTCCATAGGCTCCGCCCCCCTGACGAGCATCACAAAAATCGACGCTCAAGTCAGAGGTG
GCGAAACCCGACAGGACTATAAAGATACCAGGCGTTTCCCCCTGGAAGCTCCCTCGTGCG
CTCTCCTGTTCCGACCCTGCCGCTTACCGGATACCTGTCCGCCTTTCTCCCTTCGGGAAG
CGTGGCGCTTTCTCATAGCTCACGCTGTAGGTATCTCAGTTCGGTGTAGGTCGTTCGCTC
CAAGCTGGGCTGTGTGCACGAACCCCCCGTTCAGCCCGACCGCTGCGCCTTATCCGGTAA
CTATCGTCTTGAGTCCAACCCGGTAAGACACGACTTATCGCCACTGGCAGCAGCCACTGG
TAACAGGATTAGCAGAGCGAGGTATGTAGGCGGTGCTACAGAGTTCTTGAAGTGGTGGCC
TAACTACGGCTACACTAGAAGGACAGTATTTGGTATCTGCGCTCTGCTGAAGCCAGTTAC
CTTCGGAAAAAGAGTTGGTAGCTCTTGATCCGGCAAACAAACCACCGCTGGTAGCGGTGG
TTTTTTTGTTTGCAAGCAGCAGATTACGCGCAGAAAAAAAGGATCTCAAGAAGATCCTTT
GATCTTTTCTACGGGGTCTGACGCTCAGTGGAACGAAAACTCACGTTAAGGGATTTTGGT
CATGAGATTATCAAAAAGGATCTTCACCTAGATCCTTTTAAATTAAAAATGAAGTTTTAA
ATCAATCTAAAGTATATATGAGTAAACTTGGTCTGACAGTTACCAATGCTTAATCAGTGA
GGCACCTATCTCAGCGATCTGTCTATTTCGTTCATCCATAGTTGCCTGACTCCCCGTCGT
GTAGATAACTACGATACGGGAGGGCTTACCATCTGGCCCCAGTGCTGCAATGATACCGCG
AGACCCACGCTCACCGGCTCCAGATTTATCAGCAATAAACCAGCCAGCCGGAAGGGCCGA
GCGCAGAAGTGGTCCTGCAACTTTATCCGCCTCCATCCAGTCTATTAATTGTTGCCGGGA
AGCTAGAGTAAGTAGTTCGCCAGTTAATAGTTTGCGCAACGTTGTTGCCATTGCTACAGG
CATCGTGGTGTCACGCTCGTCGTTTGGTATGGCTTCATTCAGCTCCGGTTCCCAACGATC
AAGGCGAGTTACATGATCCCCCATGTTGTGCAAAAAAGCGGTTAGCTCCTTCGGTCCTCC
GATCGTTGTCAGAAGTAAGTTGGCCGCAGTGTTATCACTCATGGTTATGGCAGCACTGCA
TAATTCTCTTACTGTCATGCCATCCGTAAGATGCTTTTCTGTGACTGGTGAGTACTCAAC
CAAGTCATTCTGAGAATAGTGTATGCGGCGACCGAGTTGCTCTTGCCCGGCGTCAATACG
GGATAATACCGCGCCACATAGCAGAACTTTAAAAGTGCTCATCATTGGAAAACGTTCTTC
GGGGCGAAAACTCTCAAGGATCTTACCGCTGTTGAGATCCAGTTCGATGTAACCCACTCG
TGCACCCAACTGATCTTCAGCATCTTTTACTTTCACCAGCGTTTCTGGGTGAGCAAAAAC
AGGAAGGCAAAATGCCGCAAAAAAGGGAATAAGGGCGACACGGAAATGTTGAATACTCA
TACTCTTCCTTTTTCAATATTATTGAAGCATTTATCAGGGTTATTGTCTCATGAGCGGATA
CATATTTGAATGTATTTAGAAAAATAAACAAATAGGGGTTCCGCGCACATTTCCCCGAAA
AGTGCCACCTAAATTGTAAGCGTTAATATTTTGTTAAAATTCGCGTTAAATTTTTGTTAA
ATCAGCTCATTTTTTAACCAATAGGCCGAAATCGGCAAAATCCCTTATAAATCAAAAGAA
TAGACCGAGATAGGGTTGAGTGTTGTTCCAGTTTGGAACAAGAGTCCACTATTAAAGAAC
GTGGACTCCAACGTCAAAGGGCGAAAAACCGTCTATCAGGGCGATGGCCCACTACGTGAA
CCATCACCCTAATCAAGTTTTTTGGGGTCGAGGTGCCGTAAAGCACTAAATCGGAACCCT
AAAGGGAGCCCCCGATTTAGAGCTTGACGGGGAAAGCCAACCTGGCTTATCGAAATTAAT
ACGACTCACTATAGGGAGACCGGC
```

Figure 5

EIAV U5 (34bp)

EIAV R region (78bp)

27bp of EIAV U3

446bp of MLV U3

Figure 6

TGTGGGGTTTTTATGAGGGGTTTTATAATGAAAGACCCCACCTGTAGGTTTGGCAAGCT
AGCTTAAGTAACGCCATTTTGCAAGGCATGGAAAAATACATAACTGAGAATAGAGAAGTT
CAGATCAAGGTCAGGAACAGATGGAACAGCTGAATATGGGCCAAACAGGATATCTGTGG
T
AAGCAGTTCCTGCCCCGGCTCAGGGCCAAGAACAGATGGAACAGCTGAATATGGGCCAA
A
CAGGATATCTGTGGTAAGCAGTTCCTGCCCCGGCTCAGGGCCAAGAACAGATGGTCCCCA
GATGCGGTCCAGCCCTCAGCAGTTTCTAGAGAACCATCAGATGTTTCCAGGGTGCCCCAA
GGACCTGAAATGACCCTGTGCCTTATTTGAACTAACCAATCAGTTCGCTTCTCGCTTCTG
TTCGCGCGCTTCTGCTCCCCGAGCTCAATAAAAGAGCCCACAACCCCTCACTCGGGGGGC
ACTCAGATTCTGCGGTCTGAGTCCCTTCTCTGCTGGGCTGAAAAGGCCTTTGTAATAAAT

44

## Figure 7

```
AGATCTTGAATAATAAAATGTGTGTTTGTCCGAAATACGCGTTTTGAGATTTCTGTCGCCG
ACTAAATTCATGTCGCGCGATAGTGGTGTTTATCGCCGATAGAGATGGCGATATTGGAAA
AATTGATATTTGAAAATATGGCATATTGAAAATGTCGCCGATGTGAGTTTCTGTGTAACTG
ATATCGCCATTTTTCCAAAAGTGATTTTTGGGCATACGCGATATCTGGCGATAGCGCTTA
TATCGTTTACGGGGGATGGCGATAGACGACTTTGGTGACTTGGGCGATTCTGTGTGTCG
CAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCATA
CGTTGTATCCATATCGTAATATGTACATTTATATTGGCTCATGTCCAACATTACCGCCATG
TTGACATTGATTATTGACTAGTTATTAATAGTAATCAATTACGGGGTCATTAGTTCATAGC
CCATATATGGAGTTCCGCGTTACATAACTTACGGTAAATGGCCCGCCTGGCTGACCGCC
CAACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAGTA
CATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTG
GATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTGCGATCGCCCG
CCCCGTTGACGCAAATGGGCGGTAGGCGTGTACGGTGGGAGGTCTATATAAGCAGAGC
TCGTTTAGTGAACCGGGCACTCAGATTCTGCGGTCTGAGTCCCTTCTCTGCTGGGCTGA
AAAGGCCTTTGTAATAAATATAATTCTCTACTCAGTCCCTGTCTCTAGTTTGTCTGTTCGA
GATCCTACAGTTGGCGCCCGAACAGGGACCTGAGAGGGGCGCAGACCCTACCTGTTGA
ACCTGGCTGATCGTAGGATCCCCGGGACAGCAGAGGAGAACTTACAGAAGTCTTCTGGA
GGTGTTCCTGGCCAGAACACAGGAGGACAGGTAAGATTGGGAGACCCTTTGACATTGGA
GCAAGGCGCTCAAGAAGTTAGAGAAGGTGACGGTACAAGGGTCTCAGAAATTAACTACT
GGTAACTGTAATTGGGCGCTAAGTCTAGTAGACTTATTTCATGATACCAACTTTGTAAAAG
AAAAGGACTGGCAGCTGAGGGATGTCATTCCATTGCTGGAAGATGTAACTCAGACGCTG
TCAGGACAAGAAAGAGAGGCCTTTGAAAGAACATGGTGGGCAATTTCTGCTGTAAAGAT
GGGCCTCCAGATTAATAATGTAGTAGATGGAAAGGCATCATTCCAGCTCCTAAGAGCGA
AATATGAAAAGAAGACTGCTAATAAAAAGCAGTCTGAGCCCTCTGAAGAATATCTCTAGA
ACTAGTGGATCCCCCGGGCTGCAGGAGTGGGGAGGCACGATGGCCGCTTTGGTCGAG
GCGGATCCGGCCATTAGCCATATTATTCATTGGTTATATAGCATAAATCAATATTGGCTAT
TGGCCATTGCATACGTTGTATCCATATCATAATATGTACATTTATATTGGCTCATGTCCAA
CATTACCGCCATGTTGACATTGATTATTGACTAGTTATTAATAGTAATCAATTACGGGGTC
ATTAGTTCATAGCCCATATATGGAGTTCCGCGTTACATAACTTACGGTAAATGGCCCGCC
TGGCTGACCGCCCAACGACCCCCGCCCATTGACGTCAATAATGACGTATGTTCCCATAG
TAACGCCAATAGGGACTTTCCATTGACGTCAATGGGTGGAGTATTTACGGTAAACTGCCC
ACTTGGCAGTACATCAAGTGTATCATATGCCAAGTACGCCCCCTATTGACGTCAATGACG
GTAAATGGCCCGCCTGGCATTATGCCCAGTACATGACCTTATGGGACTTTCCTACTTGG
CAGTACATCTACGTATTAGTCATCGCTATTACCATGGTGATGCGGTTTTGGCAGTACATC
AATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTC
CGCCCCATTGACGCAAATGGGCGGTAGGCATGTACGGTGGGAGGTCTATATAAGCAGA
GCTCGTTTAGTGAACCGTCAGATCGCCTGGAGACGCCATCCACGCTGTTTTGACCTCCA
TAGAAGACACCGGGACCGATCCAGCCTCCGCGGCCCCAAGCTTCAGCTGCTCGAGGAT
CTGCGGATCCGGGGAATTCCCCAGTCTCAGGATCCACCATGGGGGATCCCGTCGTTTTA
CAACGTCGTGACTGGGAAAACCCTGGCGTTACCCAACTTAATCGCCTTGCAGCACATCC
CCCTTTCGCCAGCTGGCGTAATAGCGAAGAGGCCCGCACCGATCGCCCTTCCCAACAG
TTGCGCAGCCTGAATGGCGAATGGCGCTTTGCCTGGTTTCCGGCACCAGAAGCGGTGC
CGGAAAGCTGGCTGGAGTGCGATCTTCCTGAGGCCGATACTGTCGTCGTCCCCTCAAAC
TGGCAGATGCACGGTTACGATGCGCCCATCTACACCAACGTAACCTATCCCATTACGGT
CAATCCGCCGTTTGTTCCCACGGAGAATCCGACGGGTTGTTACTCGCTCACATTTAATGT
TGATGAAAGCTGGCTACAGGAAGGCCAGACGCGAATTATTTTTGATGGCGTTAACTCGG
CGTTTCATCTGTGGTGCAACGGGCGCTGGGTCGGTTACGGCCAGGACAGTCGTTTGCC
GTCTGAATTTGACCTGAGCGCATTTTTACGCGCCGGAGAAAACCGCCTCGCGGTGATGG
TGCTGCGTTGGAGTGACGGCAGTTATCTGGAAGATCAGGATATGTGGCGGATGAGCGG
CATTTTCCGTGACGTCTCGTTGCTGCATAAACCGACTACACAAATCAGCGATTTCCATGT
```

TGCCACTCGCTTTAATGATGATTTCAGCCGCGCTGTACTGGAGGCTGAAGTTCAGATGT
GCGGCGAGTTGCGTGACTACCTACGGGTAACAGTTTCTTTATGGCAGGGTGAAACGCAG
GTCGCCAGCGGCACCGCGCCTTTCGGCGGTGAAATTATCGATGAGCGTGGTGGTTATG
CCGATCGCGTCACACTACGTCTGAACGTCGAAAACCCGAAACTGTGGAGCGCCGAAATC
CCGAATCTCTATCGTGCGGTGGTTGAACTGCACACCGCCGACGGCACGCTGATTGAAG
CAGAAGCCTGCGATGTCGGTTTCCGCGAGGTGCGGATTGAAAATGGTCTGCTGCTGCT
GAACGGCAAGCCGTTGCTGATTCGAGGCGTTAACCGTCACGAGCATCATCCTCTGCATG
GTCAGGTCATGGATGAGCAGACGATGGTGCAGGATATCCTGCTGATGAAGCAGAACAAC
TTTAACGCCGTGCGCTGTTCGCATTATCCGAACCATCCGCTGTGGTACACGCTGTGCGA
CCGCTACGGCCTGTATGTGGTGGATGAAGCCAATATTGAAACCCACGGCATGGTGCCAA
TGAATCGTCTGACCGATGATCCGCGCTGGCTACCGGCGATGAGCGAACGCGTAACGCG
AATGGTGCAGCGCGATCGTAATCACCCGAGTGTGATCATCTGGTCGCTGGGGAATGAAT
CAGGCCACGGCGCTAATCACGACGCGCTGTATCGCTGGATCAAATCTGTCGATCCTTCC
CGCCCGGTGCAGTATGAAGGCGGCGGAGCCGACACCACGGCCACCGATATTATTTGCC
CGATGTACGCGCGCGTGGATGAAGACCAGCCCTTCCCGGCTGTGCCGAAATGGTCCAT
CAAAAAATGGCTTTCGCTACCTGGAGAGACGCGCCCGCTGATCCTTTGCGAATACGCCC
ACGCGATGGGTAACAGTCTTGGCGGTTTCGCTAAATACTGGCAGGCGTTTCGTCAGTAT
CCCCGTTTACAGGGCGGCTTCGTCTGGGACTGGGTGGATCAGTCGCTGATTAAATATGA
TGAAAACGGCAACCCGTGGTCGGCTTACGGCGGTGATTTTGGCGATACGCCGAACGAT
CGCCAGTTCTGTATGAACGGTCTGGTCTTTGCCGACCGCACGCCGCATCCAGCGCTGA
CGGAAGCAAAACACCAGCAGCAGTTTTTCCAGTTCCGTTTATCCGGGCAAACCATCGAA
GTGACCAGCGAATACCTGTTCCGTCATAGCGATAACGAGCTCCTGCACTGGATGGTGGC
GCTGGATGGTAAGCCGCTGGCAAGCGGTGAAGTGCCTCTGGATGTCGCTCCACAAGGT
AAACAGTTGATTGAACTGCCTGAACTACCGCAGCCGGAGAGCGCCGGGCAACTCTGGC
TCACAGTACGCGTAGTGCAACCGAACGCGACCGCATGGTCAGAAGCCGGGCACATCAG
CGCCTGGCAGCAGTGGCGTCTGGCGGAAAACCTCAGTGTGACGCTCCCCGCCGCGTCC
CACGCCATCCCGCATCTGACCACCAGCGAAATGGATTTTTGCATCGAGCTGGGTAATAA
GCGTTGGCAATTTAACCGCCAGTCAGGCTTTCTTTCACAGATGTGGATTGGCGATAAAAA
ACAACTGCTGACGCCGCTGCGCGATCAGTTCACCCGTGCACCGCTGGATAACGACATTG
GCGTAAGTGAAGCGACCCGCATTGACCCTAACGCCTGGGTCGAACGCTGGAAGGCGGC
GGGCCATTACCAGGCCGAAGCAGCGTTGTTGCAGTGCACGGCAGATACACTTGCTGAT
GCGGTGCTGATTACGACCGCTCACGCGTGGCAGCATCAGGGGAAAACCTTATTTATCAG
CCGGAAAACCTACCGGATTGATGGTAGTGGTCAAATGGCGATTACCGTTGATGTTGAAG
TGGCGAGCGATACACCGCATCCGGCGCGGATTGGCCTGAACTGCCAGCTGGCGCAGGT
AGCAGAGCGGGTAAACTGGCTCGGATTAGGGCCGCAAGAAAACTATCCCGACCGCCTT
ACTGCCGCCTGTTTTGACCGCTGGGATCTGCCATTGTCAGACATGTATACCCCGTACGT
CTTCCCGAGCGAAAACGGTCTGCGCTGCGGGACGCGCGAATTGAATTATGGCCCACAC
CAGTGGCGCGGCGACTTCCAGTTCAACATCAGCCGCTACAGTCAACAGCAACTGATGGA
AACCAGCCATCGCCATCTGCTGCACGCGGAAGAAGGCACATGGCTGAATATCGACGGTT
TCCATATGGGGGATTGGTGGCGACGACTCCTGGAGCCCGTCAGTATCGGCGGAATTCCA
GCTGAGCGCCGGTCGCTACCATTACCAGTTGGTCTGGTGTCAAAAATAATAATAACCGG
GCAGGGGGGATCCGCAGATCCGGCTGTGGAATGTGTGTCAGTTAGGGTGTGGAAAGTC
CCCAGGCTCCCCAGCAGGCAGAAGTATGCAAAGCATGCCTGCAGGAATTCGATATCAAG
CTTATCGATACCGTCGAATTGGAAGAGCTTTAAATCCTGGCACATCTCATGTATCAATGC
CTCAGTATGTTTAGAAAAACAAGGGGGGAACTGTGGGGTTTTTATGAGGGGTTTTATAAT
GAAAGACCCCACCTGTAGGTTTGGCAAGCTAGCTTAAGTAACGCCATTTTGCAAGGCAT
GGAAAAATACATAACTGAGAATAGAGAAGTTCAGATCAAGGTCAGGAACAGATGGAACA
GCTGAATATGGGCCAAACAGGATATCTGTGGTAAGCAGTTCCTGCCCCGGCTCAGGGC
CAAGAACAGATGGAACAGCTGAATATGGGCCAAACAGGATATCTGTGGTAAGCAGTTCC
TGCCCCGGCTCAGGGCCAAGAACAGATGGTCCCCAGATGCGGTCCAGCCCTCAGCAGT
TTCTAGAGAACCATCAGATGTTTCCAGGGTGCCCCAAGGACCTGAAATGACCCTGTGCC
TTATTTGAACTAACCAATCAGTTCGCTTCTCGCTTCTGTTCGCGCGCTTCTGCTCCCCGA
GCTCAATAAAAGAGCCCACAACCCCTCACTCGGGGGGCACTCAGATTCTGCGGTCTGAG
TCCCTTCTCTGCTGGGCTGAAAAGGCCTTTGTAATAAATATAATTCTCTACTCAGTCCCTG
TCTCTAGTTTGTCTGTTCGAGATCCTACAGAGCTCATGCCTTGGCGTAATCATGGTCATA
GCTGTTTCCTGTGTGAAATTGTTATCCGCTCACAATTCCACACAACATACGAGCCGGAAG
CATAAAGTGTAAAGCCTGGGGTGCCTAATGAGTGAGCTAACTCACATTAATTGCGTTGCG
CTCACTGCCCGCTTTCCAGTCGGGAAACCTGTCGTGCCAGCTGCATTAATGAATCGGCC

```
AACGCGCGGGGAGAGGCGGTTTGCGTATTGGGCGCTCTTCCGCTTCCTCGCTCACTGA
CTCGCTGCGCTCGGTCGTTCGGCTGCGGCGAGCGGTATCAGCTCACTCAAAGGCGGTA
ATACGGTTATCCACAGAATCAGGGGATAACGCAGGAAAGAACATGTGAGCAAAAGGCCA
GCAAAAGGCCAGGAACCGTAAAAAGGCCGCGTTGCTGGCGTTTTTCCATAGGCTCCGC
CCCCCTGACGAGCATCACAAAAATCGACGCTCAAGTCAGAGGTGGCGAAACCCGACAG
GACTATAAAGATACCAGGCGTTTCCCCCTGGAAGCTCCCTCGTGCGCTCTCCTGTTCCG
ACCCTGCCGCTTACCGGATACCTGTCCGCCTTTCTCCCTTCGGGAAGCGTGGCGCTTTC
TCATAGCTCACGCTGTAGGTATCTCAGTTCGGTGTAGGTCGTTCGCTCCAAGCTGGGCT
GTGTGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAACAGGAT
TAGCAGAGCGAGGTATGTAGGCGGTGCTACAGAGTTCTTGAAGTGGTGGCCTAACTACG
GCTACACTAGAAGGACAGTATTTGGTATCTGCGCTCTGCTGAAGCCAGTTACCTTCGGA
AAAAGAGTTGGTAGCTCTTGATCCGGCAAACAAACCACCGCTGGTAGCGGTGGTTTTTTT
GTTTGCAAGCAGCAGATTACGCGCAGAAAAAAAGGATCTCAAGAAGATCCTTTGATCTTT
TCTACGGGGTCTGACGCTCAGTGGAACGAAAACTCACGTTAAGGGATTTTGGTCATGAG
ATTATCAAAAAGGATCTTCACCTAGATCCTTTTAAATTAAAAATGAAGTTTTAAATCAATCT
AAAGTATATATGAGTAAACTTGGTCTGACAGTTACCAATGCTTAATCAGTGAGGCACCTA
TCTCAGCGATCTGTCTATTTCGTTCATCCATAGTTGCCTGACTCCCCGTCGTGTAGATAA
CTACGATACGGGAGGGCTTACCATCTGGCCCCAGTGCTGCAATGATACCGCGAGACCC
ACGCTCACCGGCTCCAGATTTATCAGCAATAAACCAGCCAGCCGGAAGGGCCGAGCGC
AGAAGTGGTCCTGCAACTTTATCCGCCTCCATCCAGTCTATTAATTGTTGCCGGGAAGCT
AGAGTAAGTAGTTCGCCAGTTAATAGTTTGCGCAACGTTGTTGCCATTGCTACAGGCATC
GTGGTGTCACGCTCGTCGTTTGGTATGGCTTCATTCAGCTCCGGTTCCCAACGATCAAG
GCGAGTTACATGATCCCCCATGTTGTGCAAAAAAGCGGTTAGCTCCTTCGGTCCTCCGA
TCGTTGTCAGAAGTAAGTTGGCCGCAGTGTTATCACTCATGGTTATGGCAGCACTGCATA
ATTCTCTTACTGTCATGCCATCCGTAAGATGCTTTTCTGTGACTGGTGAGTACTCAACCA
AGTCATTCTGAGAATAGTGTATGCGGCGACCGAGTTGCTCTTGCCCGGCGTCAATACGG
GATAATACCGCGCCACATAGCAGAACTTTAAAAGTGCTCATCATTGGAAAACGTTCTTCG
GGGCGAAAACTCTCAAGGATCTTACCGCTGTTGAGATCCAGTTCGATGTAACCCACTCG
TGCACCCAACTGATCTTCAGCATCTTTTACTTTCACCAGCGTTTCTGGGTGAGCAAAAC
AGGAAGGCAAAATGCCGCAAAAAAGGGAATAAGGGCGACACGGAAATGTTGAATACTCA
TACTCTTCCTTTTTCAATATTATTGAAGCATTTATCAGGGTTATTGTCTCATGAGCGGATA
CATATTTGAATGTATTTAGAAAAATAAACAAATAGGGGTTCCGCGCACATTTCCCCGAAAA
GTGCCACCTAAATTGTAAGCGTTAATATTTTGTTAAAATTCGCGTTAAATTTTTGTTAAATC
AGCTCATTTTTTAACCAATAGGCCGAAATCGGCAAAATCCCTTATAAATCAAAAGAATAGA
CCGAGATAGGGTTGAGTGTTGTTCCAGTTTGGAACAAGAGTCCACTATTAAAGAACGTG
GACTCCAACGTCAAAGGGCGAAAAACCGTCTATCAGGGCGATGGCCCACTACGTGAAC
CATCACCCTAATCAAGTTTTTTGGGGTCGAGGTGCCGTAAAGCACTAAATCGGAACCCTA
AAGGGAGCCCCCGATTTAGAGCTTGACGGGGAAAGCCAACCTGGCTTATCGAAATTAAT
ACGACTCACTATAGGGAGACCGGC
```

Figure 8

Figure 9

Figure 10

## pONY8.4TCOG

```
AGATCTTGAATAATAAAATGTGTGTTTGTCCGAAATACGCGTTTTGAGATTTCTGTCGCC
GACTAAATTCATGTCGCGCGATAGTGGTGTTTATCGCCGATAGAGATGGCGATATTGGAA
AAATTGATATTTGAAAATATGGCATATTGAAAATGTCGCCGATGTGAGTTTCTGTGTAAC
TGATATCGCCATTTTTCCAAAAGTGATTTTTGGGCATACGCGATATCTGGCGATAGCGCT
TATATCGTTTACGGGGGATGGCGATAGACGACTTTGGTGACTTGGGCGATTCTGTGTGTC
GCAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCA
TACGTTGTATCCATATCGTAATATGTACATTTATATTGGCTCATGTCCAACATTACCGCC
ATGTTGACATTGATTATTGACTAGTTATTAATAGTAATCAATTACGGGGTCATTAGTTCA
TAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAGT
ACATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTGG
ATAGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTGCGATCGCCCGCC
CCGTTGACGCAAATGGGCGGTAGGCGTGTACGGTGGGAGGTCTATATAAGCAGAGCTCGT
TTAGTGAACCGGGCACTCAGATTCTGCGGTCTGAGTCCCTTCTCTGCTGGGCTGAAAAGG
CCTTTGTAATAAATATAATTCTCTACTCAGTCCCTGTCTCTAGTTTGTCTGTTCGAGATC
CTACAGTTGGCGCCCGAACAGGGACCTGAGAGGGGCGCAGACCCTACCTGTTGAACCTGG
CTGATCGTAGGATCCCCGGGACAGCAGAGGAGAACTTACAGAAGTCTTCTGGAGGTGTTC
CTGGCCAGAACACAGGAGGACAGGTAAGATTGGGAGACCCTTTGACATTGGAGCAAGGCG
CTCAAGAAGTTAGAGAAGGTGACGGTACAAGGGTCTCAGAAATTAACTACTGGTAACTGT
AATTGGGCGCTAAGTCTAGTAGACTTATTTCATTGATACCAACTTTGTAAAAGAAAAGGA
CTGGCAGCTGAGGGATTGTCATTCCATTGCTGGAAGATTGTAACTCAGACGCTGTCAGGA
CAAGAAAGAGAGGCCTTTGAAAGAACATTGGTGGGCAATTTCTGCTGTAAAGATTGGGCC
TCCAGATTAATAATTGTAGTAGATTGGAAAGGCATCATTCCAGCTCCTAAGAGCGAAATA
TTGAAAAGAAGACTGCTAATAAAAAGCAGTCTGAGCCCTCTGAAGAATATCTCTAGCGTC
GACCAATTGATGTCTAGATTAGATAAAAGTAAAGTGATTAACAGCGCATTAGAGCTGCTT
AATGAGGTCGGAATCGAAGGTTTAACAACCCGTAAACTCGCCCAGAAGCTAGGTGTAGAG
CAGCCTACATTGTATTGGCATGTAAAAAATAAGCGGGCTTTGCTCGACGCCTTAGCCATT
GAGATGTTAGATAGGCACCATACTCACTTTTGCCCTTTAGAAGGGGAAAGCTGGCAAGAT
TTTTTACGTAATAACGCTAAAAGTTTTAGATGTGCTTTACTAAGTCATCGCGATGGAGCA
AAAGTACATTTAGGTACACGGCCTACAGAAAAACAGTATGAAACTCTCGAAAATCAATTA
GCCTTTTTATGCCAACAAGGTTTTTCACTAGAGAATGCATTATATGCACTCAGCGCTGTG
GGGCATTTTACTTTAGGTTGCGTATTGGAAGATCAAGAGCATCAAGTCGCTAAAGAAGAA
AGGGAAACACCTACTACTGATAGTATGCCGCCATTATTACGACAAGCTATCGAATTATTT
GATCACCAAGGTGCAGAGCCAGCCTTCTTATTCGGCCTTGAATTGATCATATGCGGATTA
GAAAAACAACTTAAATGTGAAAGTGGGTCCGCGTACAGCGGATCCCGGGAATTCAGATCT
TATTAAGGTACCTAACGGACCGCGGTTAACCAGCTGAGCACTGGCCGGCCTAGGTGGCCG
GTTCGAATTAGGTACCGATGTACGGGCCAGATATACGCGTTGACATTGATTATTGACTAG
TTATTAATAGTAATCAATTACGGGGTCATTAGTTCATAGCCCATATATGGAGTTCCGCGT
TACATAACTTACGGTAAATGGCCCGCCTGGCTGACCGCCCAACGACCCCCGCCCATTGAC
GTCAATAATGACGTATGTTCCCATAGTAACGCCAATAGGGACTTTCCATTGACGTCAATG
GGTGGAGTATTTACGGTAAACTGCCCACTTGGCAGTACATCAAGTGTATCATATGCCAAG
TACGCCCCCTATTGACGTCAATGACGGTAAATGGCCCGCCTGGCATTATGCCCAGTACAT
GACCTTATGGGACTTTCCTACTTGGCAGTACATCTACGTATTAGTCATCGCTATTACCAT
GGTGATGCGGTTTTGGCAGTACATCAATGGGCGTGGATAGCGGTTTGACTCACGGGGATT
TCCAAGTCTCCACCCCATTGACGTCAATGGGAGTTTGTTTTGGAACCAAAATCAACGGGA
CTTTCCAAAATGTCGTAACAACTCCGCCCCATTGACGCAAATGGGCGGTAGGCGTGTACG
GTGGGAGGTCTATATAAGCAGAGCTCTCCCTATCAGTGATAGAGATCTCCCTATCAGTGA
TAGAGATCGTCGACGAGCTCGTTTAGTGAACCGTCAGATCGCCTGGAGACGCCATCCACG
CTGTTTTGACCTCCATAGAAGACACCGGGACCGATCCAGCCTCCGGACTCTAGCGTTTAA
```

ACTTAAGCTTGTTGGGATCCACCGGTCGCCACCATGGTGAGCAAGGGCGAGGAGCTGTTC
ACCGGGGTGGTGCCCATCCTGGTCGAGCTGGACGGCGACGTAAACGGCCACAAGTTCAGC
GTGTCCGGCGAGGGCGAGGGCGATGCCACCTACGGCAAGCTGACCCTGAAGTTCATCTGC
ACCACCGGCAAGCTGCCCGTGCCCTGGCCCACCCTCGTGACCACCCTGACCTACGGCGTG
CAGTGCTTCAGCCGCTACCCCGACCACATGAAGCAGCACGACTTCTTCAAGTCCGCCATG
CCCGAAGGCTACGTCCAGGAGCGCACCATCTTCTTCAAGGACGACGGCAACTACAAGACC
CGCGCCGAGGTGAAGTTCGAGGGCGACACCCTGGTGAACCGCATCGAGCTGAAGGGCATC
GACTTCAAGGAGGACGGCAACATCCTGGGGCACAAGCTGGAGTACAACTACAACAGCCAC
AACGTCTATATCATGGCCGACAAGCAGAAGAACGGCATCAAGGTGAACTTCAAGATCCGC
CACAACATCGAGGACGGCAGCGTGCAGCTCGCCGACCACTACCAGCAGAACACCCCCATC
GGCGACGGCCCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACCGCCGCCGGG
ATCACTCTCGGCATGGACGAGCTGTACAAGTAAAGCGGCCGCGACTCTAGCCTGCAGGAA
TTCGATATCAAGCTTATCGATACCGTCGAATTGGAAGAGCTTTAAATCCTGGCACATCTC
ATGTATCAATGCCTCAGTATGTTTAGAAAAACAAGGGGGGAACTGTGGGGTTTTTATGAG
GGGTTTTATAAAAATGAAAGACCCCACCTGTAGGTTTGGCAAGCTAGCTTAAGTAACGCC
ATTTTGCAAGGCATGGAAAAATACATAACTGAGAATAGAGAAGTTCAGATCAAGGTCAGG
AACAGATGGAACAGCTGAATATGGGCCAAACAGGATATCTGTGGTAAGCAGTTCCTGCCC
CGGCTCAGGGCCAAGAACAGATGGAACAGCTGAATATGGGCCAAACAGGATATCTGTGGT
AAGCAGTTCCTGCCCCGGCTCAGGGCCAAGAACAGATGGTCCCCAGATGCGGTCCAGCCC
TCAGCAGTTTCTAGAGAACCATCAGATGTTTCCAGGGTGCCCCAAGGACCTGAAATGACC
CTGTGCCTTATTTGAACTAACCAATCAGTTCGCTTCTCGCTTCTGTTCGCGCGCTTCTGC
TCCCCGAGCTCAATAAAAGAGCCCACAACCCCTCACTCGGGGGGCACTCAGATTCTGCGG
TCTGAGTCCCTTCTCTGCTGGGCTGAAAAGGCCTTTGTAATAAATATAATTCTCTACTCA
GTCCCTGTCTCTAGTTTGTCTGTTCGAGATCCTACAGAGCTCATGCCTTGGCGTAATCAT
GGTCATAGCTGTTTCCTGTGTGAAATTGTTATCCGCTCACAATTCCACACAACATACGAG
CCGGAAGCATAAAGTGTAAAGCCTGGGGTGCCTAATGAGTGAGCTAACTCACATTAATTG
CGTTGCGCTCACTGCCCGCTTTCCAGTCGGGAAACCTGTCGTGCCAGCTGCATTAATGAA
TCGGCCAACGCGCGGGGAGAGGCGGTTTGCGTATTGGGCGCTCTTCCGCTTCCTCGCTCA
CTGACTCGCTGCGCTCGGTCGTTCGGCTGCGGCGAGCGGTATCAGCTCACTCAAAGGCGG
TAATACGGTTATCCACAGAATCAGGGGATAACGCAGGAAAGAACATGTGAGCAAAAGGCC
AGCAAAAGGCCAGGAACCGTAAAAAGGCCGCGTTGCTGGCGTTTTTCCATAGGCTCCGCC
CCCCTGACGAGCATCACAAAAATCGACGCTCAAGTCAGAGGTGGCGAAACCCGACAGGAC
TATAAAGATACCAGGCGTTTCCCCCTGGAAGCTCCCTCGTGCGCTCTCCTGTTCCGACCC
TGCCGCTTACCGGATACCTGTCCGCCTTTCTCCCTTCGGGAAGCGTGGCGCTTTCTCATA
GCTCACGCTGTAGGTATCTCAGTTCGGTGTAGGTCGTTCGCTCCAAGCTGGGCTGTGTGC
ACGAACCCCCCGTTCAGCCCGACCGCTGCGCCTTATCCGGTAACTATCGTCTTGAGTCCA
ACCCGGTAAGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGGCTACACTA
GAAGGACAGTATTTGGTATCTGCGCTCTGCTGAAGCCAGTTACCTTCGGAAAAAGAGTTG
GTAGCTCTTGATCCGGCAAACAAACCACCGCTGGTAGCGGTGGTTTTTTTGTTTGCAAGC
AGCAGATTACGCGCAGAAAAAAGGATCTCAAGAAGATCCTTTGATCTTTTCTACGGGGT
CTGACGCTCAGTGGAACGAAAACTCACGTTAAGGGATTTTGGTCATGAGATTATCAAAAA
GGATCTTCACCTAGATCCTTTTAAATTAAAAATGAAGTTTTAAATCAATCTAAAGTATAT
ATGAGTAAACTTGGTCTGACAGTTACCAATGCTTAATCAGTGAGGCACCTATCTCAGCGA
TCTGTCTATTTCGTTCATCCATAGTTGCCTGACTCCCCGTCGTGTAGATAACTACGATAC
GGGAGGGCTTACCATCTGGCCCCAGTGCTGCAATGATACCGCGAGACCCACGCTCACCGG
CTCCAGATTTATCAGCAATAAACCAGCCAGCCGGAAGGGCCGAGCGCAGAAGTGGTCCTG
CAACTTTATCCGCCTCCATCCAGTCTATTAATTGTTGCCGGGAAGCTAGAGTAAGTAGTT
CGCCAGTTAATAGTTTGCGCAACGTTGTTGCCATTGCTACAGGCATCGTGGTGTCACGCT
CGTCGTTTGGTATGGCTTCATTCAGCTCCGGTTCCCAACGATCAAGGCGAGTTACATGAT
CCCCCATGTTGTGCAAAAAAGCGGTTAGCTCCTTCGGTCCTCCGATCGTTGTCAGAAGTA
AGTTGGCCGCAGTGTTATCACTCATGGTTATGGCAGCACTGCATAATTCTCTTACTGTCA
TGCCATCCGTAAGATGCTTTTCTGTGACTGGTGAGTACTCAACCAAGTCATTCTGAGAAT
AGTGTATGCGGCGACCGAGTTGCTCTTGCCCGGCGTCAATACGGGATAATACCGCGCCAC
ATAGCAGAACTTTAAAAGTGCTCATCATTGGAAAACGTTCTTCGGGGCGAAAACTCTCAA
GGATCTTACCGCTGTTGAGATCCAGTTCGATGTAACCCACTCGTGCACCCAACTGATCTT

CAGCATCTTTTACTTTCACCAGCGTTTCTGGGTGAGCAAAAACAGGAAGGCAAAATGCCG
CAAAAAAGGGAATAAGGGCGACACGGAAATGTTGAATACTCATACTCTTCCTTTTTCAAT
ATTATTGAAGCATTTATCAGGGTTATTGTCTCATGAGCGGATACATATTTGAATGTATTT
AGAAAAATAAACAAATAGGGGTTCCGCGCACATTTCCCCGAAAAGTGCCACCTAAATTGT
AAGCGTTAATATTTTGTTAAAATTCGCGTTAAATTTTTGTTAAATCAGCTCATTTTTTAA
CCAATAGGCCGAAATCGGCAAAATCCCTTATAAATCAAAAGAATAGACCGAGATAGGGTT
GAGTGTTGTTCCAGTTTGGAACAAGAGTCCACTATTAAAGAACGTGGACTCCAACGTCAA
AGGGCGAAAAACCGTCTATCAGGGCGATGGCCCACTACGTGAACCATCACCCTAATCAAG
TTTTTTGGGGTCGAGGTGCCGTAAAGCACTAAATCGGAACCCTAAAGGGAGCCCCCGATT
TAGAGCTTGACGGGGAAAGCCAACCTGGCTTATCGAAATTAATACGACTCACTATAGGGA
GACCGGC

Figure 11

## pONY8.4TsynCOG/ pONY8.4TsynCOG1

AGATCTTGAATAATAAAATGTGTGTTTGTCCGAAATACGCGTTTTGAGATTTCTGTCGCC
GACTAAATTCATGTCGCGCGATAGTGGTGTTTATCGCCGATAGAGATGGCGATATTGGAA
AAATTGATATTTGAAAATATGGCATATTGAAAATGTCGCCGATGTGAGTTTCTGTGTAAC
TGATATCGCCATTTTTCCAAAAGTGATTTTTGGGCATACGCGATATCTGGCGATAGCGCT
TATATCGTTTACGGGGGATGGCGATAGACGACTTTGGTGACTTGGGCGATTCTGTGTGTC
GCAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCA
TACGTTGTATCCATATCGTAATATGTACATTTATATTGGCTCATGTCCAACATTACCGCC
ATGTTGACATTGATTATTGACTAGTTATTAATAGTAATCAATTACGGGGTCATTAGTTCA
TAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAGT
ACATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTGG
ATAGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTGCGATCGCCCGCC
CCGTTGACGCAAATGGGCGGTAGGCGTGTACGGTGGGAGGTCTATATAAGCAGAGCTCGT
TTAGTGAACCGGGCACTCAGATTCTGCGGTCTGAGTCCCTTCTCTGCTGGGCTGAAAAGG
CCTTTGTAATAAATATAATTCTCTACTCAGTCCCTGTCTCTAGTTTGTCTGTTCGAGATC
CTACAGTTGGCGCCCGAACAGGGACCTGAGAGGGGCGCAGACCCTACCTGTTGAACCTGG
CTGATCGTAGGATCCCCGGGACAGCAGAGGAGAACTTACAGAAGTCTTCTGGAGGTGTTC
CTGGCCAGAACACAGGAGGACAGGTAAGATTGGGAGACCCTTTGACATTGGAGCAAGGCG
CTCAAGAAGTTAGAGAAGGTGACGGTACAAGGGTCTCAGAAATTAACTACTGGTAACTGT
AATTGGGCGCTAAGTCTAGTAGACTTATTTCATTGATACCAACTTTGTAAAAGAAAAGGA
CTGGCAGCTGAGGGATTGTCATTCCATTGCTGGAAGATTGTAACTCAGACGCTGTCAGGA
CAAGAAAGAGAGGCCTTTGAAAGAACATTGGTGGGCAATTTCTGCTGTAAAGATTGGGCC
TCCAGATTAATAATTGTAGTAGATTGGAAAGGCATCATTCCAGCTCCTAAGAGCGAAATA
TTGAAAAGAAGACTGCTAATAAAAGCAGTCTGAGCCCTCTGAAGAATATCTCTAGCGTC
GACCAATTGCCGCCACCATGAGCCGCCTGGACAAGAGCAAAGTGATCAACTCCGCCCTGG
AGCTGCTGAATGAGGTCGGCATCGAGGGACTGACCACGCGCAAGCTGGCCCAAAAGCTGG
GCGTCGAGCAGCCGACCCTGTATTGGCATGTGAAGAACAAGAGGGCCCTCCTGGACGCGC
TCGCCATCGAAATGCTGGATCGGCACCACACCCACTTCTGTCCCCTCGAAGGCGAGAGCT
GGCAGGACTTTCTGAGAAACAACGCCAAGTCCTTCCGCTGCGCCCTCCTGAGCCATCGCG
ATGGGGCCAAGGTGCACCTGGGGACGCGGCCCACTGAGAAACAGTACGAAACCCTGGAGA
ATCAGCTGGCGTTCCTCTGCCAGCAGGGGTTCTCCCTGGAGAACGCCCTCTACGCACTCT
CCGCCGTGGGCCACTTTACACTCGGTTGCGTGCTGGAGGACCAGGAGCACCAAGTCGCTA
AGGAGGAGCGGGAGACCCCCACCACCGACTCCATGCCCCCACTGCTGAGGCAGGCGATTG
AGCTGTTCGACCACCAGGGAGCAGAGCCTGCGTTCCTCTTCGGGCTGGAACTCATCATCT
GCGGCCTGGAGAAGCAGCTGAAGTGCGAGAGCGGCTCCGCCTACAGCGGCAGCAGGGAGT
TCCGCTCTTACTAACGGACCGCGGTTAACCAGCTGAGCACTGGCCGGCCTAGGTGGCCGG
TTCGAATTAGGTACCGATGTACGGGCCAGATACGCGTTGACATTGATTATTGACTAGT
TATTAATAGTAATCAATTACGGGGTCATTAGTTCATAGCCCATATATGGAGTTCCGCGTT
ACATAACTTACGGTAAATGGCCCGCCTGGCTGACCGCCCAACGACCCCCGCCCATTGACG
TCAATAATGACGTATGTTCCCATAGTAACGCCAATAGGGACTTTCCATTGACGTCAATGG
GTGGAGTATTTACGGTAAACTGCCCACTTGGCAGTACATCAAGTGTATCATATGCCAAGT
ACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATG
GTGATGCGGTTTTGGCAGTACATCAATGGGCGTGGATAGCGGTTTGACTCACGGGGATTT
CCAAGTCTCCACCCCATTGACGTCAATGGGAGTTTGTTTTGGAACCAAAATCAACGGGAC
TTTCCAAAATGTCGTAACAACTCCGCCCCATTGACGCAAATGGGCGGTAGGCGTGTACGG
TGGGAGGTCTATATAAGCAGAGCTCTCCCTATCAGTGATAGAGATCTCCCTATCAGTGAT

AGAGATCGTCGACGAGCTCGTTTAGTGAACCGTCAGATCGCCTGGAGACGCCATCCACGC
TGTTTTGACCTCCATAGAAGACACCGGGACCGATCCAGCCTCCGGACTCTAGCGTTTAAA
CTTAAGCTTGTTGGGATCCACCGGTCGCCACCATGGTGAGCAAGGGCGAGGAGCTGTTCA
CCGGGGTGGTGCCCATCCTGGTCGAGCTGGACGGCGACGTAAACGGCCACAAGTTCAGCG
TGTCCGGCGAGGGCGAGGGCGATGCCACCTACGGCAAGCTGACCCTGAAGTTCATCTGCA
CCACCGGCAAGCTGCCCGTGCCCTGGCCCACCCTCGTGACCACCCTGACCTACGGCGTGC
AGTGCTTCAGCCGCTACCCCGACCACATGAAGCAGCACGACTTCTTCAAGTCCGCCATGC
CCGAAGGCTACGTCCAGGAGCGCACCATCTTCTTCAAGGACGACGGCAACTACAAGACCC
GCGCCGAGGTGAAGTTCGAGGGCGACACCCTGGTGAACCGCATCGAGCTGAAGGGCATCG
ACTTCAAGGAGGACGGCAACATCCTGGGGCACAAGCTGGAGTACAACTACAACAGCCACA
ACGTCTATATCATGGCCGACAAGCAGAAGAACGGCATCAAGGTGAACTTCAAGATCCGCC
ACAACATCGAGGACGGCAGCGTGCAGCTCGCCGACCACTACCAGCAGAACACCCCCATCG
GCGACGGCCCCGTGCTGCTGCCCGACAACCACTACCTGAGCACCCAGTCCGCCCTGAGCA
AAGACCCCAACGAGAAGCGCGATCACATGGTCCTGCTGGAGTTCGTGACCGCCGCCGGGA
TCACTCTCGGCATGGACGAGCTGTACAAGTAAAGCGGCCGCGACTCTAGCCTGCAGGAAT
TCGATATCAAGCTTATCGATACCGTCGAATTGGAAGAGCTTTAAATCCTGGCACATCTCA
TGTATCAATGCCTCAGTATGTTTAGAAAAACAAGGGGGGAACTGTGGGGTTTTTATGAGG
GGTTTTATAAAAATGAAAGACCCCACCTGTAGGTTTGGCAAGCTAGCTTAAGTAACGCCA
TTTTGCAAGGCATGGAAAAATACATAACTGAGAATAGAGAAGTTCAGATCAAGGTCAGGA
ACAGATGGAACAGCTGAATATGGGCCAAACAGGATATCTGTGGTAAGCAGTTCCTGCCCC
GGCTCAGGGCCAAGAACAGATGGAACAGCTGAATATGGGCCAAACAGGATATCTGTGGTA
AGCAGTTCCTGCCCCGGCTCAGGGCCAAGAACAGATGGTCCCCAGATGCGGTCCAGCCCT
CAGCAGTTTCTAGAGAACCATCAGATGTTTCCAGGGTGCCCCAAGGACCTGAAATGACCC
TGTGCCTTATTTGAACTAACCAATCAGTTCGCTTCTCGCTTCTGTTCGCGCGCTTCTGCT
CCCCGAGCTCAATAAAAGAGCCCACAACCCCTCACTCGGGGGGCACTCAGATTCTGCGGT
CTGAGTCCCTTCTCTGCTGGGCTGAAAAGGCCTTTGTAATAAATATAATTCTCTACTCAG
TCCCTGTCTCTAGTTTGTCTGTTCGAGATCCTACAGAGCTCATGCCTTGGCGTAATCATG
GTCATAGCTGTTTCCTGTGTGAAATTGTTATCCGCTCACAATTCCACACAACATACGAGC
CGGAAGCATAAAGTGTAAAGCCTGGGGTGCCTAATGAGTGAGCTAACTCACATTAATTGC
GTTGCGCTCACTGCCCGCTTTCCAGTCGGGAAACCTGTCGTGCCAGCTGCATTAATGAAT
CGGCCAACGCGCGGGGAGAGGCGGTTTGCGTATTGGGCGCTCTTCCGCTTCCTCGCTCAC
TGACTCGCTGCGCTCGGTCGTTCGGCTGCGGCGAGCGGTATCAGCTCACTCAAAGGCGGT
AATACGGTTATCCACAGAATCAGGGGATAACGCAGGAAAGAACATGTGAGCAAAAGGCCA
GCAAAAGGCCAGGAACCGTAAAAAGGCCGCGTTGCTGGCGTTTTTCCATAGGCTCCGCCC
CCCTGACGAGCATCACAAAAATCGACGCTCAAGTCAGAGGTGGCGAAACCCGACAGGACT
ATAAAGATACCAGGCGTTTCCCCCTGGAAGCTCCCTCGTGCGCTCTCCTGTTCCGACCCT
GCCGCTTACCGGATACCTGTCCGCCTTTCTCCCTTCGGGAAGCGTGGCGCTTTCTCATAG
CTCACGCTGTAGGTATCTCAGTTCGGTGTAGGTCGTTCGCTCCAAGCTGGGCTGTGTGCA
CGAACCCCCCGTTCAGCCCGACCGCTGCGCCTTATCCGGTAACTATCGTCTTGAGTCCAA
CCCGGTAAGACACGACTTATCGCCACTGGCAGCAGCCACTGGTAACAGGATTAGCAGAGC
GAGGTATGTAGGCGGTGCTACAGAGTTCTTGAAGTGGTGGCCTAACTACGGCTACACTAG
AAGGACAGTATTTGGTATCTGCGCTCTGCTGAAGCCAGTTACCTTCGGAAAAAGAGTTGG
TAGCTCTTGATCCGGCAAACAAACCACCGCTGGTAGCGGTGGTTTTTTTGTTTGCAAGCA
GCAGATTACGCGCAGAAAAAAAGGATCTCAAGAAGATCCTTTGATCTTTTCTACGGGGTC
TGACGCTCAGTGGAACGAAAACTCACGTTAAGGGATTTTGGTCATGAGATTATCAAAAAG
GATCTTCACCTAGATCCTTTTAAATTAAAAATGAAGTTTTAAATCAATCTAAAGTATATA
TGAGTAAACTTGGTCTGACAGTTACCAATGCTTAATCAGTGAGGCACCTATCTCAGCGAT
CTGTCTATTTCGTTCATCCATAGTTGCCTGACTCCCCGTCGTGTAGATAACTACGATACG
GGAGGGCTTACCATCTGGCCCCAGTGCTGCAATGATACCGCGAGACCCACGCTCACCGGC
TCCAGATTTATCAGCAATAAACCAGCCAGCCGGAAGGGCCGAGCGCAGAAGTGGTCCTGC
AACTTTATCCGCCTCCATCCAGTCTATTAATTGTTGCCGGGAAGCTAGAGTAAGTAGTTC
GCCAGTTAATAGTTTGCGCAACGTTGTTGCCATTGCTACAGGCATCGTGGTGTCACGCTC
GTCGTTTGGTATGGCTTCATTCAGCTCCGGTTCCCAACGATCAAGGCGAGTTACATGATC
CCCCATGTTGTGCAAAAAAGCGGTTAGCTCCTTCGGTCCTCCGATCGTTGTCAGAAGTAA
GTTGGCCGCAGTGTTATCACTCATGGTTATGGCAGCACTGCATAATTCTCTTACTGTCAT
GCCATCCGTAAGATGCTTTTCTGTGACTGGTGAGTACTCAACCAAGTCATTCTGAGAATA
GTGTATGCGGCGACCGAGTTGCTCTTGCCCGGCGTCAATACGGGATAATACCGCGCCACA

TAGCAGAACTTTAAAAGTGCTCATCATTGGAAAACGTTCTTCGGGGCGAAAACTCTCAAG
GATCTTACCGCTGTTGAGATCCAGTTCGATGTAACCCACTCGTGCACCCAACTGATCTTC
AGCATCTTTTACTTTCACCAGCGTTTCTGGGTGAGCAAAAACAGGAAGGCAAAATGCCGC
AAAAAAGGGAATAAGGGCGACACGGAAATGTTGAATACTCATACTCTTCCTTTTTCAATA
TTATTGAAGCATTTATCAGGGTTATTGTCTCATGAGCGGATACATATTTGAATGTATTTA
GAAAAATAAACAAATAGGGGTTCCGCGCACATTTCCCCGAAAAGTGCCACCTAAATTGTA
AGCGTTAATATTTTGTTAAAATTCGCGTTAAATTTTTGTTAAATCAGCTCATTTTTTAAC
CAATAGGCCGAAATCGGCAAAATCCCTTATAAATCAAAAGAATAGACCGAGATAGGGTTG
AGTGTTGTTCCAGTTTGGAACAAGAGTCCACTATTAAAGAACGTGGACTCCAACGTCAAA
GGGCGAAAAACCGTCTATCAGGGCGATGGCCCACTACGTGAACCATCACCCTAATCAAGT
TTTTTGGGGTCGAGGTGCCGTAAAGCACTAAATCGGAACCCTAAAGGGAGCCCCCGATTT
AGAGCTTGACGGGGAAAGCCAACCTGGCTTATCGAAATTAATACGACTCACTATAGGGAG
ACCGGC

Figure 12

Figure 13

Figure 14

```
MH WT CO            MH WT CO            MH  WT CO            MH  WT CO
Ala C  53 29 59     Cys C  68 67 83     Leu C  26  12 30     Ser C  28  14  43
    U  17 35  6     TG  U  32 33 17     CT  U   5   9  0     TC  U  13  14   7
GC  A  13 29 12                             A   3   9  0         A   5   7   0
    G  17  6 24     Gln A  12 75 17         G  58   3 70         G   9   0   0
                    CA  G  88 25 83     UU  A   2  55  0     AG  C  34  29  50
Arg C  37  8 42                             G   6  12  0         U  10  36   0
CG  U   7 17  0     Glu A  25 68 18
    A   6  8  0     GA  G  75 32 81     Lys A  18  83 17     Thr C  57  9 64
    G  21 25 25                         AA  G  82  17 83     AC  T  14  45 9
AG  A  10 25  8     Gly C  50  7 50                              A  14  45 9
    G  18 17 25     GG  T  12 43  7     Pro C  48   0 57         G  15   0 18
                        A  14 29 14     CC  T  19  57 14
Asn C  78 29 71         G  24 21 29         A  16  29 14     Tyr C  74 20 80
AA  T  22 71 29                             G  17  14 14     TA  T  26 80 20
                    His C  79  33 78
Ile C  77 50 83     CA  T  21  67 22     Phe C  80  30 80     Val C  25 25 38
AT  T  18 33 17                         TT  T  20  70 20     GT  T   7  0  0
    A   5 17  0     Asp C  75  13 75                             A   5 50  0
                    GA  T  25  88 25
```

Figure 15

```
                10             20             30
       -------------+--------------+--------------+-
   1  ATG AGCC G CC T G GA C AA G AG C AAAGTGAT C  CO
   1  ATG TCTA G AT T A GA T AA A AG T AAAGTGAT T  WT

                40             50             60
       -------------+--------------+--------------+-
  31  AAC TC CGC CC T G GAGCTGCT G AATGAGGTC      CO
  31  AAC AG CGC AT T A GAGCTGCT T AATGAGGTC      WT

                70             80             90
       -------------+--------------+--------------+-
  61  GG C ATCGA G GG AC T GA C CAC G CG C AA G CT G  CO
  61  GG A ATCGA A GG TT T AA C AAC C CG T AA A CT C  WT

               100            110            120
       -------------+--------------+--------------+-
  91  GCCCA A AAGCT G GG C GT C GAGCAGCC GA C C     CO
  91  GCCCA G AAGCT A GG T GT A GAGCAGCC TA C A     WT

               130            140            150
       -------------+--------------+--------------+-
 121  C TGTATTGGCATGT G AA G AA C AAG A GGGC C     CO
 121  T TGTATTGGCATGT A AA A AA T AAG C GGGC T     WT

               160            170            180
       -------------+--------------+--------------+-
 151  C T C CT G GACGC GC T C GCCAT C GA A ATG C T G  CO
 151  T T G CT C GACGC CT T A GCCAT T GA G ATG T T A  WT

               190            200            210
       -------------+--------------+--------------+-
 181  GA T C GGCACCA C AC C CACTT C TG T CC CC T C  CO
 181  GA T A GGCACCA T AC T CACTT T TG C CC TT T A  WT

               220            230            240
       -------------+--------------+--------------+-
 211  GAAGG C GA G AGCTGGCA GGA C TTT C T GA G A   CO
 211  GAAGG G GA A AGCTGGCA AGA T TTT T T AC G T   WT

               250            260            270
       -------------+--------------+--------------+-
 241  AAC AACG C C AA GTCC TT CC G C TG C GC CC T C  CO
 241  AAT AACG CT AA AAGT TT TA G A TG TGC TT T A   WT

               280            290            300
       -------------+--------------+--------------+-
 271  CT GA G C CATCGCGATGG G GC C AA G GT G CA C  CO
 271  CT AA G T CATCGCGATGG AGC AAAAGT A CA T     WT
```

60

```
                 310         320         330
        --------------+-----------+------------+-
301  C T G G G A C G C G G C C C A C T G A G A A A C A G T A C  CO
301  T T A G G T A C A C G G C C T A C A G A A A A C A G T A T  WT

                 340         350         360
        --------------+-----------+------------+-
331  G A A A C C C T G G A G A A T C A G C T G G C G T T C C T C C  CO
331  G A A A C T C T C G A A A A T C A A T T A G C C T T T T A  WT

                 370         380         390
        --------------+-----------+------------+-
361  T G C C A G C A G G G G T T C T C C C T G G A G A A C G C C C  CO
361  T G C C A C A A G G T T T T C A C T A G A G A A T G C A  WT

                 400         410         420
        --------------+-----------+------------+-
391  C T C T A C G C A C T C T C C G C C G T G G G C C A C T T T  CO
391  T T A T A T G C A C T C A G C G G T G T G G G G C A T T T T  WT

                 430         440         450
        --------------+-----------+------------+-
421  A C A C T C G G T T G C G T G C T G G A G G A C C A G G A G  CO
421  A C T T T A G G T T G C G T A T T G G A A G A T C A A G A G  WT

                 460         470         480
        --------------+-----------+------------+-
451  C A C C A A G T C G C T A A G G A G G A G C G G G A G A C C  CO
451  C A T C A A G T C G C T A A A G A A G A A A G G G A A A C A  WT

                 490         500         510
        --------------+-----------+------------+-
481  C C C A C C A C C G A C T C C A T G C C C C C A C T G C T G  CO
481  C C T A C T A C T G A T A G T A T G C C G C C A T T A T T A  WT

                 520         530         540
        --------------+-----------+------------+-
511  A G G C A G G C G A T T G A G C T G T T C G A C C A C C A G  CO
511  C G A C A A G C T A T C G A A T T A T T T G A T C A C C A A  WT

                 550         560         570
        --------------+-----------+------------+-
541  G G A G C A G A G C C T G C G T T C C T C T T C G G G C T G  CO
541  G G T G C A G A G C C A G C C T T C T T A T T C G G C C T T  WT
```

```
              580           590           600
    ----------------+---------------+------------------+-
571 GAA CT C ATCAT C TGCGG CC T G GA G AA G CA G  CO
571 GAA TT G ATCAT A TGCGG AT T A GA A AA A CA A  WT


              610           620           630
    ----------------+---------------+------------------+-
601 CT G AA G TG C GA G AG C GG C TCCGC C TACAGC  CO
601 CT T AA A TG T GA A AG T GG G TCCGC G TACAGC  WT


              640           650
    -------------------+---------------+-----------------
631 GG CAG C A GGGA G TTC C GC TCTTA C TAA    CO
631 GG ATC C C GGGA A TTC A GA TCTTA T TAA    WT
```

Figure 16

Figure 17

EP 2 348 119 B1

Figure 18

64

Figure 19

Figure 20

Figure 21

| Virus | TITRE |
|---|---|
| 1 8.1T+pONY3.1+Rev | 1.29E+06 |
| 2 8.1T+pESYNGP+pCINeo | 6.55E+03 |
| 3 8.1T+pESYNGP+Rev | 3.42E+05 |
| 4 8.4NCZ+pESYNGP+Rev | 4.15E+05 |
| 5 8.4NCZ+pESYNGP+pCINeo | 2.59E+05 |
| 6 8.4NCT+pESYNGP+Rev | 4.19E+05 |
| 7 8.4NCT+pESYNGP+pCINeo | 1.92E+05 |
| 8 8.7NCT+pESYNGP+Rev | 3.59E+05 |
| 9 8.7NCT+pESYNGP+pCINeo | 4.41E+05 |
| 10 8.4NCT+pESYNGP+Rev | 3.94E+05 |
| 11 8.4NCT+pESYNGP+pCINeo | 2.44E+05 |
| | |
| 10 8.7NCZ+pESYNGP+pCINeo | 1.15E+06 |
| 11 8.7NCZ+pESYNGP+pESYNRev | 1.67E+06 |

Figure 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9817815 A
- WO 9945126 A
- WO 9915683 A
- WO 0179518 A
- WO 9932646 A
- WO 9429438 A
- WO 9205266 A
- WO 9961639 A
- WO 9805759 A
- WO 9805754 A
- WO 9717457 A
- WO 9609400 A
- WO 9100047 A
- WO 9941397 A
- GB 9601230 W
- WO 9714809 A
- WO 9809985 A
- WO 9637623 A
- WO 9817816 A

**Non-patent literature cited in the description**

- **VERMA ; SOMIA.** *Nature,* 1997, vol. 389, 239-242
- **FULLER M ; ANSON DS.** *Human Gene Therapy,* 2001, vol. 12, 2081-2093
- **SAMBROOK et al.** Molecular Cloning, A Laboratory Manual. 1989
- **AUSUBEL et al.** Short Protocols in Molecular Biology. John Wiley & Sons, Inc, 1999
- **COFFIN et al.** Retroviruses. Cold Spring Harbour Laboratory Press, 1997, 758-763
- **LEWIS et al.** *EMBO J.,* 1992, 3053-3058
- **YU et al.** *Proc Natl Acad Sci,* 1986, vol. 83, 3194-3198
- **DOUGHERTY ; TEMIN.** *Proc Natl Acad Sci,* 1987, vol. 84, 1197-1201
- **HAWLEY et al.** *Proc Natl Acad Sci,* 1987, vol. 84, 2406-2410
- **YEE et al.** *Proc Natl Acad Sci,* 1987, vol. 91, 9564-9568
- **JOLLY et al.** *Nucleic Acids Res,* 1983, vol. 11, 1855-1872
- **EMERMAN ; TEMIN.** *Cell,* 1984, vol. 39, 449-467
- **HERMAN ; COFFIN.** Science. 1987, vol. 236, 845-848
- **CLABOUGH et al.** *J Virol.,* 1991, vol. 65, 6242-51
- **DERSE ; NEWBOLD.** *Virology,* 1993, vol. 194, 530-6
- **MAURY et al.** *Virology,* 1994, vol. 200, 632-42
- **MARTARANO et al.** *J Virol.,* 1994, vol. 68, 3102-11
- **MILLER.** *Curr Top Microbiol Immunol,* 1992, vol. 158, 1-24
- Retroviruses. Cold Spring Harbour Laboratory Press, 1997, 449
- **KARREMAN et al.** *NAR,* 1996, vol. 24, 1616-1624
- **VANIN et al.** *J. Virol,* 1997, vol. 71, 7820-7826
- **PEAR et al.** *PNAS,* 1993, vol. 90, 8392-8396
- **LANDAU ; LITTMAN.** *J. Virol.,* 1992, vol. 66, 5110
- **SONEOKA et al.** *Nucleic Acids Res,* 1995, vol. 23, 628-633
- **MEBATSION et al.** *Cell,* 1997, vol. 90, 841-847
- **BOWTELL et al.** *J.Virol.,* 1988, vol. 62, 2464
- **CORRELL et al.** *Blood,* 1994, vol. 84, 1812
- **EMERMAN ; TEMIN.** *Cell,* 1984, vol. 39, 459
- **GHATTAS et al.** *Mol.Cell.Biol.,* 1991, vol. 11, 5848
- **HANTZOPOULOS et al.** *PNAS,* 1989, vol. 86, 3519
- **HATZOGLOU et al.** *J.Biol.Chem,* 1991, vol. 266, 8416
- **HATZOGLOU et al.** *J.Biol.Chem,* 1988, vol. 263, 17798
- **LI et al.** *Hum.Gen.Ther.,* 1992, vol. 3, 381
- **MCLACHLIN et al.** *Virol.,* 1993, vol. 195, 1
- **OVERELL et al.** *Mol.Cell Biol.,* 1988, vol. 8, 1803
- **SCHARFMAN et al.** *PNAS,* 1991, vol. 88, 4626
- **VILE et al.** *Gene Ther,* 1994, vol. 1, 307
- **XU et al.** *Virol.,* 1989, vol. 171, 331
- **YEE et al.** *PNAS,* 1987, vol. 84, 5197
- **ADAM et al.** *J.Virol.,* 1991, vol. 65, 4985
- **KOO et al.** *Virology,* 1992, vol. 186, 669-675
- **CHEN et al.** *J. Virol,* 1993, vol. 67, 2142-2148

- **JANG et al.** *Enzyme,* 1990, vol. 44, 292-309
- **MOUNTFORD ; SMITH.** *TIG,* May 1995, vol. 11 (5), 179-184
- **GHATTAS, I.R. et al.** *Mol. Cell. Biol.,* 1991, vol. 11, 5848-5859
- **MACEJAK ; SAMOW.** *Nature,* 1991, vol. 353, 91
- **OH et al.** *Genes & Development,* 1992, vol. 6, 1643-1653
- **PELLETIER ; SONENBERG.** *Nature,* 1988, vol. 334, 320-325
- **MOUNTFORD ; SMITH.** *TIG,* 1985, vol. 11, 179-184
- **SAMBROOK et al.** Molecular Cloning: A Laboratory Manual. Cold Spring Harbor Laboratory press, 1989
- *Nature Biotechnology,* 1996, vol. 14, 556
- **CARROLL, MW ; GW WILKINSON ; K LUNSTROM.** Mammalian expression systems and vaccination Genetically Engineered Viruses. BIOS Scientific, 2001, 107-157
- **DAVISON ; MOSS.** *J. Mol. Biol.,* 1989, vol. 210, 749-769
- **ATTENELLO ; LEE.** *Science,* 1984, vol. 226, 187-190
- **GAZIT et al.** *Cancer Res,* 1995, vol. 55, 1660-1663
- **WANG ; SEMENZA.** *Proc Natl Acad Sci,* 1993, vol. 90, 430
- **DACHS et al.** *Nature Med,* 1997, vol. 5, 515
- **FIRTH et al.** *Proc Natl Acad Sci,* 1994, vol. 91, 6496-6500
- **MADAN et al.** *Proc Natl Acad Sci,* 1993, vol. 90, 3928
- **SEMENZA ; WANG.** *Mol Cell Biol 1992,* 1992, vol. 12, 5447-5454
- **TAKENAKA et al.** *J Biol Chem,* 1989, vol. 264, 2363-2367
- **PESHAVARIA ; DAY.** *Biochem J,* 1991, vol. 275, 427-433
- **INOUE et al.** *J Biol Chem,* 1989, vol. 264, 14954-14959
- Remington's Pharmaceutical Sciences. Mack Publishing Co, 1985
- **BIERI et al.** *Nat. Biotechnol.,* 1999, vol. 17, 1105-1108
- **LI et al.** *Nucleic Acids Research,* 2000, vol. 28 (13), 2605-2612
- **BEGER et al.** *PNAS,* 2001, vol. 98 (1), 130-135
- **KESTLER et al.** *Human Gene Ther.,* 1999, vol. 10 (10), 1619-32
- **WINTER et al.** *Annu Rev Immunol.,* 1994, vol. 12, 433-55
- **HOOGENBOOM.** *Trends Biotechnol.,* 1997, vol. 15, 62-70
- **PARMLEY ; SMITH.** *Gene,* 1988, vol. 73, 305-18
- **LOWMAN et al.** *Biochemistry,* 1991, vol. 30, 10832-8
- **NISSIM et al.** *Embo J.,* 1994, vol. 13, 692-8
- **YELAMOS et al.** *Nature,* 1995, vol. 376, 225-9
- **ZACCOLO et al.** *J. Mo.1 Biol.,* 1996, vol. 255, 589-603
- **LEUNG et al.** *Technique,* 1989, vol. 1, 11-15
- **KOWALCZYKOWSKI et al.** *Microbiol. Rev.,* 1994, vol. 58, 401-65
- **STEMMER.** *Nature,* 1994, vol. 370, 389-91
- **STEMMER.** *Proc. Natl. Acad. Sci. USA,* 1994, vol. 91, 10747-51
- **GOODCHILD, JVK.** *Arch. Biochem. Biophys.,* 1991, vol. 284, 386-391
- Molecular Biology and Biotechnology. VCH Publishers Inc, 1995, 831-8320
- Retroviruses. Cold Spring Harbour Laboratory Press, 1997, 683
- Retroviruses. Cold Spring Harbour Laboratory Press, 1997, 758-763
- **LEWIS et al.** *EMBO. J.,* 1992, vol. 11, 3053-3058
- **LEWIS ; EMERMAN.** *J. Virol.,* 1994, vol. 68, 510-516
- **YU et al.** *Proc. Natl. Acad. Sci.,* 1986, vol. 83, 3194-3198
- **DOUGHERTY ; TEMIN.** *Proc. Natl. Acad. Sci.,* 1987, vol. 84, 1197-1201
- **HAWLEY et al.** *Proc. Natl. Acad. Sci.,* 1987, vol. 84, 2406-2410
- **YEE et al.** *Proc. Natl. Acad. Sci.,* 1987, vol. 91, 9564-9568
- **JOLLY et al.** *Nucleic Acids Res.,* 1983, vol. 11, 1855-1872
- **HERMAN ; COFFIN.** *Science,* 1987, vol. 236, 845-848
- **CLABOUGH et al.** *J. Virol.,* 1991, vol. 65, 6242-51
- **MARTARANO et al.** *J. Virol.,* 1994, vol. 68, 3102-11
- **SENTER et al.** *Proc. Natl. Acad. Sci.,* 1988, vol. 85, 4842-4846
- **MULLEN et al.** *Cancer Res.,* 1994, vol. 54, 1503-1506
- **KERR et al.** *Cancer Immunol. Immunother.,* 1990, vol. 31, 202-206
- **BORRELLI et al.** *Proc. Natl. Acad. Sci.,* 1988, vol. 85, 7572-7576
- **FRIEDLOS et al.** *J. Med. Chem.,* 1997, vol. 40, 1270-1275
- **CHEN et al.** *Cancer Res.,* 1996, vol. 56, 1331-1340
- **SONEOKA et al.** *Nucleic Acids Res.,* 1995, vol. 23, 628-633
- **TOMKO et al.** *Proc. Natl. Acad. Sci.,* 1997, vol. 94, 3352-2258

- **WICKHAM et al.** *Cell,* 1993, vol. 73, 309-319
- **WICKHAM et al.** *J. Cell Biol.,* 1994, vol. 127, 257-264
- **HONG et al.** *EMBO,* 1997, vol. 16, 2294-2306
- **MEYER et al.** *J. Gen. Virol.,* 1991, vol. 72, 1031-1038
- **SMITH ; MOSS.** *Gene,* 1983, vol. 25, 21-28
- **UPTON et al.** *J. Virology,* 1986, vol. 60, 920
- **GERSHON et al.** *J. Gen. Virol.,* 1989, vol. 70, 525
- **WEIR et al.** *J. Virol.,* 1983, vol. 46, 530
- **ESPOSITO et al.** *Virology,* 1984, vol. 135, 561
- **HRUBY et al.** *PNAS,* 1983, vol. 80, 3411
- **KILPATRICK et al.** *Virology,* 1985, vol. 143, 399
- **BINNS et al.** *J. Gen. Virol.,* 1988, vol. 69, 1275
- **BOYLE et al.** *Virology,* 1987, vol. 156, 355
- **SCHNITZLEIN et al.** *J. Virological Method,* 1988, vol. 20, 341
- **LYTVYN et al.** *J. Gen. Virol.,* 1992, vol. 73, 3235-3240
- **MOSS, B.** *Science,* 1991, vol. 252, 1662-7
- **MERCHLINSKY, M. et al.** *Virology,* 1992, vol. 190, 522-526
- **SCHEIFLINGER, F. et al.** *Proc. Natl. Acad. Sci.,* 1992, vol. 89, 9977-9981
- **SCHEIFLINGER, F. et al.** *Proc.Natl. Acad. Sci. USA,* 1992, vol. 89, 9977-9981
- **PFLEIDERER et al.** *J. General Virology,* 1995, vol. 76, 2957-2962
- **MARSHALL.** *Nature Biotechnology,* 1998, vol. 16, 129
- **WANG ; SEMEMNZA.** *Proc. Natl. Acad. Sci. USA,* 1993, vol. 90, 4304
- **FIRTH et al.** *Proc. Natl. Acad. Sci. USA,* 1994, vol. 91, 6496
- **KALLIO et al.** *Embo J.,* 1998, vol. 17, 6573-86
- **SEMENZA, G et al.** *J. Biol. Chem.,* 1994, vol. 269, 23757-63
- **ROYDS et al.** *Mol. Pathol.,* 1998, vol. 51, 55-61
- **MATSUI et al.** *Cardiovasc. Res.,* 1999, vol. 42, 104-12
- **YAN et al.** *J. Biol. Chem.,* 1997, vol. 272, 4287-94
- **ESTES et al.** *Exp. Cell Res.,* 1995, vol. 220, 47-54
- **GAZIT et al.** *Cancer Res.,* 1995, vol. 55, 1660
- **MATTHIAS et al.** *NAR,* 1989, vol. 17, 6418
- **GAZIT et al.** *Cancer Res.,* 1995, vol. 55, 1660-1663
- **WANG ; SEMENZA.** *Proc. Natl. Acad. Sci.,* 1993, vol. 90, 430
- **DACHS et al.** *Nature Med.,* 1997, vol. 5, 515
- **FIRTH et al.** *Proc. Natl. Acad. Sci.,* 1994, vol. 91, 6496-6500
- **MADAN et al.** *Proc. Natl. Acad. Sci.,* 1993, vol. 90, 3928
- **SEMENZA ; WANG.** *Mol. Cell Biol.,* 1992, vol. 12, 5447-5454
- **TAKENAKA et al.** *J. Biol. Chem.,* 1989, vol. 264, 2363-2367
- **PESHAVARIA ; DAY.** *Biochem. J.,* 1991, vol. 275, 427-433
- **INOUE et al.** *J. Biol. Chem.,* 1989, vol. 264, 14954-14959
- **O'HARE, M. J. et al.** *Proc. Natl. Acad. Sci. USA,* vol. 98 (2), 646-51
- **GILLETT et al.** *Cancer Res.,* 1994, vol. 54 (7), 1812-7
- **YU et al.** *Nature,* 2001, vol. 411 (6841), 1017-21
- **STIRLING ; CHUNG.** *Eur. Respir. J.,* 2000, vol. 16 (6), 1158-74
- **WELTMAN ; KARIM.** *Expert Opin. Investig. Drugs,* 2000, vol. 9 (3), 491-6
- **CAI, H. et al.** *Nat. Neurosci.,* 2001, vol. 4 (3), 233-4
- **VIGNERI ; WANG.** *Nat. Med.,* 2001, vol. 7 (2), 228-34
- **GORRE et al.** *Science,* 03 August 2001, vol. 293 (5531), 876-80
- **MCCORMICK.** *Nature,* 19 July 2001, vol. 412 (6844), 281-2
- **JAHAGIRDAR et al.** *Exp. Hematol.,* May 2001, vol. 29 (5), 543-56
- **SILLABER et al.** *Blood.,* 15 March 2000, vol. 95 (6), 2118-25
- **LIU et al.** *Mol. Cell Biol.,* March 1996, vol. 16 (3), 998-1005
- **ARLINGHAUS.** *Crit. Rev. Oncog.,* 1998, vol. 9 (1), 1-18
- **SHAH et al.** *Mol Cell Biol.,* April 1991, vol. 11 (4), 1854-60
- **ZHU et al.** *Nucleic Acids Res.,* 11 December 1990, vol. 18 (23), 7119-7125
- **COLLINS et al.** *Mol. Cell Biol.,* August 1987, vol. 7 (8), 2870-2876
- **VONCKEN et al.** *Int. J. Mol. Med.,* November 1998, vol. 2 (5), 577-583
- **VONCKEN et al.** *Cell,* 10 March 1995, vol. 80 (5), 719-728
- **VONCKEN et al.** *Oncogene,* 16 April 1998, vol. 16 (15), 2029-2032
- **WETZLER et al.** *J. Clin. Invest,* October 1993, vol. 92 (4), 1925-1939
- **PERKINS et al.** *Blood,* 01 February 2000, vol. 95 (3), 1014-22
- **POROSNICU et al.** *Leukemia,* May 2001, vol. 15 (5), 772-778
- **KWONG ; TODD.** *Blood,* 01 May 1997, vol. 89 (9), 3487-8
- **PUCCETTI et al.** *Cancer Res.,* 01 July 2000, vol. 60 (13), 3409-3413
- **ANDREWS ; COLLINS.** *Leukemia,* October 1987, vol. 1 (10), 718-24

- **DALGAARD.** *Acta, Med, Scand.,* 1957, vol. 328, 1-255
- **BACOLLA et al.** *J. Biol. Chem.,* 25 May 2001, vol. 276 (21), 18597-604
- **ARNAOUT.** *Annu. Rev. Med.,* 2001, vol. 52, 93-123
- **CALVET ; GRANTHAM.** *Semin Nephrol.,* March 2001, vol. 21 (2), 107-23
- **BOLETTA et al.** *MoL Cell,* November 2000, vol. 6 (5), 1267-73
- **GRANTHAM.** *Curr Opin Nephrol Hypertens,* July 2001, vol. 10 (4), 533-42
- **ARNOULD et al.** *Mol. Cell Biol.,* May 1999, vol. 19 (5), 3423-34
- **PUGH et al.** *Kidney Int.,* March 1995, vol. 47 (3), 774-81
- **RICHARD et al.** *J. Clin. Invest.,* vol. 101 (5), 935-9
- **IBRAGHIMOV-BESKROVNAYA et al.** *Proc Natl Acad Sci USA.,* 10 June 1997, vol. 94 (12), 6397-402
- **KIM.** *Proc. Natl. Acad. Sci. USA.,* 15 February 2000, vol. 97 (4), 1731-6
- **PEY et al.** *Vitro Cell Dev. Biol. Anim.,* vol. 35 (10), 571-9
- **MISKIN et al.** *Science,* 1998, vol. 281, 562-565
- *J. Virol.,* vol. 74, 9412-9420
- **POWELL et al.** *J. Virol.,* vol. 70, 8527-33
- **TAIT et al.** *J. Biol. Chem.,* vol. 275, 34656-64
- **O'HARE, M. J. et al.** *Proc. Natl. Acad. Sci. U S A.,* 2001, vol. 98 (2), 646-51
- **EMMONS SW ; SOMLO S.** *Nature,* 23 September 1999, vol. 401 (6751), 339-40